# FASCICULE DE BREVET EUROPEEN

(11) **EP 0 388 309 B1**
(45) Date de publication et mention de la délivrance du brevet: **02.03.1994**
(21) Numéro de dépôt: 90400704.4
(22) Date de dépôt: 16.03.1990
(51) Int. Cl.: C07D 513/04, C07D 519/00, A61K 31/425, A61K 31/435

(54) **Nouveaux dérivés du 1h,3h-pyrrolo [1,2-c] thiazolecarboxamide-7, leur préparation et les compositions pharmaceutiques qui les contiennent.**
1H,3H,7-Pyrrolo [1,2-c]thiazolcarbonsäureamid-Derivate, ihre Herstellung und diese enthaltende pharmazeutische Zubereitungen
1H,3H,7-pyrrolo [1,2-c] thiazolecarboxamides, their preparation and pharmaceutical compositions containing them

(30) Priorité: 17.03.1989 FR 8903510
(43) Date de publication de la demande: 19.09.1990
(73) Titulaire: RHONE-POULENC SANTE, 92160 Antony (FR)
(72) Inventeur: James, Claude, F-75020 Paris (FR); Lave, Daniel, F-75019 Paris (FR); Soler, Françoise, F-75020 Paris (FR)
(74) Mandataire: Pilard, Jacques

(56) Documents cités:
- EP-A- 0 253 711

## Description

La présente invention concerne de nouveaux dérivés du 1H,3H-pyrrolo[1,2-c] thiazolecarboxamide-7 de formule générale : 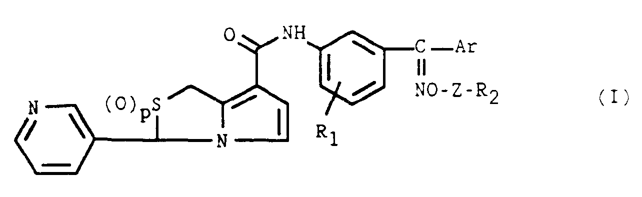
dans laquelle R₁ représente un atome d'hydrogène ou d'halogène ou un radical alcoyle, alcoyloxy, trifluorométhyle, amino, alcoylamino, dialcoylamino, hydroxy, cyano, phényle ou phénoxy, Ar représente un radical phényle, naphtyle, pyridyle, quinolyle, isoquinolyle, thiényle, thiéno[2,3-b] thiényle-2 ou thiéno[3,2-b] thiényle-2, ces radicaux pouvant éventuellement porter un ou plusieurs substituants choisis parmi les atomes d'halogène ou les radicaux alcoyle, alcoyloxy, trifluorométhyle, amino, alcoylamino, dialcoylamino, hydroxy ou cyano, p représente un nombre entier égal à zéro, un ou deux et
A- soit Z représente une liaison de valence et R₂ représente un atome d'hydrogène,
B- soit Z représente une liaison de valence et R₂ représente un radical pyridyle-2 ou -4, quinuclidinyle-3, pyrrolidinyle-3, pipéridyle-3 ou -4, ces deux derniers radicaux pouvant être éventuellement substitués sur l'atome d'azote par un radical alcoyle, hydroxyalcoyle, phényle ou phénylalcoyle,
C- soit Z représente un radical alcoylène contenant 1 à 4 atomes de carbone et R₂ représente un radical pyridyle-2, -3 ou -4, quinuclidinyle-3, pyrrolidinyle-2 ou -3, pipéridyle-2, -3 ou -4, ces deux derniers radicaux pouvant être substitués sur l'atome d'azote par un radical alcoyle, hydroxyalcoyle, phényle ou phénylalcoyle ou bien R₂ représente un radical de formule générale : 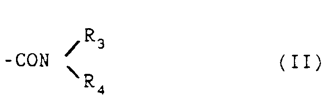 dans laquelle :
   a) soit R₃ et R₄ forment ensemble avec l'atome d'azote auquel ils sont liés un cycle pipérazine éventuellement substitué sur le deuxième atome d'azote par un radical alcoyle, hydroxyalcoyle, pyridyle, phényle ou phénylalcoyle,
   b) soit R₃ représente un atome d'hydrogène ou un radical alcoyle, phényle ou phénylalcoyle ou un radical de formule générale : 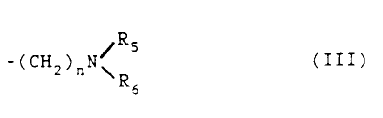 dans laquelle n représente un nombre entier compris entre 1 et 4 et R₅ et R₆, identiques ou différents, représentent un atome d'hydrogène ou un radical alcoyle, phényle, phénylalcoyle, ou bien R₅ et R₆ forment ensemble avec l'atome d'azote auquel ils sont liés un cycle morpholine, thiomorpholine, pyrrolidine, pipéridine ou pipérazine dont le second atome d'azote peut être éventuellement substitué par un radical alcoyle, hydroxyalcoyle, pyridyle, phényle, phénylalcoyle ou phénylcarbonyle, et R₄ représente un radical de formule générale (III) défini comme précédemment, étant entendu que les définitions de n, R₅ et R₆ dans les symboles R₃ et R₄ peuvent être identiques ou différents,
D- soit Z représente un radical alcoylène contenant 1 à 4 atomes de carbone et R₂ représente un radical de formule générale : 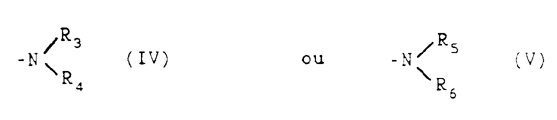 dans lesquelles R₃, R₄, R₅ et R₆ sont définis comme précédemment en C a) et b), étant entendu que les radicaux alcoyle et portions alcoyle contiennent 1 à 4 atomes de carbone en chaîne droite ou ramifiée et que l'invention concerne les produits racémiques, les énantiomères dus à la présence d'un carbone asymétrique en position 3 du cycle pyrrolothiazole, les mélanges de ces énantiomères, les diastéréoisomères purs ou en mélange dus à la présence éventuelle d'un autre centre chiral, les isomères E et Z (encore appelés syn et anti) et leurs mélanges provenant de la présence de la fonction oxime C=NO-Z-R₂, ainsi que les sels pharmaceutiquement acceptables des produits de formule (I) ainsi définie.

Selon l'invention, les produits de formule générale (I) peuvent être préparés par action d'une hydroxylamine de formule générale :

H₂NO-Z-R₂ (VI)

dans laquelle Z et R₂ sont définis comme précédemment, sur une cétone de formule générale : 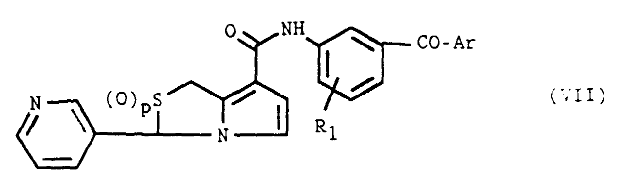
dans laquelle les symboles p, R₁ et Ar sont définis comme précédemment.

Dans la pratique, on utilise l'hydroxylamine de formule générale (VI) sous forme de chlorhydrate et on opère dans un solvant organique tel que la pyridine ou un alcool à bas poids moléculaire tel que le propanol en présence d'un accepteur d'acide tel qu'un carbonate de métal alcalin à une température comprise entre 20°C et la température de reflux du mélange réactionnel.

Les produits de formule générale (VI) peuvent être préparés par application ou adaptation de méthodes décrites dans la littérature.

Les produits de formule générale (VII) dans laquelle p est égal à 1 ou 2 peuvent être obtenus par oxydation d'un produit de formule générale (VII) dans laquelle p est égal à 0, c'est-à-dire un produit de formule générale : 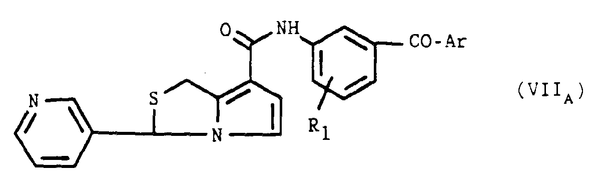
dans laquelle R₁ et Ar sont définis comme précédemment.

L'oxydation peut être réalisée par exemple en employant un agent couramment utilisé pour passer d'un sulfure à un sulfoxyde ou à une sulfone en opérant dans un solvant approprié. Par exemple, on peut employer l'eau oxygénée dans l'acétone ou l'acide acétique, un periodate alcalin dans un alcool ou l'acétonitrile, un peracide carboxylique (acide peracétique, perbenzoïque, m-chloroperbenzoïque, p-nitroperbenzoïque ou perphtalique) dans un éther (dioxanne, tétrahydrofuranne, oxyde de diéthyle), ou un solvant chloré (dichlorométhane, dichloro-1,2 éthane), l'acide acétique ou un mélange de ces solvants. La réaction s'effectue généralement à une température comprise entre -10°C et +20°C.

Il est particulièrement avantageux d'opérer dans le dichlorométhane en présence d'acide m-chloroperbenzoïque, à une température comprise entre 0 et 20°C.

Lorsqu'on désire obtenir le sulfoxyde, il est nécessaire d'effectuer la réaction avec un équivalent d'agent oxydant. Lorsqu'on désire obtenir la sulfone, il est nécessaire d'utiliser au moins deux équivalents d'agent oxydant. La réaction d'oxydation proprement dite s'effectue en présence d'au moins un équivalent d'acide tel que l'acide méthanesulfonique, à une température voisine de 20°C.

Les produits de formule générale (VII) dans laquelle p est égal à zéro, c'est-à-dire les produits de formule générale (VII_{A}) peuvent être préparés selon la méthode décrite dans la demande de brevet européen publiée sous le numéro 0 253 711.

Selon l'invention, les produits de formule générale (I) dans laquelle les symboles sont définis comme précédemment en B, C ou D peuvent être préparés par action d'un produit de formule générale :

X-Z-R₂ (VIII)

dans laquelle Z et R₂ sont définis comme précédemment et X représente un atome d'halogène tel qu'un atome de chlore, de brome ou d'iode, ou un reste d'ester réactif tel que mésyloxy ou tosyloxy, sur un produit de formule générale (I) défini comme précédemment en A, c'est-à-dire un produit de formule générale : 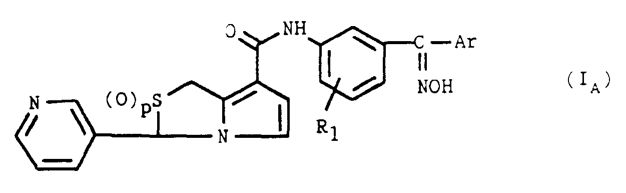
dans laquelle p, R₁ et Ar sont définis comme précédemment.

On opère généralement dans un solvant organique tel que le diméthylformamide à une température voisine de 20°C en présence d'un agent de condensation tel qu'un hydrure alcalin comme l'hydrure de sodium, ou dans un alcool à bas poids moléculaire tel que l'éthanol ou le t-butanol en présence d'un alcoolate de métal alcalin tel que l'éthylate de sodium ou le t-butylate de potassium à reflux.

Les produits de formule générale (I_{A}) peuvent être préparés par action d'un produit de formule générale (VI) dans laquelle Z représente une liaison de valence et R₂ représente un atome d'hydrogène, sur un produit de formule générale (VII) comme il a été dit précédemment.

Les produits de formule générale (I_{A}) dans laquelle p est égal à 1 ou 2 peuvent également être préparés par oxydation d'un produit de formule générale (I) dans laquelle p est égal à zéro, R₁ et Ar sont définis comme précédemment en A, c'est-à-dire un produit de formule générale :

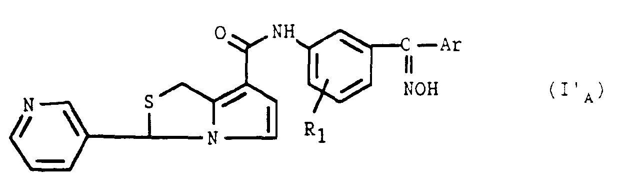

L'oxydation peut être réalisée comme il a été dit précédemment pour l'oxydation d'un produit de formule générale (VII_{A}).

Selon l'invention, les produits de formule générale (I) dans laquelle p est égal à 0 peuvent aussi être obtenus par action d'un dérivé du chloroformyl-7 (pyridyl-3)-3 1H,3H-pyrrolo[1,2-c] thiazole de formule générale : 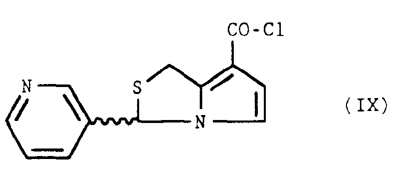
sous forme R ou S ou leurs mélanges, sur une oxime de formule générale : 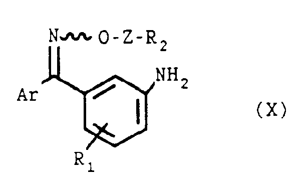
dans laquelle Ar, Z, R₁ et R₂ sont définis comme précédemment, sous forme E ou Z ou leurs mélanges.

Généralement, la condensation est effectuée dans les conditions habituelles de préparation des amides par action d'un chlorure d'acide sur une amine primaire.

Le produit de formule générale (X) peut être obtenu par action d'un produit de formule générale (VIII) sur une oxime de formule générale : 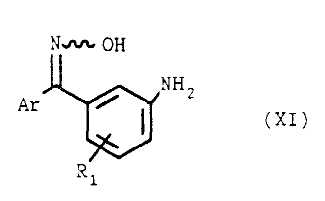
dans laquelle Ar et R₁ sont définis comme précédemment, sous forme E ou Z ou leurs mélanges.

Généralement la réaction est effectuée en présence d'un hydrure de métal alcalin tel que l'hydrure de sodium en opérant dans un solvant organique tel que le diméthylformamide à une température voisine de 20°C.

L'oxime de formule générale (XI) sous forme E ou Z peut être obtenue par séparation, par exemple par chromatographie, du mélange des formes E et Z.

Le mélange des formes E et Z de l'oxime de formule générale (XI) peut être obtenu par action d'un sel d'hydroxylamine (chlorhydrate, tosylate) sur un produit de formule générale : 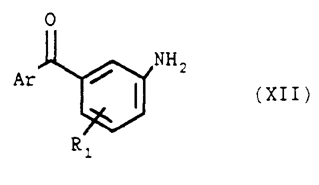
dans laquelle Ar et R₁ sont définis comme précédemment.

Généralement, on opère dans un solvant organique tel que la pyridine ou un alcool à bas poids moléculaire tel que le propanol en présence d'un accepteur d'acide tel qu'un carbonate de métal alcalin comme le carbonate de sodium à une température comprise entre 20°C et la température d'ébullition du mélange réactionnel.

Le produit de formule générale (X) peut aussi être obtenu par action d'une hydroxylamine de formule générale (VI) sur une cétone de formule générale (XII) dans les conditions décrites précédemment pour l'action d'une hydroxylamine de formule générale (VI) sur une cétone de formule générale (VII).

Les produits de formule générale (I) dans laquelle p est égal à 1 peuvent être obtenus par oxydation d'un produit de formule générale (I) dans laquelle p est égal à 0.

Généralement l'oxydation est effectuée au moyen d'un agent oxydant tel que l'eau oxygénée ou l'acide m.chloroperbenzoïque. Il est particulièrement avantageux d'utiliser l'eau oxygénée.

Lorsque, dans la présente invention, on se réfère à un produit déterminé par son nom chimique, il est entendu qu'en l'absence de spécifications particulières, on se réfère toujours au produit racémique ou au mélange des isomères syn et anti.

Les produits de formule générale (I) dans laquelle les substituants Ar et O-Z-R₂ de la fonction oxime sont en position syn ou anti (E ou Z) peuvent être préparés à partir des mélanges correspondants par les méthodes conventionnelles classiques telles que la chromatographie liquide haute performance (CLHP).

Les produits de formule générale (I) peuvent exister sous forme racémique ou sous forme d'énantiomères purs du fait que l'atome de carbone en position 3 du cycle pyrrolothiazole est asymétrique. Pour obtenir les produits de formule générale (I) à l'état d'énantiomères purs, il est avantageux de mettre en oeuvre les procédés décrits précédemment en utilisant un produit de formule générale (VII) déjà dédoublé ; celui-ci peut être préparé selon la méthode décrite dans la demande de brevet européen publiée sous le numéro 0 253 711.

Il est entendu pour l'homme du métier que, pour la mise en oeuvre des procédés décrits précédemment, il peut être nécessaire d'introduire des groupements protecteurs au niveau de certaines fonctions présentes dans les radicaux Ar, R₁ et R₂ des différents produits mis en oeuvre. Le groupement protecteur pourra ensuite être éliminé au moment le plus opportun de la synthèse. Ainsi, lorsque dans le radical Ar, R₁ et/ou R₂ une fonction amino ou alcoylamino est présente, celle-ci pourra être protégée par exemple par un radical t-butyloxycarbonyle puis libérée après réaction au moyen d'un acide aqueux, par exemple au moyen d'une solution aqueuse d'acide chlorhydrique, ou mieux au moyen d'une solution acétique de gaz chlorhydrique. Lorsque dans le radical Ar, R₁ et/ou R₂ est présente une fonction hydroxy, celle-ci pourra être avantageusement protégée sous forme de radical tétrahydropyranyloxy ou méthoxyméthyloxy, puis libérée après réaction par hydrolyse.

Les nouveaux produits de formule générale (I) peuvent être purifiés par les méthodes connues habituelles par exemple par cristallisation ou par chromatographie.

Les nouveaux produits de formule générale (I) peuvent être transformés en sels d'addition avec les acides par réaction avec un acide dans un solvant organique tel qu'un alcool, une cétone, un éther ou un solvant chloré. Le sel précipite, éventuellement après concentration de sa solution ; il est séparé par filtration ou décantation.

Les nouveaux produits selon l'invention ainsi que leurs sels pharmaceutiquement acceptables présentent des propriétés intéressantes associées à une faible toxicité. Ils sont généralement actifs à des concentrations inférieures à 0,5 nM dans le test de mesure de l'activité inhibitrice *in vitro* vis-à-vis de l'agrégation plaquettaire induite par l'O-octadécyl-1 O-acétyl-2 ns-glycérophosphorylcholine-3 (PAF-acéther), selon la technique de G.V.R. BORN et coll., J. Physiol., 168, 178 (1963).

Leur dose toxique (exprimée par la DL₅₀) chez la souris est généralement comprise entre 300 et 900 mg/kg par voie orale.

Pour l'emploi thérapeutique, il pourra être fait usage des nouveaux produits de formule (I) tels quels ou, le cas échéant, à l'état de sels pharmaceutiquement acceptables, c'est-à-dire non toxiques aux doses d'utilisation.

Comme sels pharmaceutiquement acceptables, peuvent être cités les sels d'addition avec les acides minéraux tels que chlorhydrates, sulfates, nitrates, phosphates, ou organiques tels que acétates, propionates, succinates, benzoates, fumarates, maléates, méthanesulfonates, iséthionates, théophillineacétates, salicylates, phénolphtalinates, méthylène-bis-b-oxynaphtoates ou des dérivés de substitution de ces composés.

Les exemples suivants, montrent comment l'invention peut être mise en pratique.

### EXEMPLE 1

A une suspension de 21,27 g de (+)-N-(benzoyl-3 phényl) (pyridyl-3)-3 1H,3H-pyrrolo[1,2-c] thiazolecarboxamide-7 dans 250 cm3 de propanol-1, on ajoute 5,30 g de carbonate de sodium et 6,95 g de chlorhydrate d'hydroxylamine. La suspension est chauffée au reflux, sous agitation pendant 2 heures. Le solvant est évaporé sous pression réduite (20 mm de mercure ; 2,7 kPa) à une température voisine de 45°C. Le résidu est repris dans 250 cm3 d'eau distillée et la cristallisation est amorcée. Les cristaux apparus sont séparés par filtration, lavés 4 fois par 300 cm3 au total d'eau distillée puis séchés à l'air. On obtient ainsi 22,04 g de produit fondant à 210°C. 2 g de ce produit sont dissous dans 80 cm3 de propanol-1 bouillant. La solution obtenue est filtrée à chaud et le filtrat est refroidi à une température voisine de 0°C pendant 48 heures. Les cristaux apparus sont séparés par filtration, lavés trois fois par 10 cm3 au total d'éthanol absolu puis séchés sous pression réduite (0,1 mm de mercure ; 13,5 Pa) à une température voisine de 60°C. On obtient ainsi 1,74 g de (+)-N-[α-(hydroxyiminobenzyl)-3 phényl] (pyridyl-3)-3 1H,3H-pyrrolo [1,2-c] thiazolecarboxamide-7 (mélange des formes E et Z) sous forme de cristaux blancs fondant à 151-155°C.

Le (+)-N-(benzoyl-3 phényl) (pyridyl-3)-3 1H,3H-pyrrolo [1,2-c] thiazolecarboxamide-7 est préparé selon la méthode décrite dans la demande de brevet européen publiée sous le n° 0 253 711.

### EXEMPLE 2

A un mélange de 325 mg d'une dispersion à 50 % d'hydrure de sodium dans l'huile de vaseline et de 4 cm3 de N,N-diméthylformamide anhydre, on ajoute, sous courant d'azote, en cinq minutes environ, une solution de 2 g de (+)-N-[α-(hydroxyiminobenzyl)-3 phényl] (pyridyl-3)-3 1H,3H-pyrrolo[1,2-c] thiazolecarboxamide-7 (mélange des formes E et Z) dans 10 cm3 de N,N-diméthylformamide anhydre. Après un quart d'heure d'agitation à température ambiante, on ajoute en 2 minutes environ une solution de 1,19 g de (chloro-3 propyl)-1 phényl-4 pipérazine dans 3 cm3 de N,N-diméthylformamide et agite la solution obtenue pendant 16 heures à température ambiante. On ajoute alors 150 cm3 d'eau distillée et amorce la cristallisation. Après 3 heures d'agitation à température ambiante, les cristaux obtenus sont séparés par filtration, lavés 3 fois par 45 cm3 au total d'eau distillée et séchés sous pression réduite (0,1 mm de mercure ; 13,5 Pa) à une température voisine de 20°C. On obtient ainsi 2,74 g de produit. Ce produit est dissous dans 15 cm3 d'acétone. La solution obtenue est additionnée de 0,77 g d'acide oxalique et agitée pendant 3 heures à température ambiante. Les cristaux obtenus sont séparés par filtration, lavés 4 fois par 20 cm3 au total d'acétone et séchés sous pression réduite (0,1 mm de mercure ; 13,5 Pa) à une température voisine de 60°C. On obtient ainsi 2,74 g de dioxalate acide de (+)-N-{{α-[(phényl-4 pipérazinyl-1)-3 propoxyimino] benzyl}-3 phényl} (pyridyl-3)-3 1H,3H-pyrrolo[1,2-c] thiazolecarboxamide-7, (mélange des formes E et Z), sous forme de cristaux crème fondant à 130°C.

La (chloro-3 propyl)-1 phényl-4 pipérazine peut être préparée d'après C.B. POLLARD, J. Org. Chem. 24, 764, 1958.

Le(+)-N-[(α-hydroxyiminobenzyl)-3 phényl] (pyridyl-3)-3 1H,3H-pyrrolo[1,2-c] thiazolecarboxamide-7 est obtenu comme décrit à l'exemple 1.

### EXEMPLE 3

A une solution de 2 g de (+)-N-[(α-hydroxyiminobenzyl)-3 phényl] (pyridyl-3)-3 1H,3H-pyrrolo[1,2-c] thiazolecarboxamide-7 (mélange des formes E et Z) dans 10 cm3 de N,N-diméthylformamide anhydre, on ajoute, à température ambiante, sous courant d'azote et sous agitation 0,44 g d'une dispersion à 50 % d'hydrure de sodium dans l'huile de vaseline. On ajoute ensuite une solution de 0,72 g de chlorhydrate de diméthylamino-3 chloro-1 propane dans 20 cm3 de N,N-diméthylformamide anhydre en 1 minute environ. La solution est alors agitée pendant 6 heures à température ambiante, puis additionnée de 10 cm3 d'eau distillée. Le solvant est évaporé sous pression réduite (0,1 mm de mercure ; 13,5 Pa) à une température voisine de 50°C. Le résidu est repris dans 100 cm3 d'acétone. Après 15 minutes d'agitation à température ambiante, le solide est séparé par filtration et lavé 2 fois par 40 cm3 au total d'acétone. Le filtrat est évaporé sous pression réduite (20 mm de mercure ; 2,7 kPa) à une température voisine de 50°C. On obtient 2,5 g d'un résidu orangé. Ce produit est chromatographié sur une colonne de 3 cm de diamètre contenant 65 g de silice (0,02-0,045 mm). On élue par un mélange d'acétate d'éthyle et de méthanol (50-50 en volumes) sous une pression de 0,5 bar (51 kPa) en recueillant des fractions de 25 cm3. Les 19 premières fractions sont éliminées. Les 31 fractions suivantes sont réunies et concentrées à sec sous pression réduite (20 mm de mercure ; 2,7 kPa) à une température voisine de 40°C. On obtient ainsi 1,3 g de produit. Ce produit est dissous dans 40 cm3 d'éther éthylique. La solution obtenue est additionnée de 1,65 cm3 d'une solution 4N de gaz chlorhydrique dans l'éther éthylique et agitée à une température voisine de 20°C pendant 15 minutes. Les cristaux obtenus sont séparés par filtration et lavés 4 fois par 50 cm3 au total d'éther éthylique et séchés sous pression réduite (0,1 mm de mercure ; 13,5 Pa) à une température voisine de 60°C. On obtient ainsi 1,35 g de dichlorhydrate de (+)-N-{α-[(diméthylamino-3 propoxyimino) benzyl]-3 phényl} (pyridyl-3)-3 1H,3H-pyrrolo[1,2-c] thiazolecarboxamide-7, (mélange des formes E et Z), sous forme de cristaux crème fondant à 140°C.

Le(+)-N-[(α-hydroxyiminobenzyl)-3 phényl] (pyridyl-3)-3 1H,3H-pyrrolo[1,2-c] thiazolecarboxamide-7 est obtenu comme décrit à l'exemple 1.

### EXEMPLE 4

A une solution de 2,64 g de (+)-N-[(α-hydroxyiminobenzyl)-3 phényl] (pyridyl-3)-3 1H,3H-pyrrolo[1,2-c] thiazolecarboxamide-7, (mélange des formes E et Z) dans 10 cm3 de N,N-diméthylformamide anhydre, on ajoute, à température ambiante, sous courant d'azote et sous agitation 0,86 g d'une dispersion à 50 % d'hydrure de sodium dans l'huile de vaseline, puis, par petites fractions, 1,24 g de chlorhydrate de (chloro-3 propyl)-1 pipéridine en 5 minutes environ. La solution est alors agitée pendant 48 heures à une température voisine de 20°C. Le solvant est évaporé sous pression réduite (20 mm de mercure ; 2,7 kPa). On obtient ainsi 4,85 g d'un résidu orange pâteux qui est chromatographié sur une colonne de 4 cm de diamètre contenant 150 g de silice (0,02-0,045 mm). On élue par un mélange d'acétate d'éthyle et de méthanol (70-30 en volumes) sous une pression de 0,5 bar (51 kPa) en recueillant des fractions de 80 cm3. Les 10 premières fractions sont éliminées. Les 31 fractions suivantes sont réunies et concentrées à sec sous pression réduite (20 mm de mercure ; 2,7 kPa) à une température voisine de 40°C. On obtient ainsi 2,8 g de produit. Ce produit est dissous dans 30 cm3 d'acétate d'éthyle. L'insoluble est séparé par filtration et le filtrat est évaporé sous pression réduite (20 mm de mercure ; 2,7 kPa) à une température voisine de 40°C. Les 2,70 g de produit obtenus sont dissous dans 15 cm3 d'une solution aqueuse d'acide chlorhydrique 1N. La solution obtenue est filtrée puis lyophilisée. Les 3,2 g ainsi obtenus sont dissous dans 50 cm3 d'eau distillée. La solution obtenue est amenée à un pH voisin de 8 par addition de bicarbonate de sodium puis est extraite 3 fois par 50 cm3 au total d'acétate d'éthyle. Les extraits organiques sont réunis, séchés sur du sulfate de magnésium anhydre, filtrés et concentrés sous pression réduite (20 mm de Hg ; 2,7 kPa) à une température voisine de 35°C. On obtient ainsi 2 g d'une meringue beige. Ce produit est chromatographié sur une colonne de 3 cm de diamètre contenant 65 g de silice (0,02-0,045 mm). On élue par de l'acétate d'éthyle sous une pression de 0,5 bar (51 kPa) en recueillant des fractions de 250 cm3. La première fraction est éliminée. Les 12 fractions suivantes sont réunies et concentrées à sec sous pression réduite (20 mm de mercure ; 2,7 kPa) à une température voisine de 40°C. On obtient ainsi 1,3 g de produit que l'on redissout dans 3 cm3 d'acétonitrile. La solution obtenue est additionnée de 20 cm3 d'éther éthylique puis de 2 cm3 d'une solution 4N de gaz chlorhydrique dans l'éther éthylique et agitée à une température voisine de 20°C pendant 15 minutes. Les cristaux obtenus sont séparés par filtration et lavés 4 fois par 50 cm3 au total d'éther éthylique et séchés sous pression réduite (0,1 mm de mercure ; 13,5 Pa) à une température voisine de 50°C. On obtient ainsi 1,3 g de dichlorhydrate de (+)-N-{[α-(pipéridino-3 propoxyimino) benzyl]-3 phényl} (pyridyl-3)-3 1H,3H pyrrolo[1,2-c] thiazolecarboxamide-7 (mélange des formes E et Z), sous forme de cristaux crème fondant à 142°C.

Le(+)-N-[(α-hydroxyiminobenzyl)-3 phényl] (pyridyl-3)-3 1H,3H-pyrrolo[1,2-c] thiazolecarboxamide-7 est obtenu comme décrit à l'exemple 1.

### EXEMPLE 5

A une solution de 5,9 g de (+)-N-(benzoyl-3 phényl) (pyridyl-3)-3 1H,3H-pyrrolo[1,2-c] thiazolecarboxamide-7 dans 100 cm3 de propanol-1 chauffée au reflux, on ajoute sous agitation 3,1 g de dichlorhydrate de morpholino-2 éthoxyamine et 1,5 g de carbonate de sodium. La suspension est agitée pendant 5 heures et 30 minutes au reflux puis ramenée à une température voisine de 20°C. L'insoluble est séparé par filtration et lavé par 20 cm3 de propanol-1. Le filtrat est concentré sous pression réduite (20 mm de mercure ; 2,7 kPa) à une température voisine de 40°C. On obtient ainsi 9 g d'un produit orange pâteux. Ce produit est chromatographié sur une colonne de 6 cm de diamètre contenant 1 kg d'alumine activée acide (0,050-0,200 mm). On élue par un mélange d'acétate d'éthyle et de cyclohexane (50-50 en volumes) sous une pression de 0,5 bar (51 kPa) en recueillant des fractions de 500 cm3. Les 20 premières fractions sont éliminées ; les 10 fractions suivantes sont réunies et concentrées à sec sous pression réduite (20 mm de mercure ; 2,7 kPa) à une température voisine de 40°C. On obtient ainsi 8,1 g de produit. Ce produit est chromatographié sur une colonne de 3 cm de diamètre contenant 125 g de silice (0,02-0,045 mm). On élue par un mélange d'acétate d'éthyle et de méthanol (90-10 en volumes) sous une pression de 0,5 bar (51 kPa) en recueillant des fractions de 25 cm3. Les 19 premières fractions sont éliminées les 7 fractions suivantes sont réunies et concentrées à sec sous pression réduite (20 mm de mercure ; 2,7 kPa) à une température voisine de 40°C. On obtient ainsi 4 g d'une meringue jaune. Le produit est chromatographié sur une colonne de 6 cm de diamètre contenant 65 g d'alumine neutre (0,100-0,125 mm). On élue avec un mélange d'acétate d'éthyle et de cyclohexane (20-80 en volumes) en recueillant des fractions de 300 cm3. Les 3 premières fractions sont éliminées ; les 3 suivantes sont réunies et concentrées à sec sous pression réduite (20 mm de mercure ; 2,7 kPa). On obtient 2 g de produit sous la forme d'une meringue blanche. Ce produit est chromatographié sur une colonne de 4 cm de diamètre contenant 25 g d'alumine neutre (0,100-0,125 mm). On élue avec un mélange d'acétate d'éthyle et de cyclohexane (50-50 en volumes) en recueillant des fractions de 100 cm3. Les 9 premières fractions sont éliminées ; les 7 suivantes sont concentrées à sec sous pression réduite (20 mm de mercure ; 2,7 kPa) à une température voisine de 40°C. On obtient 1,55 g de produit. 850 mg de ce produit sont mis en solution dans 15 cm3 d'une solution aqueuse d'acide méthanesulfonique 0,21N. La solution aqueuse obtenue est lyophilisée. On obtient ainsi 0,98 g de diméthanesulfonate de (+)-N-{[α-(morpholino-2 éthoxyimino) benzyl]-3 phényl} (pyridyl-3)-3 1H,3H-pyrrolo[1,2-c] thiazolecarboxamide-7, (mélange des formes E et Z), sous forme de cristaux jaune clair fondant à 122°C.

Le dichlorhydrate de morpholino-2 éthoxyamine peut être préparé selon D. FAVARA et coll., Il Farmaco, Ed. Sci., 42, 697, 1987.

Le (+)-N-(benzoyl-3 phényl) (pyridyl-3)-3 1H,3H-pyrrolo[1,2-c] thiazolecarboxamide-7 est obtenu comme décrit dans la demande de brevet européen publiée sous le numéro 0 253 711.

### EXEMPLE 6

A une solution de 9,3 g de (+)-N-(benzoyl-3 phényl) (pyridyl-3)-3 1H,3H-pyrrolo[1,2-c] thiazolecarboxamide-7 dans 160 cm3 de propanol-1 chauffée à une température voisine de 75°C, on ajoute, sous agitation, 6,2 g de trichlorhydrate de (méthyl-4 pipérazinyl-1)-3 propoxyamine et 3,8 g de carbonate de sodium. La suspension obtenue est chauffée au reflux pendant 29 heures puis le mélange réactionnel est concentré à sec sous pression réduite (20 mm de mercure ; 2,7 kPa) à une température voisine de 60°C. Le résidu obtenu est repris par un mélange de 300 cm3 d'eau et de 300 cm3 de chlorure de méthylène. La phase organique est séparée et la phase aqueuse est extraite 2 fois par 600 cm3 au total de chlorure de méthylène. Les extraits organiques sont réunis, lavés 2 fois par 600 cm3 au total d'eau distillée, séchés sur du sulfate de magnésium anhydre, filtrés et concentrés à sec sous pression réduite (20 mm de mercure ; 2,7 kPa) à une température voisine de 50°C. On obtient ainsi 14,3 g de produit brut. Ce produit est chromatographié sur une colonne de 6 cm de diamètre contenant 450 g de silice (0,02-0,045 mm). On élue par un mélange de chlorure de méthylène et de méthanol (50-50 en volumes) sous une pression de 0,5 bar (51 kPa) en recueillant des fractions de 500 cm3. Les 7 premières fractions sont éliminées. Les 13 fractions suivantes sont réunies et concentrées à sec sous pression réduite (20 mm de mercure ; 2,7 kPa) à une température voisine de 40°C. On obtient ainsi 7,2 g de produit. Ce produit est réuni à 0,8 g d'un autre échantillon provenant d'une préparation antérieure et est dissous dans 200 cm3 d'acétone. La solution obtenue est ajoutée, à une température voisine de 20°C, à une solution de 2,5 g d'acide oxalique dans 100 cm3 d'acétone. La suspension obtenue est agitée à une température voisine de 20°C pendant 1 heure et 30 minutes ; les cristaux apparus sont séparés par filtration, lavés 3 fois par 150 cm3 au total d'acétone puis 2 fois par 100 cm3 au total d'éther éthylique et enfin séchés sous pression réduite (20 mm de mercure ; 2,7 kPa) à une température voisine de 20°C. On obtient ainsi 9,1 g de dioxalate acide du (+)-N-{(α-[(méthyl-4 pipérazinyl-1)-3 propoxyimino] benzyl}-3 phényl} (pyridyl-3)-3 1H,3H-pyrrolo[1,2-c] thiazolecarboxamide-7, (mélange des formes E et Z), sous forme de cristaux blancs fondant à 190°C.

On dissout 1,16 g de (+)-N-{{α-[(méthyl-4 pipérazinyl-1)-3 propoxyimino] benzyl}-3 phényl} (pyridyl-3)-3 1H,3H-pyrrolo[1,2-c] thiazolecarboxamide-7, sous forme de base, dans 10 cm3 d'acétate d'éthyle. La solution obtenue est traitée par 50 mg de noir décolorant et filtrée. L'insoluble est lavé par 10 cm3 d'acétate d'éthyle. Le filtrat est additionné de 1 cm3 d'éther chlorhydrique 4N et agite à une température voisine de 20°C pendant 15 minutes. Les cristaux apparus sont séparés par filtration, lavés 3 fois par 9 cm3 au total d'acétate d'éthyle et séchés sous pression réduite (0,1 mm de mercure ; 13,5 Pa) à une température voisine de 20°C. On obtient ainsi 1,27 g de dichlorhydrate de (+)-N-{{α-[(méthyl-4 pipérazinyl-1)-3 propoxyimino] benzyl}-3 phényl} (pyridyl-3)-3 1H,3H-pyrrolo[1,2-c] thiazolecarboxamide-7, à l'état de mélange des formes E et Z, sous forme de cristaux crèmes fondant à 150-155°C.

Le (+)-N-(benzoyl-3 phényl) (pyridyl-3)-3 1H,3H-pyrrolo[1,2-c] thiazolecarboxamide-7 est obtenu comme décrit dans la demande de brevet européen publiée sous le numéro 0 253 711.

Le trichlorhydrate de la (méthyl-4 pipérazinyl-1)-3 propoxyamine peut être obtenu de la façon suivante : On chauffe au reflux pendant 3 heures une solution de 5,7 g de N-[(méthyl-4 pipérazinyl-1)-3 propoxy] phtalimide dans 30 cm3 d'acide chlorhydrique 6N. Après 18 heures d'agitation à une température voisine de 20°C, la suspension obtenue est filtrée et le filtrat est concentré à sec sous pression réduite (20 mm de mercure ; 2,7 kPa) à une température voisine de 60°C. On obtient ainsi 6 g de produit brut que l'on remet en suspension dans 30 cm3 d'éthanol. Les cristaux apparus sont séparés par filtration, lavés 3 fois par 45 cm3 au total d'éthanol puis 3 fois par 75 cm3 au total d'éther éthylique et enfin séchés sous pression réduite (20 mm de mercure ; 2,7 kPa) à une température voisine de 20°C. On obtient ainsi 3,4 g de trichlorhydrate de (méthyl-4 pipérazinyl-1)-3 propoxyamine sous forme de cristaux crème fondant à 240°C.

Le N-[(méthyl-4 pipérazinyl)-3 propoxy] phtalimide peut être obtenu de la façon suivante : Une solution de 8,4 g d'(hydroxy-3 propyl)-1 méthyl-4 pipérazine, de 8,2 g de N-hydroxy phtalimide et de 13,1 g de triphénylphosphine dans 120 cm3 de tétrahydrofuranne est refroidie à une température voisine de 0°C et est additionnée en 30 minutes de 10,1 g d'azodicarboxylate d'éthyle. La solution obtenue est agitée à une température voisine de 20°C pendant 18 heures puis est concentrée à sec sous pression réduite (20 mm de mercure ; 2,7 kPa) à une température voisine de 60°C. L'huile brute obtenue est chromatographiée sur une colonne de 5 cm de diamètre contenant 400 g de silice (0,063-0,2 mm). On élue avec des mélanges d'acétate d'éthyle et de méthanol en recueillant des fractions de 500 cm3. Les 5 premières fractions provenant de l'élution par de l'acétate d'éthyle pur et les 5 fractions suivantes provenant de l'élution par un mélange d'acétate d'éthyle et de méthanol (70-30 en volumes) sont éliminées. La fraction suivante provenant de l'élution par un mélange d'acétate d'éthyle et de méthanol (70-30 en volumes), les 5 fractions suivantes provenant de l'élution par un mélange d'acétate d'éthyle et de méthanol (50-50 en volumes) et les 2 fractions suivantes provenant de l'élution par du méthanol pur sont réunies et concentrées à sec sous pression réduite (20 mm de mercure ; 2,7 kPa) à une température voisine de 60°C. On obtient ainsi 11,4 g de N-[(méthyl-4 pipérazinyl-1)-3 propoxy] phtalimide sous forme d'une huile rouge (Rf = 0,2 ; chromatographie sur couche mince de silice éluant : acétate d'éthyle-méthanol 50-50 en volumes).

La séparation des formes E et Z peut être effectuée de la manière suivante : On dissout 50 mg de (+)-N-{{α[(méthyl-4 pipérazinyl-1)-3 propoxyimino] benzyl}-3 phényl} (pyridyl-3)-3 1H, 3H-pyrrolo[1,2-c] thiazolecarboxamide-7, (mélange des formes E et Z), obtenus précédemment dans 2 cm3 d'un mélange d'acétonitrile et d'eau (50-50 en volumes). La solution obtenue est injectée sur une colonne de chromatographie de 25 cm de long et 2 cm de diamètre remplie d'une phase stationnaire de silice greffée en C18 [silice ULTRABASE (nom déposé de la Société Française de Chromatographie sur colonne à Neuilly-Plaisance, France)]. On élue la colonne avec un mélange d'acétonitrile, d'eau et de triéthylamine (40-60-0,5 en volumes) à un débit de 5 cm3 par minute. Les éluats sont analysés par absorptiométrie ultra-violette à 270 nm. Les deux isomères sont détectés aux temps de rétention respectifs de 90 et 110 minutes. Les éluats contenant les produits cherchés sont réunis et concentrés à sec sous pression réduite (20 mm de mercure ; 2,7 kPa) à une température voisine de 40°C et séchés sous pression réduite (1 mm de mercure ; 0,7 kPa) à 20°C pendant une nuit. On obtient ainsi 14 mg de forme A et 18 mg de forme B de (+)-N-{{α-[(méthyl-4 pipérazinyl-1)-3 propoxyimino] benzyl}-3 phényl} (pyridyl-3)-3 1H, 3H pyrrolo [1,2-c] thiazolecarboxamide-7, à l'état de dioxalate acide.

150 mg de forme A du dioxalate acide de (+)-N-{{α-[(méthyl-4 pipérazinyl-1)-3 propoxyimino] benzyl}-3 phényl} (pyridyl-3)-3 1H,3H-pyrrolo [1,2-c] thiazolecarboxamide-7, obtenus par répétition de l'opération précédente, sont mis en solution dans 5 cm3 de soude 1N. La solution est extraite 3 fois par 25 cm3 au total d'acétate d'éthyle. Les extraits organiques sont réunis, séchés sur du sulfate de magnésium anhydre, filtrés et concentrés à sec sous pression réduite (20 mm de mercure ; 2,7 kPa) à une température voisine de 45°C. Les 145 mg de produit obtenus sont dissous dans 5,23 cm3 d'acide chlorhydrique 0,1 N et la solution obtenue est lyophilisée. On obtient ainsi 153 mg de (+)-N-{{α-[(méthyl-4 pipérazinyl-1)-3 propoxyimino] benzyl}-3 phényl} (pyridyl-3)-3 1H, 3H pyrrolo[1,2-c] thiazolecarboxamide-7, forme A, à l'état de dichlorhydrate.

120 mg de forme B du dioxalate acide de (+)-N-{{α-[(méthyl-4 pipérazinyl-1)-3 propoxyimino] benzyl}-3 phényl} (pyridyl-3)-3 1H,3H-pyrrolo[1,2-c] thiazolecarboxamide-7, obtenus par répétition de l'opération précédente, sont mis en solution dans 5 cm3 de soude 1N. La solution est extraite 3 fois par 30 cm3 au total d'acétate d'éthyle. Les extraits organiques sont réunis, séchés sur du sulfate de magnésium anhydre, filtrés et concentrés sous pression réduite (20 mm de mercure ; 2,7 kPa) à une température voisine de 45°C. Les 100 mg de produit obtenus sont dissous dans 3,6 cm3 d'acide chlorhydrique 0,1N et la solution obtenue est lyophilisée. On obtient ainsi 108 mg de(+)-N-{{α-[(méthyl-4 pipérazinyl-1)-3 propoxyimino] benzyl}-3 phényl} (pyridyl-3)-3 1H,3H pyrrolo[1,2-c] thiazolecarboxamide-7, forme B, à l'état de dichlorhydrate.

Les deux formes ont été identifiées par leur spectre R.M.N. du proton (200 MHz, DMSO, d en ppm, J en Hz) avec les spécifications suivantes : S = singulet, D = doublet, T = triplet, Mf = massif, Mt = multiplet, DD = double doublet, DT = double triplet.

### Forme A:

2,2 (Mf, 2H : -CH₂-CH₂-CH₂-) ; 2,86 (S, 3H : .NCH₃) 3,24 (Mf, 2H : -CH₂-CH₂-N, ) ; 3,3 à 3,9 (Mt, 8H : .NCH₂- de la pipérazine); 4,24 (T, J=7, 2H : -OCH₂-) ; 4,45 et 4,63 (D, J=15 et DD, J=15 et 1, 1H chacun : CH₂ du pyrrolothiazole) ; 6,74 (D, J=2,5, 1H : CH en 6 du pyrrolothiazole) ; 6,86 (D, J=1, 1H : CH en 3 du pyrrolothiazole) ; 7,09 (DT, J=8, 1H, aromatique en para du -NH-CO-) ; 7,11 (D, J=2,5, 1H: CH en 5 du pyrrolothiazole) ; 7,4 à 7,6 (Mt, 6H : aromatiques) 7,8 (Mt, 2H : H en 5 de la pyridine et aromatique en ortho du -NH-CO- et du .C=N-) ; 7,92 (D large, J=8, 1H : H en 4 de la pyridine) ; 8,01 (DT, J=8, 1H : aromatique en ortho du -NH-CO-) ; 8,71 (S large, 1H: H en 2 de la pyridine) ; 8,78 (D large, J=5, 1H : H en 6 de la pyridine); 9,84 (S,1H : -CONH-) ; ∼11,8 (Mf étalé, ∼1H : .NHs-).

### Forme B:

2,19 (Mt, 2H : -CH₂-CH₂-CH₂) ; 2,87 (S, 3H : .NCH₃) ; 3,20 (Mf, 2H : -CH₂-CH₂-N,) ; 3,3 à 3,9 (Mt, 8H : .NCH₂- de la pipérazine); 4,23 (T, J=7, 2H : -OCH₂-) ; 4,44 et 4,63 (D, J=15 et DD, J=15 et 1, 1H chacun : CH₂ du pyrrolothiazole) ; 6,73 (D, J=2,5, 1H : CH en 6 du pyrrolothiazole) ; 6,86 (D, J=1, 1H : CH en 3 du pyrrolothiazole) ; 7,08 (D, J=2,5, 1H : CH en 5 du pyrrolo-thiazole) ; 7,08 (Mt, 1H : aromatique en para du -NH-CO-) ; 7,3 à 7,65 (Mt, 6H : aromatiques) ; 7,77 (DD, J=8 et 5, 1H : H en 5 de la pyridine) ; 7,85 à 8,05 (Mt, 3H: aromatiques en ortho du -NH-CO- et en 4 de la pyridine) ; 8,7 (S large, 1H : H en 2 de la pyridine) ; 8,77 (D, J=5, 1H : H en 6 de la pyridine) ; 9,79 (S, 1H : -CONH-) ; ∼11,7 (Mf étalé, ∼1H: .NHs-).

### EXEMPLE 7

A une solution de 9,15 g de (+)-N-(benzoyl-3 phényl) (pyridyl-3)-3 1H,3H-pyrrolo[1,2-c] thiazolecarboxamide-7 dans 110 cm3 de propanol-1 chauffée à une température voisine de 80°C, on ajoute, sous agitation, 3,8 g de dichlorhydrate de diméthylamino-2 éthoxyamine et 2,3 g de carbonate de sodium. La suspension obtenue est chauffée au reflux pendant 8 heures puis, après addition de 0,23 g de carbonate de sodium, pendant 8 heures supplémentaires. Le mélange réactionnel est concentré à sec sous pression réduite (20 mm de mercure ; 2,7 kPa) à une température voisine de 60°C. Après refroidissement, le résidu obtenu est repris par un mélange de 250 cm3 d'eau et de 250 cm3 de chlorure de méthylène. La phase organique est séparée et la phase aqueuse est extraite 2 fois par 500 cm3 au total de chlorure de méthylène. Les extraits organiques sont réunis, lavés 2 fois par 500 cm3 au total d'eau distillée, séchés sur du sulfate de magnésium anhydre, filtrés et concentrés à sec sous pression réduite (20 mm de mercure ; 2,7 kPa) à une température voisine de 50°C. On obtient ainsi 11 g de produit brut. Ce produit est chromatographié sur une colonne de 6 cm de diamètre contenant 450 g de silice (0,02-0,045 mm). On élue par des mélanges de chlorure de méthylène et de méthanol sous une pression de 0,5 bar (51 kPa) en recueillant des fractions de 500 cm3. Les 4 premières fractions provenant de l'élution par un mélange de chlorure de méthylène et de méthanol (90-10 en volumes) sont éliminées. Les 9 fractions suivantes provenant de l'élution par un mélange de chlorure de méthylène et de méthanol (90-10 en volumes) et les 7 fractions suivantes provenant de l'élution par un mélange de chlorure de méthylène et de méthanol (80-20 en volumes) sont réunies et concentrées à sec sous pression réduite (20 mm de mercure ; 2,7 kPa) à une température voisine de 50°C. On obtient ainsi 5,9 g de produit. 4 g de ce produit sont chromatographiés sur une colonne de silice de 6 cm de diamètre contenant 450 g de silice (0,02-0,045 mm). On élue par des mélanges d'acétate d'éthyle et de méthanol sous une pression de 0,5 bar (51 kPa) en recueillant des fractions de 500 cm3. Les 10 premières fractions provenant de l'élution par un mélange d'acétate d'éthyle et de méthanol (50-50 en volumes) sont éliminées; les 16 fractions suivantes provenant de l'élution par du méthanol pur sont réunies et concentrées à sec sous pression réduite (20 mm de mercure ; 2,7 kPa) à une température voisine de 50°C. On obtient ainsi 2,9 g de produit que l'on reprend par 57 cm3 d'une solution aqueuse 0,1N d'acide chlorhydrique. La solution trouble obtenue est amenée à un pH voisin de 8 par addition de bicarbonate de sodium puis est extraite 3 fois par 750 cm3 au total de chlorure de méthylène. Les extraits organiques sont réunis, séchés sur du sulfate de magnésium anhydre, filtrés et concentrés sous pression réduite (20 mm de mercure ; 2,7 kPa) à une température voisine de 50°C. On obtient ainsi 2,2 g de produit que l'on redissout dans 43 cm3 d'une solution aqueuse 0,1N d'acide chlorhydrique. La solution est alors lyophilisée. On obtient ainsi 2,1 g de chlorhydrate de (+)-N-{[α-(diméthylamino-2 éthoxyimino) benzyl]-3 phényl} (pyridyl-3)-3 1H,3H-pyrrolo[1,2-c] thiazolecarboxamide-7, (mélange des formes E et Z), sous forme d'un lyophilisat jaune pâle.

Le (+)-N-(benzoyl-3 phényl) (pyridyl-3)-3 1H,3H-pyrrolo[1,2-c] thiazolecarboxamide-7 est obtenu comme décrit dans la demande de brevet européen publiée sous le numéro 0 253 711.

Le dichlorhydrate de la diméthylamino-2 éthoxyamine peut être obtenu selon la méthode décrite D. FAVARA et coll., Il Farmaco, Ed. Sci., 42, 697 (1987).

### EXEMPLE 8

A une solution de 3 g de (+)-N-(benzoyl-3 phényl) (pyridyl-3)-3 1H,3H-pyrrolo [1,2-c] thiazolecarboxamide-7 dans 50 cm3 de propanol-1 chauffée à une température voisine de 70°C, on ajoute, sous agitation, 1,6 g de dichlorhydrate de morpholino-3 propoxyamine et 0,74 g de carbonate de sodium. La suspension obtenue est chauffée au reflux pendant 8 heures puis le mélange réactionnel est filtré et le filtrat est concentré à sec sous pression réduite (20 mm de mercure ; 2,7 kPa) à une température voisine de 40°C. On obtient ainsi 4,7 g de produit brut. Ce produit est chromatographié sur une colonne de 5 cm de diamètre contenant 500 g de silice (0,02-0,045 mm). On élue par un mélange d'acétate d'éthyle et de méthanol (50-50 en volumes) sous une pression de 0,5 bar (51 kPa) en recueillant des fractions de 500 cm3. Les 26 premières fractions sont éliminées ; les 14 fractions suivantes sont réunies et concentrées à sec sous pression réduite (20 mm de mercure ; 2,7 kPa) à une température voisine de 40°C. Le produit brut obtenu est repris par 20 cm3 d'éthanol. La suspension obtenue est filtrée et le filtrat obtenu est concentré à sec sous pression réduite (20 mm de mercure ; 2,7 kPa) à une température voisine de 60°C. On obtient ainsi 1,08 g de produit que l'on reprend par 9,3 cm3 d'une solution aqueuse 0,205N d'acide méthanesulfonique. La solution trouble est filtrée et le filtrat est lyophilisé. On obtient ainsi 1,2 g de méthanesulfonate du (+)-N-{[α-(morpholino-3 propoxyimino) benzyl]-3 phényl} (pyridyl-3)-3 1H,3H-pyrrolo[1,2-c] thiazolecarboxamide-7, (mélange des formes E et Z), sous forme d'un lyophilisat beige.

Le (+)-N-(benzoyl-3 phényl) (pyridyl-3)-3 1H,3H-pyrrolo[1,2-c] thiazolecarboxamide-7 est obtenu comme décrit dans la demande de brevet européen publiée sous le numéro 0 253 711.

Le dichlorhydrate de la morpholino- 3 propoxyamine peut être obtenu de la façon suivante : On chauffe au reflux pendant 6 heures une solution de 13,8 g de N-(morpholino-3 propoxy) phtalimide dans 50 cm3 d'acide chlorhydrique 6N. Après 18 heures d'agitation à une température voisine de 20°C, la suspension obtenue est filtrée et le filtrat obtenu est concentré à sec sous pression réduite (20 mm de mercure ; 2,7 kPa) à une température voisine de 50°C. On obtient ainsi 6,8 g de produit brut que l'on met en suspension dans 10 cm3 d'éthanol. Les cristaux apparus sont séparés par filtration puis séchés sous pression réduite (20 mm de mercure ; 2,7 kPa) à une température voisine de 20°C. On obtient ainsi 6,1 g de dichlorhydrate de morpholino-3 propoxyamine sous forme de cristaux crème fondant à 160°C.

Le N-(morpholino-3 propoxy) phtalimide peut être obtenu de la façon suivante : Une solution de 27,5 g de morpholino-3 propanol, de 30,2 g de N-hydroxy phtalimide et de 47,2 g de triphénylphosphine dans 400 cm3 de tétrahydrofuranne est refroidie à une température voisine de 0°C et est additionnée en 1 heure et 30 minutes de 37,65 g d'azodicarboxylate d'éthyle. La solution obtenue est agitée à une température voisine de 20°C pendant 18 heures puis est concentrée à sec sous pression réduite (20 mm de mercure ; 2,7 kPa) à une température voisine de 40°C. Le résidu est repris par 500 cm3 d'acétate d'éthyle et la suspension obtenue est filtrée. Le filtrat est extrait par 200 cm3 d'une solution aqueuse d'acide chlorhydrique N. La phase aqueuse est décantée puis extraite 3 fois par 600 cm3 au total d'acétate d'éthyle. La phase aqueuse est amenée à un pH voisin de 10 par addition d'une solution aqueuse de soude N et est extraite 3 fois par 300 cm3 au total d'acétate d'éthyle. Les extraits organiques sont réunis, lavés 3 fois par 150 cm3 au total d'eau distillée, séchés sur du sulfate de magnésium anhydre, filtrés et concentrés à sec sous pression réduite (20 mm de mercure ; 2,7 kPa) à une température voisine de 40°C. On obtient ainsi 25,8 g de produit brut que l'on chromatographie sur une colonne de 8 cm de diamètre contenant 1 kg de silice (0,060 mm). On élue avec de l'acétate d'éthyle pur en recueillant des fractions de 500 cm3. Les 12 premières fractions sont éliminées ; les 48 fractions suivantes sont réunies et concentrées à sec sous pression réduite (20 mm de mercure ; 2,7 kPa) à une température voisine de 40°C. On obtient ainsi 15,5 g de N-(morpholino-3 propoxy) phtalimide sous forme de cristaux blancs fondant à 98C.

Le morpholino-3 propanol peut être obtenue selon O. HROMATKA, Ber., 75, 131, (1942).

### EXEMPLE 9

A une solution de 4,7 g de (+)-N-(benzoyl-3 phényl) (pyridyl-3)-3 1H,3H-pyrrolo[1,2-c] thiazolecarboxamide-7 dans 85 cm3 de propanol-1 chauffée à une température voisine de 70°C, on ajoute, sous agitation, 3 g de trichlorhydrate de (méthyl-4 pipérazinyl-1)-2 éthoxyamine et 1,8 g de carbonate de sodium. La suspension obtenue est chauffée au reflux pendant 10 heures puis le mélange réactionnel est concentré sous pression réduite (20 mm de mercure ; 2,7 kPa) à une température voisine de 40°C. Le résidu obtenu est repris par 100 cm3 d'une solution aqueuse d'acide chlorhydrique N. La solution obtenue est extraite 3 fois par 150 cm3 au total d'éther éthylique, amenée à un pH voisin de 7 par addition d'une solution aqueuse saturée de bicarbonate de sodium et extraite 3 fois par 300 cm3 au total d'éther éthylique. Les extraits éthérés sont réunis, lavés par 100 cm3 d'eau distillée, séchés sur du sulfate de magnésium anhydre, filtrés et concentrés à sec sous pression réduite (20 mm de mercure ; 2,7 kPa) à une température voisine de 40°C. On obtient ainsi 3,1 g de produit brut que l'on chromatographie sur une colonne de 5 cm de diamètre contenant 300 g de silice (0,02-0,045 mm). On élue par un mélange d'acétonitrile et d'ammoniaque (d=0,92) (95-5 en volumes) sous une pression de 0,5 bar (51 kPa) en recueillant des fractions de 200 cm3. Les 10 premières fractions sont éliminées. Les 2 fractions suivantes sont réunies et concentrées à sec sous pression réduite (20 mm de mercure ; 2,7 kPa) à une temperature voisine de 40°C. On obtient ainsi 1,55 g de produit. Ce produit est redissous dans 5 cm3 d'acétone. La solution obtenue est ajoutée, à une température voisine de 20°C, à une solution de 0,46 g d'acide oxalique dans 10 cm3 d'acétone. La suspension obtenue est agitée à une température voisine de 20°C pendant 30 minutes puis les cristaux apparus sont séparés par filtration, lavés 2 fois par 5 cm3 au total d'acétone, séchés sous pression réduite (20 mm de mercure ; 2,7 kPa) à une température voisine de 20°C. On obtient ainsi 1,7 g de dioxalate acide du (+)-N-{{α-[(méthyl-4 pipérazinyl-1)-2 éthoxyimino] benzyl}-3 phényl} (pyridyl-3)-3 1H,3H-pyrrolo[1,2-c] thiazolecarboxamide-7 (mélange des formes E et Z), sous forme de cristaux blancs fondant à 172°C.

Le (+)-N-(benzoyl-3 phényl) (pyridyl-3)-3 1H,3H-pyrrolo [1,2-c] thiazolecarboxamide-7 est obtenu comme décrit dans la demande de brevet européen publiée sous le numéro 0 253 711.

Le trichlorhydrate de la (méthyl-4 pipérazinyl-1)-2 éthoxyamine peut être obtenu selon la méthode décrite par R. CRICCHIO et coll., J. Med. Chem., 17, 396 (1974).

### EXEMPLE 10

A une solution de 3 g de (+)-N-(benzoyl-3 phényl) (pyridyl-3)-3 1H,3H-pyrrolo[1,2-c] thiazolecarboxamide-7 dans 50 cm3 de propanol-1 chauffée à une température voisine de 70°C, on ajoute 2,1 g de dichlorhydrate de (méthyl-1 pipéridyl-4) oxyamine, à l'état de dichlorhydrate, et 0,72 g de carbonate de sodium. La suspension obtenue est chauffée à une température voisine de 90°C pendant 2 heures et 30 minutes puis est agitée à une température voisine de 20°C pendant 18 heures et enfin filtrée. Le filtrat est concentré sous pression réduite (20 mm de mercure ; 2,7 kPa) à une température voisine de 35°C. On obtient ainsi 6,1 g de produit brut que l'on reprend par 60 cm3 d'acétonitrile. Les cristaux obtenus sont séparés par filtration et repris par un mélange de 50 cm3 de soude N et de 20 cm3 d'acétate d'éthyle. La phase organique est décantée et la phase aqueuse est extraite 2 fois par 40 cm3 au total d'acétate d'éthyle. Les extraits organiques sont réunis, lavés 3 fois par 45 cm3 au total d'eau distillée, séchés sur du sulfate de magnésium anhydre, filtrés et concentrés à sec sous pression réduite (20 mm de mercure ; 2,7 kPa) à une température voisine de 35°C. Les 3,2 g de produit obtenus sont chromatographiés sur une colonne de 4 cm de diamètre contenant 35 g de silice (0,02-0,045 mm). On élue par 200 cm3 d'un mélange d'acétate d'éthyle et de méthanol (60-30 en volumes). La fraction obtenue est concentrée à sec sous pression réduite (20 mm de mercure ; 2,7 kPa) à une température voisine de 35°C. On obtient ainsi 1,25 g de produit que l'on redissout dans 5 cm3 d'acétate d'éthyle. On ajoute alors à la solution 0,42 cm3 d'une solution 5,6N de gaz chlorhydrique dans l'éther éthylique. Les cristaux apparus sont séparés par filtration et séchés sous pression réduite (20 mm de mercure ; 2,7 kPa) à une température voisine de 20°C. On obtient ainsi 0,82 g de produit que l'on redissout dans 20 cm3 d'eau distillée. La solution obtenue est amenée à un pH voisin de 10 par addition d'une solution aqueuse de soude 2N puis est extraite 3 fois par 30 cm3 au total d'acétate d'éthyle. Les extraits organiques sont réunis, lavés 2 fois par 20 cm3 au total d'eau distillée, séchés sur du sulfate de magnésium anhydre, filtrés et concentrés à sec sous pression réduite (20 mm de mercure ; 2,7 kPa) à une température voisine de 40°C. Les 0,65 g obtenus sont réunis à 0,55 g de produit obtenus précédemment selon la même méthode et sont chromatographiés sur une colonne de 4 cm de diamètre contenant 300 g de silice (0,02-0,045 mm). On élue par un mélange d'acétate d'éthyle et de diéthylamine (95-5 en volumes) sous une pression de 0,5 bar (51 kPa) en recueillant des fractions de 500 cm3. Les 7 premières fractions sont éliminées. Les 5 fractions suivantes sont réunies et concentrées à sec sous pression réduite (20 mm de mercure ; 2,7 kPa) à une température voisine de 40°C. On obtient ainsi 880 mg de produit que l'on dissout dans 16,4 cm3 d'une solution aqueuse d'acide chlorhydrique 0,1 N. La solution obtenue est extraite 2 fois par 40 cm3 au total d'éther éthylique et la phase aqueuse est amenée à un pH voisin de 10 par addition d'une solution aqueuse de soude N puis est extraite 3 fois par 60 cm3 au total d'acétate d'éthyle. Les extraits organiques sont réunis, lavés 2 fois par 20 cm3 au total d'eau distillée, séchés sur du sulfate de magnésium anhydre, filtrés et concentrés à sec sous pression réduite (20 mm de mercure ; 2,7 kPa) à une température voisine de 40°C. On obtient ainsi 630 mg de produit. 530 mg de ce produit sont dissous dans 5 cm3 d'acétone. La solution obtenue est ajoutée à une solution de 0,17 g d'acide oxalique dans 5 cm3 d'acétone. Les cristaux apparus sont séparés par filtration, lavés par 2 cm3 d'acétone puis par 2 cm3 d'un mélange d'acétone et d'éther éthylique (50-50 en volumes), et enfin séchés sous pression réduite (20 mm de mercure ; 2,7 kPa) à une température voisine de 20°C. On obtient ainsi 570 mg de dioxalate acide du (+)-N-{{α-[(méthyl-1 pipéridyl-4) oxyimino] benzyl}-3 phényl} (pyridyl-3)-3 1H,3H-pyrrolo [1,2-c] thiazolecarboxamide-7 (mélange des formes E et Z), sous forme de cristaux crème, fondant à 170°C.

On dissout 1,0 g de (+)-N-{{α-[(méthyl-1 pipéridyl-4) oxyimino] benzyl}-3 phényl} (pyridyl-3)-3 1H,3H-pyrrolo[1,2-c] thiazolecarboxamide-7, sous forme de base, dans 4 cm3 d'éthanol absolu. La solution obtenue est additionnée de 0,93 cm3 d'éther chlorhydrique 4N et diluée par 20 cm3 d'acétate d'éthyle puis 100 cm3 d'éther diéthylique. Après 15 heures d'agitation à une température voisine de 20°C, les cristaux apparus sont séparés par filtration, lavés 4 fois par 20 cm3 au total d'éther diéthylique et séchés à l'air à une température voisine de 20°C. On obtient ainsi 1,1 g de dichlorhydrate de (+)-N-{{α-[(méthyl-1 pipéridyl-4) oxyimino] benzyl}-3 phényl} (pyridyl-3)-3 1H,3H-pyrrolo[1,2-c] thiazolecarboxamide-7, à l'état de mélange des formes E et Z, sous forme de cristaux crèmes fondant à 200-205°C.

Le (+)-N-(benzoyl-3 phényl) (pyridyl-3)-3 1H,3H-pyrrolo[1,2-c] thiazolecarboxamide-7 est obtenu selon la méthode décrite dans la demande de brevet européen publiée sous le N° 0 253 711.

Le dichlorhydrate de (méthyl-1 pipéridyl-4) oxyamine peut être obtenu de la façon suivante : On chauffe au reflux pendant 3 heures une solution aqueuse de 18,7 g de N-(méthyl-1 pipéridinyl-4 oxy) phtalimide dans 100 cm3 d'une solution aqueuse d'acide chlorhydrique 6N. La suspension obtenue est filtrée et le filtrat est concentré à sec sous pression réduite (20 mm de mercure ; 2,7 kPa) à une température voisine de 45°C. Le produit obtenu est mis en suspension dans 100 cm3 d'éthanol et les cristaux apparus sont séparés par filtration, lavés 2 fois par 100 cm3 au total d'éthanol et séchés sous pression réduite (20 mm de mercure ; 2,7 kPa) à une température voisine de 20°C. On obtient ainsi 11,2 g de dichlorhydrate de la (méthyl-1 pipéridyl-4) oxyamine sous forme de cristaux blancs fondant à 220°C.

Le N-(méthyl-1 pipéridyl-4 oxy) phtalimide peut être obtenu de la façon suivante : A une suspension de 30,6 g d'hydroxy-4 méthyl-1 pipéridine, de 43,7 g de N-hydroxyphtalimide et de 68,3 g de triphényl phosphine dans 450 cm3 de tétrahydrofuranne, on ajoute en 90 minutes, à une température voisine de 0°C, 53,8 g d'azodicarboxylate de diéthyle. La suspension obtenue est agitée à une température voisine de 20°C pendant 18 heures puis le mélange réactionnel est concentré à sec sous pression réduite (20 mm de mercure ; 2,7 kPa) à une température voisine de 40°C. Le produit obtenu est repris par 500 cm3 d'acétate d'éthyle et la suspension obtenue est filtrée. Le filtrat est concentré à sec sous pression réduite (20 mm de mercure ; 2,7 kPa) à une température voisine de 40°C. Le produit obtenu est redissous dans 500 cm3 d'une solution aqueuse d'acide chlorhydrique N. La suspension obtenue est filtrée et le filtrat est extrait 3 fois par 300 cm3 au total d'éther éthylique, amené à un pH voisin de 10 par addition de 500 cm3 d'une solution aqueuse de soude N. Les cristaux apparus sont séparés par filtration, lavés 3 fois par 300 cm3 au total d'eau distillée et séchés sous pression réduite (20 mm de mercure ; 2,7 kPa) à une température voisine de 20°C. On obtient ainsi 19 g de N-(méthyl-1 pipéridyl-4 oxy) phtalimide sous forme de cristaux beiges fondant à 90°C.

### EXEMPLE 11

A une solution de 3 g de (+)-N-[(α-hydroxyiminobenzyl)-3 phényl] (pyridyl-3)-3 1H,3H-pyrrolo[1,2-c] thiazolecarboxamide-7 (mélange des formes E et Z) dans 15 cm3 de N,N-diméthylformamide anhydre, on ajoute, à température ambiante, sous courant d'azote et sous agitation, 0,36 g d'une dispersion à 50 % d'hydrure de sodium dans l'huile de vaseline. On ajoute ensuite une solution de 2,61 g de para-toluènesulfonate de (benzyl éthylamino)-3 propanol dans 13 cm3 de N,N-diméthylformamide anhydre en 5 minutes environ puis agite la solution pendant 12 heures à une température voisine de 20°C. On ajoute alors 250 cm3 d'eau distillée et la solution est extraite 3 fois par 240 cm3 au total d'acétate d'éthyle. Les extraits organiques sont réunis, lavés par 80 cm3 d'une solution aqueuse d'acide chlorhydrique 0,1N. La phase aqueuse est ensuite amenée à pH 8 par addition d'une solution aqueuse de soude 1N et la solution est extraite 3 fois par 240 cm3 au total d'acétate d'éthyle. Les extraits organiques sont réunis, séchés sur du sulfate de magnésium anhydre, filtrés et concentrés à sec sous pression réduite (20 mm de mercure ; 2,7 kPa) à une température voisine de 45°C. On obtient ainsi 1,61 g d'un résidu marron pâteux. Ce produit est chromatographié sur une colonne de 4 cm de diamètre contenant 200 g d'alumine neutre (0,032-0,063 mm). On élue par 2,8 litres d'acétate d'éthyle puis par 750 cm3 d'un mélange d'acétate d'éthyle et de méthanol (95-5 en volumes) sous une pression de 0,5 bar (51 kPa) en recueillant des fractions de 50 cm3. Les 27 premières fractions sont éliminées. Les 39 fractions suivantes sont réunies et concentrées à sec sous pression réduite (20 mm de mercure ; 2,7 kPa) à une température voisine de 40°C. On obtient ainsi 1 g de produit que l'on dissout dans 5 cm3 d'acétate d'éthyle. La solution est additionnée de 0,1 g de noir décolorant puis filtrée à chaud et le filtre est rincé par 5 cm3 d'acétate d'éthyle. A cette solution, on ajoute 0,8 cm3 d'une solution 4N de gaz chlorhydrique dans l'éther éthylique et on agite à une température voisine de 20°C pendant 15 minutes. Les cristaux obtenus sont séparés par filtration, lavés 4 fois par 50 cm3 au total d'éther éthylique et séchés sous pression réduite (0,1 mm de mercure ; 13,5 Pa) à une température voisine de 60°C. On obtient ainsi 0,96 g de dichlorhydrate de (+)-N-{{α-[(benzyl éthylamino)-3 propoxyimino] benzyl}-3 phényl} (pyridyl-3)-3 1H,3H-pyrrolo[1,2-c] thiazolecarboxamide-7, à l'état de mélange des formes E et Z, sous forme de cristaux crème fondant à 175°C.

Le (+)-N-(benzoyl-3 phényl) (pyridyl-3)-3 1H,3H-pyrrolo [1,2-c] thiazolecarboxamide-7 est obtenu comme décrit dans la demande de brevet européen publiée sous le numéro 0 253 711.

Le tosylate du (benzyl éthylamino)-3 propanol peut être préparé de la façon suivante : A une solution de 3,86 g de (benzyl éthylamino)-3 propanol dans 10 cm3 de pyridine, on ajoute, sous agitation, en 20 minutes environ, à 20°C, une solution de 3,8 g de chlorure de para-toluènesulfonyle dans 10 cm3 de pyridine et le mélange réactionnel est agité pendant 20 minutes à une température voisine de 20°C. La solution est additionnée de 200 cm3 d'eau distillée puis est extraite 2 fois par 160 cm3 au total d'acétate d'éthyle. Les extraits organiques sont réunis, lavés par 200 cm3 au total d'eau distillée, puis séchés sur du sulfate de magnésium anhydre, filtrés et concentrés à sec sous pression réduite (20 mm de mercure ; 2,7 kPa) à une température voisine de 40°C. On obtient 4,78 g de paratoluènesulfonate de (benzyl éthylamino)-3 propanol, sous la forme d'une huile orangée employée brute dans les synthèses ultérieures.

Le (benzyl éthylamino)-3 propanol peut être préparé de la façon suivante : Une solution de 6,76 g de benzyl éthylamine, 6,15 cm3 d'acétate de chloro-3 propyle et de 5,3 g de carbonate de sodium dans 25 cm3 de propanol-2 est chauffée au reflux, sous agitation pendant 48 heures puis refroidie à une température voisine de 20°C. Le chlorure de sodium formé est séparé par filtration et le solvant est évaporé à sec sous pression réduite (20 mm de mercure ; 2,7 kPa) à une température voisine de 45°C. On obtient 17,4 g d'une huile orangée que l'on reprend par 30 cm3 d'eau distillée et 30 cm3 d'une solution aqueuse d'acide chlorhydrique 10N. La solution obtenue est chauffée pendant 6 heures au reflux puis refroidie à une température voisine de 20°C. On ajoute 200 mg de noir décolorant. La solution est filtrée et le filtre est rincé avec 50 cm3 au total d'eau distillée. On ajoute 40 cm3 d'ammoniaque (d=0,92) et extrait 3 fois par 240 cm3 au total d'acétate d'éthyle. Les extraits organiques sont réunis, séchés sur sulfate de magnésium anhydre, filtrés et concentrés à sec sous pression réduite (20 mm de mercure ; 2,7 kPa) à une température voisine de 45°C. Le résidu est distillé sous pression réduite. On obtient ainsi 5,38 g de N-éthyl N-(acétoxy-3 propyl) benzylamine bouillant à 95°C sous 0,2 mm de mercure (13,4 Pa).

### EXEMPLE 12

A 0,62 g d'une suspension à 50 % d'hydrure de sodium dans l'huile de vaseline, on ajoute sous azote et en 45 minutes, à une température voisine de 20°C, une solution de 4 g de N-[(α-hydroxyiminobenzyl)-3 phényl] (pyridyl-3)-3 1H,3H-pyrrolo[1,2-c] thiazolecarboxamide-7 (mélange des formes E et Z) dans 40 cm3 de N,N-diméthylformamide anhydre. A la solution obtenue on ajoute en 40 minutes, à une température voisine de 18°C, une solution de 1,77 g de dichlorhydrate de N-(chloro-2 éthyl) morpholine dans 40 cm3 de N,N-diméthylformamide anhydre. La suspension obtenue est agitée à une température voisine de 20°C pendant 18 heures. On rajoute alors 0,21 g d'une suspension à 50 % d'hydrure de sodium dans l'huile de vaseline puis 0,85 g de chlorhydrate de N-(chloro-2 éthyl) morpholine. La suspension obtenue est chauffée à une température voisine de 65°C pendant 20 heures puis, après refroidissement à une température voisine de 20°C, est additionnée de 650 cm3 d'eau distillée et extraite 3 fois par 300 cm3 au total d'acétate d'éthyle. Les extraits organiques sont réunis, extraits par 100 cm3 d'une solution aqueuse d'acide chlorhydrique 0,1N et la phase aqueuse est extraite 2 fois par 100 cm3 au total d'éther éthylique. La phase aqueuse est amenée à un pH voisin de 10 par addition d'une solution aqueuse de soude N et extraite 3 fois par 300 cm3 d'acétate d'éthyle. Les extraits organiques sont réunis, lavés 3 fois par 150 cm3 au total d'eau distillée, séchés sur du sulfate de magnésium anhydre, filtrés et concentrés à sec sous pression réduite (20 mm de mercure ; 2,7 kPa) à une température voisine de 30°C. On obtient 5 g de produit que l'on chromatographie sur une colonne de 5 cm de diamètre contenant 600 g de silice (0,02-0,045 mm). On élue par un mélange d'acétate d'éthyle et de méthanol (90 -10 en volumes) sous une pression de 0,5 bar (51 kPa) en recueillant des fractions de 500 cm3. Les 8 premières fractions sont éliminées. Les 32 fractions suivantes sont réunies et concentrées à sec sous pression réduite (20 mm de mercure ; 2,7 kPa) à une température voisine de 40°C. On obtient ainsi 3 g de produit que l'on redissout dans 30 cm3 d'acétate d'éthyle. La solution obtenue est additionnée de 1,95 cm3 d'une solution 5,6 N de gaz chlorhydrique dans l'éther éthylique. Les cristaux apparus sont séparés par filtration, lavés 2 fois par 10 cm3 au total d'acétate d'éthyle et séchés sous pression réduite (20 mm de mercure ; 2,7 kPa) à une température voisine de 20°C. Les 2,8 g de produit obtenus sont dissous dans 30 cm3 d'eau distillée et la solution résultante est amenée à un pH voisin de 10 par addition d'une solution aqueuse de soude N, extraite 3 fois par 60 cm3 d'acétate d'éthyle. Les extraits organiques sont réunis, lavés 2 fois par 20 cm3 au total d'eau distillée, séchés sur du sulfate de magnésium anhydre, filtrés et concentrés à sec sous pression réduite (20 mm de mercure ; 2,7 kPa) à une température voisine de 35°C. On obtient ainsi 1,77 g de produit. Ce produit est dissous dans 23,2 cm3 d'un solution aqueuse d'acide chlorhydrique 0,13N et la solution obtenue est lyophilisée. On obtient ainsi 1,64 g de N-{{α-[(morpholino-2 éthoxyimino] benzyl}-3 phényl} (pyridyl-3)-3 1H,3H-pyrrolo[1,2-c] thiazolecarboxamide-7 (mélange des formes E et Z), sous forme d'un lyophilisat crème.

Le N-[(α-hydroxyiminobenzyl)-3 phényl] (pyridyl-3)-3 1H,3H-pyrrolo[1,2-c] thiazolecarboxamide-7 (mélange des formes E et Z) peut être obtenu de la façon suivante : A une solution de 23 g de N-(benzoyl-3 phényl) (pyridyl-3)-3 1H,3H-pyrrolo[1,2-c] thiazolecarboxamide-7 dans 270 cm3 de propanol-1 chauffée à une température voisine de 70°C, on ajoute 7,5 g de chlorhydrate d'hydroxylamine et 5,7 g de carbonate de sodium. La suspension obtenue est chauffée au reflux pendant 3 heures et agitée à une température voisine de 20°C pendant 18 heures. Les cristaux apparus sont séparés par filtration, lavés 3 fois par 150 cm3 au total d'eau distillée et séchés sous pression réduite (20 mm de mercure ; 2,7 kPa) à une température voisine de 20°C. On obtient ainsi 8,3 g de N-[(α-hydroxyiminobenzyl)-3 phényl] (pyridyl-3)-3 1H,3H-pyrrolo[1,2-c] thiazolecarboxamide-7 (mélange des formes E et Z), sous forme de cristaux crème fondant à 210°C.

Le N-(benzoyl-3 phényl) (pyridyl-3)-3 1H,3H-pyrrolo[1,2-c] thiazolecarboxamide-7 peut être obtenu selon la méthode décrite dans la demande de brevet européen publiée sous le N° 0 253 711.

### EXEMPLE 13

A une solution de 1,63 g de (+)-N-[(α-hydroxyiminobenzyl)-3 phényl] (pyridyl-3)-3 1H,3H-pyrrolo [1,2-c] thiazolecarboxamide-7 (mélange des formes E et Z) dans 16 cm3 de N,N-diméthylformamide anhydre, on ajoute, à température ambiante, sous courant d'azote et sous agitation 0,22 g d'une dispersion à 50 % d'hydrure de sodium dans l'huile de vaseline. On ajoute ensuite une solution de 0,9 g de dichlorhydrate de (chloro-2 éthyl)-1 benzyl-4 pipérazine dans 5 cm3 de N,N-diméthylformamide anhydre en 5 minutes environ. La solution est alors agitée pendant 20 heures à température ambiante et concentrée à sec sous pression réduite (20 mm de mercure 2,7 kPa) à une température voisine de 40°C. Le résidu pâteux est trituré dans 40 cm3 d'acétone, le chlorure de sodium formé est séparé par filtration et lavé par 15 cm3 au total d'acétone et le filtat est évaporé sous pression réduite (20 mm de mercure ; 2,7 kPa) à une température voisine de 40°C. On obtient ainsi 3,1 g d'un résidu orange pâteux. Ce produit est chromatographié sur une colonne de 3 cm de diamètre contenant 65 g de silice (0,02-0,045 mm). On élue par un mélange d'acétate d'éthyle sous une pression de 0,5 bar (51 kPa) en recueillant des fractions de 90 cm3. Les 15 premières fractions sont éliminées ; les 19 fractions suivantes sont réunies et concentrées à sec sous pression réduite (20 mm de mercure ; 2,7 kPa) à une température voisine de 40°C. On obtient ainsi 1,45 g de produit. Ce produit est dissous dans 5 cm3 d'acétate d'éthyle. La solution obtenue est additionnée de 10 cm3 d'éther éthylique et de 2 cm3 d'une solution 4 N de gaz chlorhydrique dans l'éther éthylique, puis diluée avec 20 cm3 d'éther éthylique et agitée à une température voisine de 20°C pendant 15 minutes. Les cristaux obtenus sont séparés par filtration et lavés 4 fois par 40 cm3 au total d'éther éthylique et séchés sous pression réduite (0,1 mm de mercure ; 13,5 Pa) à une température voisine de 20°C. On obtient ainsi 1,5 g de dichlorhydrate de (+)-N-{{α-[(benzyl-4 pipérazinyl-1)-2 éthoxyimino] benzyl}-3 phényl} (pyridyl-3)-3 1H,3H-pyrrolo[1,2-c] thiazolecarboxamide-7 (mélange des formes E et Z), sous forme de cristaux blancs fondant à 186°C.

Le (+)-N-[(α-hydroxyiminobenzyl)-3 phényl] (pyridyl-3)-3 1H,3H-pyrrolo[1,2-c] thiazolecarboxamide-7 est obtenu comme décrit à l'exemple 1.

Le dichlorhydrate de la benzyl-1 (chloro-2 éthyl-1))-4 pipérazine peut être obtenu selon la méthode décrite par D.C. KRIESEL, O. GRISVOLD, J. Pharm. Sci., 56, 325 (1967).

### EXEMPLE 14

A une solution de 3,0 g de (+)-N-[(α-hydroxyiminobenzyl)-3 phényl] (pyridyl-3)-3 1H,3H-pyrrolo[1,2-c] thiazolecarboxamide-7 (mélange des formes E et Z) dans 22 cm3 de N,N-diméthylformamide anhydre, on ajoute, à température ambiante, sous courant d'azote et sous agitation, 0,68 g d'hydrure de sodium (en dispersion à 50 % dans l'huile de vaseline). Après 15 minutes d'agitation à température ambiante, on ajoute 0,9 g de chlorhydrate de (chloro-2 éthyl) méthylamine et l'agitation à température ambiante est maintenue pendant 15 heures puis on verse le milieu réactionnel dans 250 cm3 d'eau distillée. Après 1 heure d'agitation à température ambiante, le solide est séparé par filtration, lavé à l'eau distillée jusqu'à neutralité puis dissous dans 16 cm3 d'acétate d'éthyle et filtré. On ajoute alors 1,57 cm3 d'éther chlorhydrique 4,1N et après 1 heure sous agitation à température ambiante, les cristaux sont séparés par filtration, lavés par 6 cm3 d'acétate d'éthyle et 40 cm3 d'éther diéthylique et séchés sous pression réduite (0,1 mm de mercure ; 13,5 Pa) à une temperature voisine de 20°C. Le chlorhydrate obtenu (3,04 g) est dissous dans 30 cm3 d'eau distillée, 6 cm3 de soude 1N sont ajoutés et on extrait 2 fois par 40 cm3 au total d'acétate d'éthyle. Les extraits organiques sont réunis, lavés par 15 cm3 d'eau distillée, séchés sur du sulfate de magnésium anhydre, filtrés et concentrés sous pression réduite (20 mm de mercure ; 2,7 kPa) à une température voisine de 40°C. On obtient une meringue ocre (2,4 g) qui est chromatographiée sur une colonne de 4 cm de diamètre contenant 150 g environ de silice (0,02-0,045 mm). On élue par un mélange d'acétonitrile et d'ammoniaque (d= 0,92) (95-5 en volumes) sous une pression de 0,5 bar (51 kPa) en recueillant des fractions de 80 cm3. Les 14 premières fractions sont éliminées. Les 3 fractions suivantes sont réunies et concentrées à sec sous pression réduite (20 mm de mercure ; 2,7 kPa) à une temperature voisine de 45°C. On obtient ainsi 1,47 g de produit. Ce produit est dissous dans 9 cm3 d'acétate d'éthyle, traité par 0,2 g de noir décolorant, filtré et additionné de 1,50 cm3 d'éther chlorhydrique 4N puis de 15 cm3 d'éther diéthylique. Les cristaux apparus sont séparés par filtration, lavés par 15 cm3 au total d'éther diéthylique et séchés sous pression réduite (0,1 mm de mercure ; 13,5 Pa) à une température voisine de 40°C. On obtient ainsi 1,6 g de chlorhydrate de (+)-N-{[α-(méthylamino-2 éthoxyimino) benzyl]-3 phényl} (pyridyl-3)-3 1H,3H-pyrrolo[1,2-c] thiazolecarboxamide-7, à l'état de mélange des formes E et Z, sous forme de cristaux blancs fondant à 150-155°C.

Le(+)-N-[(α-hydroxyiminobenzyl)-3 phényl] (pyridyl-3)-3 1H,3H-pyrrolo[1,2-c] thiazolecarboxamide-7 (mélange des formes E et Z) est obtenu comme décrit à l'exemple 1.

### EXEMPLE 15

A une solution de 1 g de (+)-N-{{α-[(méthyl-1 pipéridyl-4) oxyimino] benzyl}-3 phényl} (pyridyl-3)-3 1H,3H-pyrrolo[1,2-c] thiazolecarboxamide-7, (mélange des formes E et Z), dans 20 cm3 d'acide acétique à 100 %, sont ajoutés successivement 3,8 cm3 d'une solution éthanolique 1N d'acide méthanesulfonique, puis 0,2 cm3 d'eau oxygénée à 30 % (110 volumes) ; l'agitation à température ambiante est maintenue pendant 15 heures et le solvant est concentré sous pression réduite (0,1 mm de mercure ; 13,5 Pa) à une température voisine de 30°C. Le résidu est dissous dans 25 cm3 d'eau distillée, alcalinisé par 8 cm3 de soude (d=1,33) et dilué par 15 cm3 d'acétone et 30 cm3 d'acétate d'éthyle ; la phase organique est décantée, la phase aqueuse est extraite par 30 cm3 d'acétate d'éthyle. Les extraits organiques sont réunis, lavés 2 fois par 40 cm3 au total d'eau distillée, séchés sur du sulfate de magnésium anhydre et évaporés à sec sous pression réduite (20 mm de mercure ; 2,7 kPa) à une température voisine de 45°C. On obtient ainsi 814 mg d'une meringue rosée. Ce produit est chromatographié sur une colonne de 3 cm de diamètre contenant 50 g de silice environ (0,02-0,045 mm). On élue par un mélange d'acétonitrile et d'ammoniaque (d=0,92) (95-5 en volumes) sous une pression de 0,5 bar (51 kPa) en recueillant des fractions de 25 cm3. Les 10 premières fractions sont éliminées. Les 4 fractions suivantes sont réunies et concentrées à sec sous pression réduite (20 mm de mercure ; 2,7 kPa) à une température voisine de 40°C. On obtient ainsi 430 mg de produit. Ce produit est dissous dans 3 cm3 d'acétate d'éthyle, traité par 100 mg de noir décolorant, filtré et rincé par 1,5 cm3 au total d'acétate d'éthyle. La solution obtenue est additionnée de 0,47 cm3 d'éther chlorhydrique 3,25N et agitée à une température voisine de 20°C pendant 30 minutes. On ajoute 9 cm3 d'éther diéthylique et maintient l'agitation pendant 1 heure supplémentaire à température ambiante puis les cristaux sont séparés par filtration et lavés par 10 cm3 au total d'éther diéthylique et séchés à l'air à une température voisine de 20°C. On obtient ainsi 460 mg de dichlorhydrate de (+)-N-{{α-[(méthyl-1 pipéridyl-4) oxyimino] benzyl}-3 phényl} (pyridyl-3)-3 1H,3H-pyrrolo[1,2-c] thiazolecarboxamide-7 oxyde-2-(2RS,3R), à l'état de mélange des formes E et Z, fondant à 200°C.

Le (+)-N-{{α-[(méthyl-1 pipéridyl-4) oxyimino] benzyl}-3 phényl} (pyridyl-3)-3 1H,3H-pyrrolo[1,2-c] thiazolecarboxamide-7, à l'état de mélange des formes E et Z, peut être préparé comme dans l'exemple 10.

### EXEMPLE 16

Une solution de 4,24 g de (+)-N-{{α-[(tert-butyloxy carbonyl-4 pipérazinyl-1)-3 propoxyimino] benzyl}-3 phényl} (pyridyl-3)-3 1H,3H-pyrrolo[1,2-c] thiazolecarboxamide-7 (mélange des formes E et Z) dans 100 cm3 d'acétate d'éthyle est agitée, en présence de 8 cm3 d'éther chlorhydrique 3,2N, pendant 5 heures à température ambiante puis est filtrée. Le solide obtenu, lavé par 10 cm3 d'éther diéthylique et 15 cm3 d'acétate d'éthyle est ensuite dissous dans 50 cm3 d'une solution à 10 % de carbonate de sodium et extrait 3 fois par 150 cm3 au total de dichlorométhane. Les extraits organiques sont lavés par 50 cm3 de saumure à 250 g/l, séchés sur du sulfate de magnésium anhydre et concentrés à sec sous pression réduite (20 mm de mercure ; 2,7 kPa). Les 3,6 g obtenus sont mis en suspension dans 50 cm3 d'éther diéthylique, additionnés de 25 cm3 d'éther chlorhydrique 3N, dilués par 20 cm3 d'acétate d'éthyle et agités pendant 4 heures et trente minutes à température ambiante. Les cristaux apparus sont séparés par filtration, lavés par 20 cm3 d'éther diéthylique, puis dissous dans 50 cm3 d'eau distillée, alcalinisés à pH 9 avec une solution à 10 % de carbonate de sodium, et extraits par 450 cm3 au total de dichlorométhane. Les phases organiques sont séchées sur sulfate de magnésium anhydre et évaporées à sec sous pression réduite (20 mm de mercure ; 2,7 kPa) à une températutre voisine de 40°C. On obtient ainsi 2,7 g d'une meringue rosée. Ce produit est chromatographié sur une colonne de 1,8 cm de diamètre contenant 50 g d'alumine neutre (0,032-0,063 mm) désactivée par 10 % d'eau. On élue par un mélange d'acétate d'éthyle et de méthanol (80-20 en volumes) sous une pression de 0,5 bar (51 kPa) en recueillant des fractions de 20 cm3. Les 3 premières fractions sont éliminées. Les 17 fractions suivantes sont réunies et concentrées à sec sous pression réduite (20 mm de mercure ; 2,7 kPa) à une température voisine de 40°C. On obtient ainsi 1,82 g d'une meringue beige, qui additionnée de 0,4 g provenant d'une autre opération est chromatographié une deuxième fois sur une colonne de 1,4 cm de diamètre contenant 20 g d'alumine neutre (0,032-0,063 mm) désactivée par 10 % d'eau. On élue par 300 cm3 d'acétate d'éthyle, 180 cm3 d'un mélange d'acétate d'éthyle et de méthanol (95-5 en volumes), 180 cm3 d'un mélange 90-10 et 400 cm3 d'un mélange acétate d'éthyle et de méthanol 80-20, sous une pression de 0,5 bar (51 kPa) en recueillant des fractions de 100 cm3. La première fraction est éliminée. Les 10 fractions suivantes sont réunies et concentrées à sec sous pression réduite (20 mm de mercure ; 2,7 kPa) à une température voisine de 40°C. On obtient 1,7 g de produit qui est dissous dans 20 cm3 d'acétate d'éthyle. Cette solution est additionnée de 0,7 cm3 d'éther chlorhydrique 3,05N et agitée à une température voisine de 20°C pendant 10 minutes. Les cristaux obtenus sont séparés par filtration, lavés par 10 cm3 d'acétate d'éthyle et 10 cm3 d'éther diéthylique et séchés sous pression réduite (0,1 mm de mercure ; 13,5 Pa) à une température voisine de 60°C. On obtient ainsi 0,6 g de dichlorhydrate de (+)-N-{{α-[(pipérazinyl-1)-3 propoxyimino] benzyl}-3 phényl} (pyridyl-3)-3 1H,3H-pyrrolo[1,2-c] thiazolecarboxamide-7, à l'état de mélange des formes E et Z, fondant à 195°C.

Le (+)-N-{{α-[(tert-butyloxycarbonyl-4 pipérazinyl-1)-3 propoxyimino] benzyl}-3 phényl} (pyridyl-3)-3 1H,3H-pyrrolo[1,2-c] thiazolecarboxamide-7 (mélange des formes E et Z) peut être préparé de la manière suivante : à une solution de 5,5 g de (+)-N-[(α-hydroxyiminobenzyl)-3 phényl] (pyridyl-3)-3 1H,3H-pyrrolo [1,2-c] thiazolecarboxamide-7 (mélange des formes E et Z) dans 50 cm3 de N,N-diméthylformamide anhydre, on ajoute, à température ambiante, sous courant d'azote et sous agitation, 0,56 g d'hydrure de sodium (en dispersion à 60 dans l'huile de vaseline). Après 1 heure et 25 minutes d'agitation à température ambiante, une solution de 6,25 g de paratoluènesulfonate de (tert-butoxycarbonyl-4 pipérazinyl-1)-3 propyle dans 25 cm3 de N,N-diméthylformamide anhydre est introduite en 5 minutes environ. L'agitation est maintenue pendant 19 heures à température ambiante et le solvant est évaporé à sec sous pression réduite (0,1 mm de mercure ; 13,5 Pa) à une température voisine de 45°C. Le résidu est mis en suspension dans 100 cm3 d'acétate d'éthyle à température ambiante ; l'insoluble est séparé par filtration, lavé avec 40 cm3 d'acétate d'éthyle et le filtrat est évaporé à sec sous pression réduite (20 mm de mercure ; 2,7 kPa) à une température voisine de 35°C. Le résidu (13,5 g) est chromatographié sur une colonne de 3,5 cm de diamètre contenant 250 g d'alumine neutre (0,032-0,063 mm) désactivée par 10 % d'eau. On élue par un mélange d'acétate d'éthyle et de cyclohexane (80-20 en volumes) sous une pression de 0,5 bar (51 kPa) en recueillant des fractions de 50 cm3. Les 2 premières fractions sont éliminées. Les 2 fractions suivantes sont réunies et concentrées à sec sous pression réduite (20 mm de mercure ; 2,7 kPa) à une température voisine de 45°C. On obtient ainsi 7,1 g de produit qui sont rechromatographiés une deuxième fois sur une colonne de 2,7 cm de diamètre contenant 150 g d'alumine neutre (0,032-0,063 mm) désactivée par 10 % d'eau. On élue par un mélange d'acétate d'éthyle et de cyclohexane (50-50 en volumes). Les 4 premières fractions sont éliminées. Les 7 fractions suivantes sont réunies et concentrées à sec sous pression réduite (20 mm de mercure ; 2,7 kPa) à une température voisine de 40°C. On obtient ainsi 4,4 g de (+)-N-{{α-[(tert-butyloxycarbonyl-4 pipérazinyl-1)-3 propoxyimino] benzyl}-3 phényl} (pyridyl-3)-3 1H,3H-pyrrolo[1,2-c] thiazolecarboxamide-7 (mélange des formes E et Z), sous la forme d'une huile orangée.

Le paratoluènesulfonate de (tert-butoxycarbonyl-4 pipérazinyl-1)-3 propyle peut être préparé de la manière suivante : on ajoute en 1 heure et 50 minutes une solution de 11,6 g de chlorure de paratoluènesulfonyle dans 50 cm3 de pyridine à une solution de 13,5 g de (hydroxy-3 propyl)-1 tert-butoxycarbonyl-4 pipérazine dans 50 cm3 de pyridine refroidie à 0°C et on agite 15 heures à une température voisine de 20°C. La température est amenée à 10-15°C, 8,5 cm3 de triéthylamine sont introduits, puis 170 cm3 d'eau distillée et 170 cm3 d'acétate d'éthyle. La phase organique est décantée, la phase aqueuse est extraite par 300 cm3 au total d'acétate d'éthyle. Les extraits organiques sont réunis, lavés par 750 cm3 d'eau distillée, séchés sur sulfate de magnésium anhydre, traités par 0,2 g de noir décolorant, filtrés et évaporés à sec sous pression réduite (20 mm de mercure ; 2,7 kPa) à une température voisine de 45°C. On obtient ainsi 16,3 g de paratoluènesulfonate de (tert-butoxycarbonyl-4 pipérazinyl-1)-3 propyle sous la forme d'une huile rouge.

L'(hydroxy-3 propyl)-1 tert-butoxycarbonyl-4 pipérazine peut être préparée de la manière suivante : une solution de 12 g de di-tert-butyldicarbonate dans 100 cm3 de dichlorométhane est additionnée à une solution de 7,2 g d'(hydroxy-3 propyl)-1 pipérazine dans 100 cm3 de dichlorométhane ; l'agitation est maintenue pendant 24 heures à température ambiante et le solvant est évaporé à sec sous pression réduite (20 mm de mercure ; 2,7 kPa) à une température voisine de 45°C. On obtient ainsi 13,8 g d'(hydroxy-3 propyl)-1 tert-butoxycarbonyl-4 pipérazine, sous la forme d'une huile jaune. (Rf = 0,25, chromatographie sur couche mince de silice, éluant : acétate d'éthyle).

### EXEMPLE 17

Une solution de 0,5 g de (+)-N-(benzoyl-3 phényl) (pyridyl-3)-3 1H,3H-pyrrolo[1,2-c] thiazolecarboxamide-7 dioxyde-2,2 et de 0,23 g de (méthyl-1 pipéridyl-4) oxyamine à l'état de dichlorhydrate, dans 10 cm3 de pyridine est chauffée au reflux pendant 4 heures. On ajoute 0,12 g de dichlorhydrate de (méthyl-1 pipéridyl-4) oxyamine et le reflux est poursuivi pendant 1 heure. Le mélange est refroidi à une température voisine de 20°C, le solvant est concentré sous pression réduite (20 mm de mercure ; 2,7 kPa) à une température voisine de 45°C, le résidu est dilué dans 30 cm3 d'eau distillée et additionné de 3,4 cm3 de soude 1N. Les cristaux apparus sont séparés par filtration, lavés par 20 cm3 au total d'eau distillée et séchés sous pression réduite (0,1 mm de mercure ; 13,5 Pa) à une température voisine de 20°C. La poudre beige obtenue (0,59 g) est dissoute dans 4 cm3 d'acétone. La solution est filtrée, additionnée de 2,5 cm3 d'acétate d'éthyle puis de 0,49 cm3 d'éther chlorhydrique 4,1N. Après 15 minutes d'agitation à température ambiante, les cristaux apparus sont séparés par filtration, lavés par 15 cm3 au total d'éther diéthylique et séchés sous pression réduite (0,1 mm de mercure ; 13,5 Pa) à une température voisine de 60°C. Le dichlorhydrate (590 mg), dissous dans 10 cm3 d'eau distillée est alcalinisé par 2 cm3 de soude 1N, puis les cristaux sont séparés par filtration, lavés à l'eau distillée jusqu'à neutralité et séchés sous pression réduite (0,1 mm de mercure ; 13,5 Pa) à une température voisine de 20°C. Le solide (416 mg) est dissous dans 2 cm3 d'éthanol absolu, traité par 10 mg de noir décolorant, filtré, lavé par 6 cm3 d'acétate-d'éthyle et additionné de 0,36 cm3 d'éther chlorhydrique 3,65N. Après 15 heures d'agitation à température ambiante, les cristaux sont séparés par filtration, lavés par 30 cm3 au total d'éther diéthylique et séchés à l'air. On obtient ainsi 470 mg de dichlorhydrate de N-{α-[(méthyl-1 pipéridyl-4) oxyiminobenzyl]-3 phényl} (pyridyl-3)-3 1H,3H-pyrrolo [1,2-c] thiazolecarboxamide-7, dioxyde-2,2-(3RS) à l'état de mélange des formes E et Z, sous forme de cristaux crème, fondant à 200°C.

Le (+)-N-(benzoyl-3 phényl) (pyridyl-3)-3 1H,3H-pyrrolo [1,2-c] thiazolecarboxamide-7 dioxyde-2,2 est obtenu selon la méthode décrite dans la demande de brevet européen publiée sous le N° 0 253 711.

Le dichlorhydrate de (méthyl-1 pipéridyl-4) oxyamine peut être obtenue comme décrit dans l'exemple 10.

### EXEMPLE 18

A une solution de 1,74 g de (+)-N-{[α-hydroxyimino (méthoxy-4 benzyl)]-3 phényl} (pyridyl-3)-3 1H,3H-pyrrolo[1,2-c] thiazolecarboxamide-7 (mélange des formes E et Z) dans 17,5 cm3 de N,N-diméthylformamide anhydre, on ajoute, à température ambiante, sous courant d'azote et sous agitation 0,18 g d'hydrure de sodium (en dispersion à 50 % dans l'huile de vaseline). Après 30 minutes d'agitation à température ambiante, une solution de 2,61 g de (chloro-3 propyl)-1 méthyl-4 pipérazine dans 2 cm3 de N,N-diméthylformamide anhydre est introduite en 5 minutes environ, puis la solution est agitée pendant 15 heures à une température voisine de 20°C. Le solvant est concentré sous pression réduite (0,5 mm de mercure ; 67,5 Pa) à une température voisine de 50°C, puis 25 cm3 d'eau distillée sont ajoutés et la cristallisation est amorcée. Après 3 heures d'agitation à température ambiante, les cristaux sont séparés par filtration, lavés à l'eau distillée jusqu'à neutralité et séchés à l'air. Les 2,30 g de solide obtenus sont alors dissous dans 40 cm3 d'acétate d'éthyle et on ajoute 37 cm3 d'acide chlorhydrique 0,1N. La phase aqueuse est décantée, lavée par 20 cm3 d'acétate d'éthyle, alcalinisée avec 3,7 cm3 de soude 1N et extraite par 40 cm3 d'acétate d'éthyle. Les extraits organiques sont réunis, séchés sur du sulfate de magnésium anhydre, traités par 50 mg de noir décolorant, filtrés et concentrés sous pression réduite (20 mm de mercure ; 2,7 kPa) à une température voisine de 45°C. On obtient ainsi 1,41 g d'une meringue jaune pâle qui est dissoute dans 10 cm3 d'acétate d'éthyle, filtrée, rincée par 8 cm3 au total d'acétate d'éthyle et additionnée de 1,5 cm3 d'éther chlorhydrique 3N. On agite à une température voisine de 20°C pendant 1 heure. Les cristaux apparus sont séparés par filtration, lavés 3 fois par 12 cm3 au total d'éther diéthylique et séchés sous pression réduite (0,1 mm de mercure ; 13,5 Pa) à une température voisine de 50°C. On obtient ainsi 1,53 g de dichlorhydrate de (+)-N-{{α-[(méthyl-4 pipérazinyl-1)-3 propoxyimino] (méthoxy-4 benzyl)}-3 phényl} (pyridyl-3)-3 1H,3H-pyrrolo[1,2-c] thiazolecarboxamide-7, à l'état de mélange des formes E et Z, sous forme de cristaux crème fondant à 170°C.

Le (+)-N-{α-[hydroxyimino (méthoxy-4 benzyl)]-3 phényl} (pyridyl-3)-3 1H,3H-pyrrolo[1,2-c] thiazolecarboxamide-7 (mélange des formes E et Z) peut être préparé de la manière suivante : à une suspension de 1,82 g de (+)-N-[(méthoxy-4 benzoyl)-3 phényl] (pyridyl-3)-3 1H,3H-pyrrolo[1,2-c] thiazolecarboxamide-7 dans 22 cm3 de propanol-1, on ajoute 425 mg de carbonate de sodium et 280 mg de chlorhydrate d'hydroxylamine. La suspension est chauffée au reflux, sous agitation pendant 2 heures. Le solvant est évaporé sous pression réduite (20 mm de mercure ; 2,7 kPa) à une température voisine de 50°C ; le résidu est dilué dans 25 cm3 d'eau distillée et la cristallisation est amorcée. Les cristaux sont séparés par filtration, lavés à l'eau distillée jusqu'à neutralité et séchés à l'air. On obtient ainsi 1,80 g de (+)-N-{α-[hydroxyimino (méthoxy-4 benzyl)]-3 phényl} (pyridyl-3)-3 1H,3H-pyrrolo[1,2-c] thiazolecarboxamide-7 (mélange des formes E et Z), sous forme de cristaux blancs fondant à 235°C.

Le (+)-N-[(méthoxy-4 benzoyl)-3 phényl] (pyridyl-3)-3 1H,3H-pyrrolo[1,2-c] thiazolecarboxamide-7 est préparé selon la méthode décrite dans la demande de brevet européen publiée sous le n° 0 253 711.

### EXEMPLE 19

A une solution de 480 mg de (+)-N-[α-(hydroxyiminobenzyl)-3 chloro-6 phényl] (pyridyl-3)-3 1H,3H-pyrrolo[1,2-c] thiazolecarboxamide-7 (mélange des formes E et Z) dans 5 cm3 de N,N-diméthylformamide anhydre, on ajoute, à température ambiante, sous courant d'azote et sous agitation 9 mg d'hydrure de sodium (en dispersion à 60 % dans l'huile de vaseline). Après 40 minutes d'agitation à température ambiante, 190 mg de (chloro-2 éthyl)-4 morpholine sont additionnés puis la solution est agitée pendant 23 heures à une température voisine de 20°C. On ajoute 23 mg d'hydrure de sodium (en dispersion à 60 % dans l'huile de vaseline) et 43 mg de (chloro-2 éthyl)-4 morpholine et l'agitation est poursuivie pendant 24 heures à une température voisine de 20°C. Le solvant est concentré sous pression réduite (0,1 mm de mercure ; 13,5 Pa) à une température voisine de 60°C. On ajoute 15 cm3 d'acétate d'éthyle. Le chlorure de sodium est séparé par filtration et lavé par 20 cm3 d'acétate d'éthyle. Après évaporation du solvant sous pression réduite (20 mm de mercure ; 2,7 kPa) à une température voisine de 40°C, le résidu (660 mg) est chromatographié sur une colonne de 1,4 cm de diamètre contenant 20 g d'alumine basique (0,050-0,200 mm). On élue par de l'acétate d'éthyle sous une pression de 0,5 bar (51 kPa) en recueillant des fractions de 5 cm3. Les 8 premières fractions sont éliminées. Les 58 fractions suivantes sont réunies et concentrées à sec sous pression réduite (20 mm de mercure ; 2,7 kPa) à une température voisine de 40°C. On obtient ainsi 370 mg de produit qui est dissous dans 10 cm3 d'acétate d'éthyle et additionné de 0,42 cm3 d'éther chlorhydrique 3N. On agite à une température voisine de 20°C pendant 10 minutes et les cristaux apparus sont séparés par filtration, lavés par 5 cm3 d'acétate d'éthyle et 10 cm3 d'éther diéthylique et séchés sous pression réduite (0,1 mm de mercure ; 13,5 Pa) à une température voisine de 25°C. On obtient ainsi 388 mg de dichlorhydrate de (+)-N-{{α-[(morpholino-2 éthoxyimino] benzyl}-3 chloro-6 phényl} (pyridyl-3)-3 1H,3H-pyrrolo[1,2-c] thiazolecarboxamide-7, à l'état de mélange des formes E et Z, sous forme de cristaux crème fondant à 154°C.

Le (+)-N-[(α-hydroxyiminobenzyl)-3 chloro-6 phényl] (pyridyl-3)-3 1H,3H-pyrrolo[1,2-c] thiazolecarboxamide-7 (mélange des formes E et Z) peut être préparé de la manière suivante : à une suspension de 490 mg de (+)-N-[benzoyl-3 chloro-6 phényl] (pyridyl-3)-3 1H,3H-pyrrolo[1,2-c] thiazolecarboxamide-7 dans 5,5 cm3 de propanol-1, on ajoute 113 mg de carbonate de sodium et 150 mg de chlorhydrate d'hydroxylamine. La suspension est chauffée au reflux, sous agitation pendant 2 heures et le solvant est évaporé sous pression réduite (20 mm de mercure ; 2,7 kPa) à une température voisine de 40°C. Le résidu est dilué dans 40 cm3 d'eau distillée et la cristallisation est amorcée. Les cristaux sont séparés par filtration, lavés avec 20 cm3 d'eau distillée et séchés à l'air. On obtient ainsi 490 mg de (+)-N-[(α-hydroxyiminobenzyl)-3 chloro-6 phényl] (pyridyl-3)-3 1H,3H-pyrrolo[1,2-c] thiazolecarboxamide-7 (mélange des formes E et Z), sous forme de cristaux blancs fondant à 135°C.

Le (+)-N-[benzoyl-3 chloro-6 phényl] (pyridyl-3)-3 1H,3H-pyrrolo[1,2-c] thiazolecarboxamide-7 est préparé selon la méthode décrite dans la demande de brevet européen publiée sous le n° 0 253 711.

### EXEMPLE 20

A une solution de 2,02 g de (+)-N-{[hydroxyimino (pyridyl-2) méthyl]-3 phényl} (pyridyl-3)-3 1H,3H-pyrrolo[1,2-c] thiazolecarboxamide-7 (mélange des formes E et Z) dans 20 cm3 de N,N-diméthylformamide anhydre, on ajoute, à température ambiante, sous courant d'azote et sous agitation 220 mg d'hydrure de sodium (en dispersion à 50 % dans l'huile de vaseline). Après 25 minutes d'agitation à une température voisine de 20°C, une solution de 813 mg de (chloro-3 propyl)-1 méthyl-4 pipérazine dans 2 cm3 de N,N-diméthylformamide anhydre est introduite en 5 minutes environ, puis la solution est agitée pendant 21 heures à température ambiante. Le solvant est concentré sous pression réduite (0,5 mm de mercure ; 67,5 Pa) à une température voisine de 50°C, puis on ajoute 40 cm3 d'eau distillée et 40 cm3 d'acétate d'éthyle. La phase organique est décantée et la phase aqueuse est extraite par 40 cm3 d'acétate d'éthyle. Les extraits organiques sont réunis, séchés sur du sulfate de magnésium anhydre, traités par 50 mg de noir décolorant, filtrés et concentrés sous pression réduite (20 mm de mercure ; 2,7 kPa) à une température voisine de 45°C. On obtient ainsi 2,32 g d'une meringue beige qui est dissoute dans 23 cm3 d'acétate d'éthyle. La solution est filtrée, rincée par 8 cm3 d'acétate d'éthyle et additionnée de 2,6 cm3 d'éther chlorhydrique 3N. Après 1 heure d'agitation à température ambiante, les cristaux apparus sont séparés par filtration, lavés 3 fois par 12 cm3 au total d'acétate d'éthyle et séchés sous pression réduite (0,1 mm de mercure; 13,5 Pa) à une température voisine de 50°C. Le dichlorhydrate (2,29 g) est dissous dans 40 cm3 d'eau distillée alcalinisée par 4 cm3 de soude 2N. Après addition de 30 cm3 d'acétone pour solubiliser, on ajoute 40 cm3 d'acétate d'éthyle et 40 cm3 d'eau distillée. La phase organique est décantée, la phase aqueuse est extraite par 40 cm3 d'acétate d'éthyle. Les extraits organiques sont réunis, séchés sur du sulfate de magnésium anhydre et évaporés sous pression réduite (20 mm de mercure ; 2,7 kPa) à une température voisine de 45°C. La meringue beige obtenue (1,74 g) est dissoute dans 10 cm3 d'acétate d'éthyle. La solution est filtrée, lavée par 7,5 cm3 d'acétate d'éthyle et additionnée de 1,87 cm3 d'éther chlorhydrique 3N. Les cristaux apparus sont séparés par filtration, lavés par 15 cm3 d'acétate d'éthyle et séchés sous pression réduite (0,1 mm de mercure ; 13,5 Pa) à une température voisine de 50°C. On obtient 1,65 g de (+)-N-{{[(méthyl-4 pipérazinyl-1)-3 propoxyimino] (pyridyl-2) méthyl}-3 phényl} (pyridyl-3)-3 1H,3H-pyrrolo[1,2-c] thiazolecarboxamide-7, à l'état de mélange des formes E et Z, sous forme de cristaux crème fondant à 180°C.

Le (+)-N-{[hydroxyimino (pyridyl-2) méthyl]-3 phényl} (pyridyl-3)-3 1H,3H-pyrrolo[1,2-c] thiazolecarboxamide-7 (mélange des formes E et Z) peut être préparé de la manière suivante : à une suspension de 2,13 g de (+)-N-[(pyridyl-2 carbonyl)-3 phényl] (pyridyl-3)-3 1H,3H-pyrrolo[1,2-c] thiazolecarboxamide-7 dans 26 cm3 de propanol-1, on ajoute 530 mg de carbonate de sodium et 350 mg de chlorhydrate d'hydroxylamine. La suspension est chauffée au reflux, sous agitation, pendant 1 heure et le solvant est évaporé sous pression réduite (0,5 mm de mercure ; 67,5 Pa) à une température voisine de 50°C puis le résidu est dilué dans 25 cm3 d'eau distillée et la cristallisation est amorcée. Après 24 heures d'agitation à températutre ambiante, les cristaux sont séparés par filtration, lavés à l'eau distillée jusqu'à neutralité et séchés à l'air. On obtient ainsi 2,11 g de (+)-N-{[hydroxyimino (pyridyl-2) méthyl]-3 phényl} (pyridyl-3)-3 1H,3H-pyrrolo[1,2-c] thiazolecarboxamide-7 (mélange des formes E et Z) sous forme de cristaux blancs fondant à 150-160°C.

Le (+)-N-[(pyridyl-2) carbonyl)-3 phényl] (pyridyl-3)-3 1H,3H-pyrrolo[1,2-c] thiazolecarboxamide-7 est préparé selon la méthode décrite dans la demande de brevet européen publiée sous le n° 0 253 711.

### EXEMPLE 21

Une suspension de 1 g de (+)-N-{{α-[(pipérazinyl-1)-3 propoxyimino] benzyl}-3 phényl} (pyridyl-3)-3 1H,3H-pyrrolo[1,2-c] thiazolecarboxamide-7 (mélange des formes E et Z), 134 mg de carbonate de potassium et 0,134 cm3 de bromo-2 éthanol dans 20 cm3 d'acétonitrile est agitée à une température voisine de 20°C pendant 24 heures. L'insoluble est séparé par filtration et le filtrat est concentré à sec sous pression réduite (20 mm de mercure ; 2,7 kPa) à une température voisine de 40°C. On obtient 1,13 g d'une huile jaune qui est chromatographiée sur une colonne de 1,7 cm de diamètre contenant 50 g d'alumine neutre (0,032-0,063 mm) désactivée par 10 % d'eau. On élue par un mélange d'acétate d'éthyle et de méthanol (80-20 en volumes) sous une pression de 0,5 bar (51 kPa) en recueillant des fractions de 25 cm3. Les 2 premières fractions sont éliminées. Les 2 fractions suivantes sont réunies et concentrées à sec sous pression réduite (20 mm de mercure ; 2,7 kPa) à une température voisine de 40°C. La meringue jaune (800 mg) obtenue est chromatographiée une deuxième fois sur une colonne de 1,4 cm de diamètre contenant 25 g d'alumine neutre désactivée à 10 % d'eau (0,032-0,063 mm). On élue par un mélange d'acétate d'éthyle et de méthanol (90-10 en volumes) sous une pression de 0,5 bar (51 kPa) en recueillant des fractions de 15 cm3. Les 5 premières fractions sont éliminées. Les 7 fractions suivantes sont réunies et concentrées à sec sous pression réduite (20 mm de mercure ; 2,7 kPa) à une température voisine de 40°C. Les 465 mg de produit obtenus sont dissous dans 5 cm3 d'acétate d'éthyle et additionnés de 0,51 cm3 d'éther chlorhydrique 3N. Après 15 minutes d'agitation à une température voisine de 20°C, les cristaux apparus sont séparés par filtration, lavés par 2 cm3 d'acétate d'éthyle et 5 cm3 d'éther diéthylique et séchés sous pression réduite (0,1 mm de mercure ; 13,5 Pa) à une temperature voisine de 20°C. On obtient ainsi 460 mg de dichlorhydrate de (+)-N-{{{α-[(hydroxy-2 éthyl)-4 pipérazinyl-1]-3 propoxyimino} benzyl}-3 phényl} (pyridyl-3)-3 1H,3H-pyrrolo[1,2-c] thiazolecarboxamide-7, à l'état de mélange des formes E et Z, sous forme de cristaux blancs fondant à 178°C.

Le (+)-N-{{α-[(pipérazinyl-1)-3 propoxyimino] benzyl}-3 phényl} (pyridyl-3)-3 1H,3H-pyrrolo[1,2-c] thiazolecarboxamide-7 (mélange des formes E et Z) est obtenu comme décrit à l'exemple 16.

### EXEMPLE 22

Une suspension de 3,11 g de (+)-N-{{{α-[N-(chloro-2 éthyl) anilino]-2 éthoxyimino} benzyl}-3 phényl} (pyridyl-3)-3 1H,3H-pyrrolo[1,2-c] thiazolecarboxamide-7 (mélange des formes E et Z), 680 mg de chlorhydrate de méthylamine, 2,07 g de carbonate de potassium, 950 mg d'iodure cuivreux dans 62 cm3 d'acétonitrile est chauffée en autoclave pendant 6 heures à 100°C. L'insoluble est filtré, rincé 3 fois par 60 cm3 au total d'acétonitrile. Le filtrat est évaporé à sec sous pression réduite (20 mm de mercure ; 2,7 kPa) à une température voisine de 45°C. On obtient 1,30 g d'une laque verte. Par ailleurs, le solide est mis en suspension dans 300 cm3 d'ammoniaque (d=0,92), 300 cm3 d'acétate d'éthyle sont ajoutés et la phase organique est décantée, lavée par 200 cm3 d'eau distillée, séchée sur du sulfate de magnésium anhydre et concentrée à sec sous pression réduite (20 mm de mercure ; 2,7 kPa) à une température voisine de 45°C. Le résidu est dilué dans 20 cm3 d'acétate d'éthyle ; on ajoute 20 cm3 d'acide chlorhydrique 0,5N, la phase aqueuse est décantée, alcalinisée par 2,2 cm3 de soude 5N et extraite par 40 cm3 au total d'acétate d'éthyle. Les extraits organiques sont réunis, séchés sur du sulfate de magnésium anhydre et évaporés à sec sous pression réduite (20 mm de mercure ; 2,7 kPa) à une température voisine de 40°C. On obtient 1,15 g d'une meringue ocre, qui additionnée des 1,3 g de laque verte précédemment obtenus est chromatographiée sur une colonne de 3,5 cm de diamètre contenant 125 g environ de silice (0,02-0,045 mm). On élue par un mélange d'acétonitrile et d'ammoniaque (d=0,92) (95-5 en volumes) sous une pression de 0,3 bar (30,6 kPa) en recueillant des fractions de 80 cm3. Les 15 premières fractions sont éliminées. Les 3 fractions suivantes sont réunies et concentrées à sec sous pression réduite (20 mm de mercure ; 2,7 kPa) à une température voisine de 50°C. On obtient ainsi 370 mg d'une meringue jaune qui est dissoute dans 3,7 cm3 d'acétate d'éthyle, filtrée et l'insoluble est rincé par 1,7 cm3 d'acétate d'éthyle. La solution obtenue est additionnée de 0,39 cm3 d'éther chlorhydrique 3N et est agitée à une température voisine de 20°C pendant 15 minutes. La suspension est diluée par 20 cm3 d'éther diéthylique, agitée 1 heure à une température voisine de 20°C et les cristaux sont séparés par filtration, lavés par 20 cm3 au total d'éther diéthylique et séchés sous pression réduite (0,1 mm de mercure ; 13,5 Pa) à une température voisine de 50°C. On obtient ainsi 370 mg de dichlorhydrate de (+)-N-{{{α-[N-(méthylamino-2 éthyl) anilino]-2 éthoxyimino} benzyl}-3 phényl} (pyridyl-3)-3 1H,3H-pyrrolo[1,2-c] thiazolecarboxamide-7 (mélange des formes E et Z), sous forme cristaux jaunâtres, fondant à 150-155°C.

Le (+)-N-{{{α-[N-(chloro-2 éthyl) anilino]-2 éthoxyimino} benzyl}-3 phényl} (pyridyl-3)-3 1H,3H-pyrrolo[1,2-c] thiazolecarboxamide-7 (mélange des formes E et Z) peut être préparé de la manière suivante : à une suspension de 480 mg d'hydrure de sodium (en dispersion à 50 % dans l'huile de vaseline) dans 10 cm3 de N,N-diméthylformamide anhydre, on ajoute, sous courant d'azote, en 25 minutes environ, une solution de 4,4 g de (+)-N-[α-(hydroxyiminobenzyl)-3 phényl] (pyridyl-3)-3 1H,3H-pyrrolo [1,2-c] thiazolecarboxamide-7 (mélange des formes E et Z) dans 10 cm3 de N,N-diméthylformamide anhydre. Après 15 minutes d'agitation à température ambiante, une solution de 2,18 g de N,N-bis(chloro-2 éthyl) aniline dans 10 cm3 de N,N-diméthylformamide anhydre est introduite en 2 minutes environ. La solution est alors agitée pendant 16 heures à température ambiante. Le solvant est concentré à sec sous pression réduite (0,1 mm de mercure ; 13,5 Pa) à une température voisine de 45°C. Le résidu est mis en suspension dans 80 cm3 d'eau distillée. Après 1 heure d'agitation à une température voisine de 20°C, les cristaux sont séparés par filtration, lavés par 20 cm3 au total d'eau distillée et séchés sous pression réduite (0,1 mm de mercure ; 13,5 Pa) à une température voisine de 20°C. On obtient ainsi 6,32 g d'un résidu qui est chromatographié sur une colonne de 5,5 cm de diamètre contenant 250 g environ de silice (0,02-0,045 mm). On élue par de l'acétate d'éthyle sous une pression de 0,5 bar (51 kPa) en recueillant des fractions de 100 cm3. Les 10 premières fractions sont éliminées. Les 8 fractions suivantes sont réunies et concentrées à sec sous pression réduite (20 mm de mercure ; 2,7 kPa) à une température voisine de 45°C. On obtient ainsi 3,41 g de (+)-N-{{{α-[N-(chloro-2 éthyl) anilino]-2 éthoxyimino} benzyl}-3 phényl} (pyridyl-3)-3 1H,3H-pyrrolo[1,2-c] thiazolecarboxamide-7, (mélange des formes E et Z), sous forme d'une meringue jaune (Rf=0,54, chromatographie sur couche mince de silice ; éluant : acétate d'éthyle).

Le(+)-N-[(α-hydroxyiminobenzyl)-3 phényl] (pyridyl-3)-3 1H,3H-pyrrolo[1,2-c] thiazolecarboxamide-7 (mélange des formes E et Z) est obtenu comme décrit à l'exemple 1.

### EXEMPLE 23

A une solution de 4,18 g de (+)-N-[(α-hydroxyiminobenzyl)-3 phényl] (pyridyl-3)-3 1H,3H-pyrrolo [1,2-c] thiazolecarboxamide-7 (mélange des formes E et Z) dans 40 cm3 de N,N-diméthylformamide anhydre, on ajoute, à température ambiante, sous courant d'azote et sous agitation, 0,42 g d'hydrure de sodium (en dispersion à 60 % dans l'huile de vaseline). La suspension est agitée 1 heure à une température voisine de 20°C puis une solution de 3,27 g de paratoluènesulfonate de quinuclidine-3-(3RS) dans 25 cm3 de N,N-diméthylformamide anhydre est ensuite introduite en 5 minutes environ. La solution est agitée 18 heures à température ambiante puis chauffée à 70°C pendant 6 heures. Après avoir ramené la température vers 20°C, le solvant est évaporé sous pression réduite (0,1 mm de mercure ; 13,5 Pa) à une température voisine de 40°C. Le résidu est dilué dans 40 cm3 d'acétate d'éthyle ; l'insoluble est filtré, lavé avec 20 cm3 d'acétate d'éthyle et le filtrat est évaporé à sec sous pression réduite (20 mm de mercure ; 2,7 kPa) à une température voisine de 40°C. On obtient ainsi 7,5 g d'un résidu rouge pâteux. Ce produit est chromatographié sur une colonne de 2,4 cm de diamètre contenant 150 g d'alumine neutre (0,050-0,200 mm). On élue par un mélange d'acétate d'éthyle et de méthanol (90-10 en volumes) en recueillant des fractions de 100 cm3. Les 12 premières fractions sont éliminées. Les 4 fractions suivantes sont réunies et concentrées à sec sous pression réduite (20 mm de mercure ; 2,7 kPa) à une température voisine de 40°C. Les 3,34 g de produit obtenus sont chromatographiés une deuxième fois sur une colonne de 1,9 cm de diamètre contenant 60 g d'alumine (0,050-0,200 mm). On élue avec 1000 cm3 d'acétate d'éthyle et 1000 cm3 d'un mélange d'acétate d'éthyle et de méthanol (90-10 en volumes) en recueillant des fractions de 100 cm3. Les 10 premières fractions sont éliminées. Les 10 fractions suivantes sont réunies et concentrées à sec sous pression réduite (20 mm de mercure ; 2,7 kPa) à une température voisine de 40°C. On obtient ainsi 0,97 g d'une meringue orangée qui est chromatographiée sur une colonne de 3 cm de diamètre contenant 65 g de silice (0,02-0,045 mm). On élue par un mélange d'acétate d'éthyle et de diéthylamine (95-5 en volumes) sous une pression de 0,5 bar (51 kPa) en recueillant des fractions de 40 cm3. Les 21 premières fractions sont éliminées. Les 19 fractions suivantes sont réunies et concentrées à sec sous pression réduite (20 mm de mercure ; 2,7 kPa) à une température voisine de 40°C. On obtient ainsi 0,64 g de produit. Ce produit est dissous dans 10 cm3 d'acétate d'éthyle et additionné de 0,81 cm3 d'éther chlorhydrique 3N. Après 10 minutes d'agitation à température ambiante, les cristaux apparus sont séparés par filtration et lavés avec 20 cm3 d'acétate d'éthyle et 20 cm3 au total d'éther diéthylique, et séchés sous pression réduite (0,1 mm de mercure ; 13,5 Pa) à une température voisine de 50°C. On obtient ainsi 0,64 g de dichlorhydrate de (+)-N-{{α-[(quinuclidinyl-3-(3RS) oxyimino] benzyl}-3 phényl} (pyridyl-3)-3 1H,3H-pyrrolo[1,2-c] thiazolecarboxamide-7 (mélange des formes E et Z), sous forme d'une poudre beige fondant à 200°C.

Le(+)-N-[(α-hydroxyiminobenzyl)-3 phényl] (pyridyl-3)-3 1H,3H-pyrrolo[1,2-c] thiazolecarboxamide-7 est obtenu comme décrit à l'exemple 1.

Le paratoluènesulfonate de quinuclidine-3-(3RS) peut être obtenu de la manière suivante : on ajoute, sous un courant d'azote et sous agitation, en 1 heure et 30 minutes environ, 6,3 g de chlorure de paratoluènesulfonyle en solution dans 15 cm3 de pyridine à une solution de 3,8 g de quinuclidinol-3-(3RS) dans 15 cm3 de pyridine refroidie à 0°C et on agite 2 heures entre 5 et 10°C, puis 12 heures à une température voisine de 20°C. La température est ramenée à 15°C et on additionne 4,63 cm3 de triéthylamine. Le milieu réactionnel est versé dans 100 cm3 d'eau distillée. On ajoute 100 cm3 d'acétate d'éthyle; la phase aqueuse est décantée et lavée par 200 cm3 au total d'acétate d'éthyle. Les extraits organiques sont réunis, lavés 2 fois par 200 cm3 au total d'eau distillée, séchés sur sulfate de magnésium anhydre et évaporés à sec sous pression réduite (20 mm de mercure ; 2,7 kPa) à une température voisine de 35°C. On obtient ainsi 6,5 g de paratoluènesulfonate de quinuclidine-3-(3RS), sous la forme d'une huile rouge utilisée telle quelle.

### EXEMPLE 24

A une solution de 2,0 g de (+)-N-[(α-hydroxyiminobenzyl)-3 phényl] (pyridyl-3)-3 1H,3H-pyrrolo [1,2-c] thiazolecarboxamide-7 (mélange des formes E et Z) dans 10 cm3 de N,N-diméthylformamide anhydre, on ajoute, à une température voisine de 20°C, sous courant d'azote et sous agitation, 220 mg d'hydrure de sodium (en dispersion à 50 % dans l'huile de vaseline). La suspension est agitée 30 minutes à une température voisine de 20°C, refroidie à 0°C, puis une solution de 1,2 g de paratoluènesulfonate de méthyl-1 pipéridyle-3-(3RS) dans 6 cm3 de N,N-diméthylformamide anhydre est introduite en 30 minutes environ. La solution, agitée 2 heures à 0°C puis 16 heures à température ambiante est alors versée dans 100 cm3 d'eau distillée. La suspension est extraite 3 fois par 150 cm3 au total d'acétate d'éthyle et lavée 2 fois par 100 cm3 au total d'eau distillée. La phase organique est acidifiée par 50 cm3 d'acide chlorhydrique 0,1N, la phase aqueuse est décantée et neutralisée par 1,2 cm3 de soude 5N. On extrait 2 fois par 50 cm3 d'acétate d'éthyle, traite par 100 mg de noir décolorant, sèche sur du sulfate de magnésium anhydre, et évapore le solvant sous pression réduite (20 mm de mercure ; 2,7 kPa) à une température voisine de 45°C. On obtient 1,6 g d'une meringue ocre qui est chromatographiée sur une colonne de 3,5 cm de diamètre contenant 125 g de silice (0,020-0,045 mm). On élue par un mélange d'acétonitrile et d'ammoniaque (d=0,92) (97-3 en volumes) sous une pression de 0,3 bar (21 kPa) en recueillant des fractions de 80 cm3. Les 20 premières fractions sont éliminées. Les 7 fractions suivantes sont réunies et concentrées à sec sous pression réduite (20 mm de mercure ; 2,7 kPa) à une température voisine de 45°C. On obtient ainsi 1,6 g d'une meringue jaune pâle. Ce produit est dissous dans 6,6 cm3 d'acétate d'éthyle, la solution est filtrée, additionnée de 1,4 cm3 d'éther chlorhydrique 3N et diluée par 20 cm3 d'éther diéthylique. Après 30 minutes d'agitation à température ambiante, les cristaux apparus sont séparés par filtration, lavés par 20 cm3 au total d'éther diéthylique et séchés sous pression réduite (0,1 mm de mercure ; 13,5 Pa) à une température voisine de 50°C. On obtient ainsi 1,3 g de (+)-N-{{α-[(méthyl-1 pipéridyl-3-(3RS)) oxyimino] benzyl}-3 phényl} (pyridyl-3)-3 1H,3H-pyrrolo[1,2-c] thiazolecarboxamide-7-(3R) (mélange des formes E et Z), sous forme d'une poudre jaune fondant à 165-170°C.

Le(+)-N-[(α-hydroxyiminobenzyl)-3 phényl] (pyridyl-3)-3 1H,3H- pyrrolo[1,2-c] thiazolecarboxamide-7 est obtenu comme décrit à l'exemple 1.

Le paratoluènesulfonate de méthyl-1 pipéridyle-3-(3RS) peut être obtenu de la manière suivante : on ajoute, sous agitation, en 30 minutes environ, une solution de 9,5 g de chlorure de paratoluènesulfonyle dans 25 cm3 de pyridine à une solution de 5,8 g de méthyl-1 pipéridinol-3-(3RS) dans 25 cm3 de pyridine refroidie à 0°C et on agite 2 heures entre 5 et 10°C, puis 12 heures à une température voisine de 20°C. On additionne 7,1 cm3 de triéthylamine en maintenant la température vers 20°C. Le milieu réactionnel est versé dans 150 cm3 d'eau distillée. On ajoute 150 cm3 d'acétate d'éthyle ; la phase aqueuse est décantée et lavée par 100 cm3 au total d'acétate d'éthyle. Les extraits organiques sont réunis, lavés 2 fois par 80 cm3 au total d'eau distillée, séchés sur sulfate de magnésium anhydre et évaporés à sec sous pression réduite (20 mm de mercure ; 2,7 kPa) à une température voisine de 45°C. On obtient ainsi 8,8 g de paratoluènesulfonate de méthyl-1 pipéridyle-3-(3RS) sous la forme d'une huile rouge [Rf = 0,72, chromatographie sur couche mince de silice ; éluant : acétonitrile-ammoniaque (d=0,92) (95-5 en volumes)].

### EXEMPLE 25

A une solution de 2,2 g de (+)-N-[(α-hydroxyiminobenzyl)-3 phényl] (pyridyl-3)-3 1H,3H-pyrrolo[1,2-c] thiazolecarboxamide-7 (mélange des formes E et Z) dans 20 cm3 de N,N-diméthylformamide anhydre, on ajoute, à température ambiante, sous courant d'azote et sous agitation, 0,29 g d'hydrure de sodium (en dispersion à 50 % dans l'huile de vaseline). Après 45 minutes d'agitation à température ambiante, une solution de 0,75 g de N,N-diéthylchloroacétamide dans 5 cm3 de N,N-diméthylformamide anhydre est introduite en 1 minute environ, l'agitation à température ambiante étant maintenue pendant 15 heures puis le solvant est concentré à sec sous pression réduite (0,1 mm de mercure ; 13,5 Pa) à une température voisine de 60°C. Le résidu est dilué dans 30 cm3 d'acétone, l'insoluble est filtré et lavé 2 fois par 20 cm3 au total d'acétone. Le filtrat est évaporé à sec sous pression réduite (20 mm de mercure ; 2,7 kPa) à une température voisine de 40°C. On obtient 3,4 g d'un produit orangé qui est chromatographié sur une colonne de 3 cm de diamètre contenant 150 g environ de silice (0,02-0,045 mm). On élue par de l'acétate d'éthyle sous une pression de 0,5 bar (51 kPa) en recueillant des fractions de 30 cm3. Les 18 premières fractions sont éliminées. Les 13 fractions suivantes sont réunies et concentrées à sec sous pression réduite (20 mm de mercure ; 2,7 kPa) à une température voisine de 45C. Les 2,15 g de produit obtenus sont dissous dans 6 cm3 d'acétonitrile et sont additionnés de 2,9 cm3 d'éther chlorhydrique 4N. Après 15 minutes d'agitation à température ambiante, les cristaux apparus sont séparés par filtration, lavés 2 fois par 20 cm3 au total d'éther diéthylique et séchés sous pression réduite (0,1 mm de mercure; 13,5 Pa) à une température voisine de 20°C. On obtient ainsi 2 g de dichlorhydrate de (+)-N-{[α-(diéthylcarbamoylméthoxyimino) benzyl]-3 phényl} (pyridyl-3)-3 1H,3H-pyrrolo[1,2-c] thiazolecarboxamide-7, à l'état de mélange des formes E et Z, sous forme de cristaux blancs fondant à 135°C.

Le(+)-N-[(α-hydroxyiminobenzyl)-3 phényl] (pyridyl-3)-3 1H,3H-pyrrolo[1,2-c] thiazolecarboxamide-7 (mélange des formes E et Z) est obtenu comme décrit à l'exemple 1.

### EXEMPLE 26

A une solution de 2,2 g de (+)-N-[(α-hydroxyiminobenzyl)-3 phényl] (pyridyl-3)-3 1H,3H-pyrrolo[1,2-c] thiazolecarboxamide-7 (mélange des formes E et Z) dans 20 cm3 de N,N-diméthylformamide anhydre, on ajoute, à température ambiante, sous courant d'azote et sous agitation, 0,5 g d'hydrure de sodium (en dispersion à 60 % dans l'huile de vaseline). La suspension est agitée pendant 1 heure à température ambiante puis on additionne 0,72 g de chlorhydrate de (chloro-2 éthyl)-4 morpholine. Après 15 heures d'agitation à température ambiante, le solvant est évaporé à sec sous pression réduite (0,1 mm de mercure ; 13,5 Pa) à une température voisine de 45°C. On obtient ainsi 3,8 g d'un résidu orange pâteux. Ce produit est chromatographié sur une colonne de 3 cm de diamètre contenant 65 g environ de silice (0,02-0,045 mm). On élue par de l'acétate d'éthyle, sous une pression de 0,5 bar (51 kPa) en recueillant des fractions de 50 cm3. Les 7 premières fractions sont éliminées. Les 43 fractions suivantes sont réunies et concentrées à sec sous pression réduite (20 mm de mercure ; 2,7 kPa) à une température voisine de 40°C. Le résidu (1,8 g) est chromatographié une deuxième fois sur une colonne de 3 cm de diamètre contenant 65 g de silice (0,02-0,045 mm). On élue par de l'acétate d'éthyle sous une pression de 0,5 bar (51 kPa) en recueillant des fractions de 150 cm3. Les 15 premières fractions sont éliminées. Les 21 fractions suivantes sont réunies et concentrées à sec sous pression réduite (20 mm de mercure ; 2,7 kPa) à une température voisine de 40°C. La meringue obtenue (1,4 g) est mise en solution dans 40 cm3 d'acétate d'éthyle et on ajoute 2 cm3 d'éther chlorhydrique 4N. Les cristaux apparus sont séparés par filtration, lavés successivement par 15 cm3 d'acétate d'éthyle et par 20 cm3 d'éther diéthylique, et séchés sous pression réduite (0,1 mm de mercure ; 13,5 Pa) à une température voisine de 60°C. Le résidu blanc obtenu (1,4 g) est dissous dans 10 cm3 d'eau distillée. Après addition de 10 cm3 d'une solution à 10 % de carbonate de sodium, la solution est extraite 3 fois par 50 cm3 au total d'acétate d'éthyle. Les extraits organiques sont réunis, lavés par 40 cm3 d'eau distillée, séchés sur du sulfate de magnésium anhydre, traités par 0,2 g de noir décolorant, filtrés et concentrés sous pression réduite (20 mm de mercure ; 2,7 kPa) à une température voisine de 35°C. On obtient ainsi 0,95 g d'une meringue jaune pâle qui est chromatographiée sur une colonne de 1,7 cm de diamètre contenant 30 g d'alumine neutre désactivée à 10 % d'eau (0,032-0,063 mm). On élue par 200 cm3 d'acétate d'éthyle et 210 cm3 d'un mélange d'acétate d'éthyle et de méthanol 95-5 (en volumes), sous une pression de 0,5 bar (51 kPa) en recueillant des fractions de 10 cm3. Les 21 premières fractions sont éliminées. Les 25 fractions suivantes sont réunies et concentrées à sec sous pression réduite (20 mm de mercure ; 2,7 kPa) à une température voisine de 40°C. Le résidu (0,55 g) est dissous dans 2 cm3 d'acétate d'éthyle. La solution obtenue est additionnée de 0,58 cm3 d'éther chlorhydrique 3,9N, de 2 cm3 d'acétate d'éthyle et de 5 cm3 d'éther diéthylique. Après 5 minutes d'agitation à une température voisine de 20°C, les cristaux obtenus sont séparés par filtration, lavés par 2 cm3 d'acétate d'éthyle et 5 cm3 d'éther diéthylique et séchés sous pression réduite (0,1 mm de mercure ; 13,5 Pa) à une température (voisine de 60°C. On obtient ainsi 0,58 g de (+)-N-{[α-(morpholino-2 éthoxyimino) benzyl]-3 phényl} (pyridyl-3)-3 1H,3H-pyrrolo[1,2-c] thiazolecarboxamide-7, à l'état de mélange des formes E et Z, sous forme de dichlorhydrate, fondant à 163°C.

Le(+)-N-[(α-hydroxyiminobenzyl)-3 phényl] (pyridyl-3)-3 1H,3H-pyrrolo[1,2-c] thiazolecarboxamide-7 est obtenu comme décrit à l'exemple 1.

### EXEMPLE 27

A une solution de 1 g de (+)-N-[(α-hydroxyiminobenzyl)-3 phényl] (pyridyl-3)-3 1H,3H-pyrrolo[1,2-c] thiazolecarboxamide-7 (mélange des formes E et Z) dans 7,4 cm3 de N,N-diméthylformamide anhydre, on ajoute, à température ambiante, sous courant d'azote et sous agitation, 0,12 g d'hydrure de sodium (en dispersion à 50 % dans l'huile de vaseline). Une solution de 0,67 g de paratoluènesulfonate de méthyl-1 pipéridyle-4 dans 6,7 cm3 de N,N-diméthylformamide anhydre est ensuite introduite en 5 minutes environ. La solution est agitée pendant 15 heures à température ambiante puis on rajoute 0,14 g de paratoluènesulfonate de méthyl-1 pipéridyle-4 et on poursuit l'agitation pendant 3 heures supplémentaires. Cette opération est répétée 3 fois et le milieu réactionnel est dilué par 100 cm3 d'eau distillée. Après 15 minutes d'agitation à température ambiante, le solide est séparé par filtration et lavé 2 fois par 40 cm3 au total d'eau distillée. Le résidu est mis en solution dans 50 cm3 d'acétate d'éthyle et on ajoute 46 cm3 d'acide chlorhydrique 0,1N. La phase aqueuse est décantée et alcalinisée avec 5 cm3 de soude (d=1,33). Après deux heures d'agitation à température ambiante, les cristaux sont séparés par filtration et lavés avec 20 cm3 au total d'eau distillée. On obtient ainsi 0,82 g de (+)-N-{{α-[(méthyl-1 pipéridyl-4) oxyimino] benzyl}-3 phényl} (pyridyl-3)-3 1H,3H-pyrrolo[1,2-c] thiazolecarboxamide-7 (mélange des formes E et Z), sous forme d'une poudre crème [Rf = 0,33 ; chromatographie sur couche mince ; éluant : acétonitrile-ammoniaque (d=0,92) (95-5 en volumes].

Le(+)-N-[(α-hydroxyiminobenzyl)-3 phényl] (pyridyl-3)-3 1H,3H-pyrrolo[1,2-c] thiazolecarboxamide-7 est obtenu comme décrit à l'exemple 1.

Le paratoluènesulfonate de méthyl-1 pipéridyle-4 peut être obtenu de la manière suivante : on ajoute, en 1 heure et 30 minutes environ, 19,60 g de chlorure de paratoluènesulfonyle à une solution de 11,50 g d'hydroxy-4 méthyl-1 pipéridine dans 50 cm3 de pyridine refroidie à 0°C et on agite 4 heures à 0°C, puis 12 heures à une température voisine de 20°C. Après addition de 14,10 g de triéthylamine et agitation pendant 15 minutes supplémentaires, on ajoute 300 cm3 d'eau distillée et 300 cm3 d'acétate d'éthyle. La phase aqueuse est décantée et lavée par 200 cm3 d'acétate d'éthyle. Les extraits organiques sont réunis, lavés par 500 cm3 au total d'eau distillée, séchés sur sulfate de magnésium anhydre et évaporés à sec sous pression réduite (20 mm de mercure ; 2,7 kPa) à une température voisine de 45°C. Les dernières traces de pyridine sont éliminées sous un vide plus poussé (0,1 mm de mercure ; 13,5 Pa). On obtient ainsi 19,6 g de paratoluènesulfonate de méthyl-1 pipéridyle-4 sous la forme d'une huile brune. [Rf = 0,56 ; chromatographie sur couche mince de silice ; éluant : acétonitrile-ammoniaque (d=0,92) (95-5 en volumes].

### EXEMPLE 28

On ajoute successivement, en 5 minutes environ, 3,3 g de chlorhydrate de chloroformyl-7 (pyridyl-3)-3 1H,3H-pyrrolo[1,2-c] thiazole puis 2,3 cm3 de triéthylamine à une solution de 3,5 g de O-[(méthyl-4 pipérazinyl-1)-3 propyl] amino-3 benzophénone-oxime Z dans 45 cm3 de dichlorométhane et on agite à température ambiante pendant 12 heures. Après addition de 0,6 g de chlorhydrate de chloroformyl-7 (pyridyl-3)-3 1H,3H-pyrrolo[1,2-c] thiazole et de 0,6 g de triéthylamine supplémentaires, la solution est agitée de nouveau pendant 1 heure et le solvant est évaporé à sec sous pression réduite (20 mm de mercure ; 2,7 kPa) à une température voisine de 35°C. Le résidu est alors additionné de 50 cm3 d'eau distillée et de 50 cm3 d'acétate d'éthyle ; la phase organique est décantée, lavée par 35 cm3 au total d'eau distillée, séchée sur sulfate de magnésium anhydre et concentrée sous pression réduite (20 mm de mercure ; 2,7 kPa) à une température voisine de 40°C. Le résidu ( 6,6 g) est chromatographié sur une colonne de 6 cm de diamètre contenant 400 g environ de silice (0,063-0,200 mm) en utilisant le gradiant d'élution suivant : 280 cm3 d'un mélange d'acétonitrile et d'ammoniaque (d=0,92) 99-1 en volumes ; cette fraction est éliminée ; 350 cm3 d'un mélange 95-5 et 280 cm3 d'un mélange 90-10 d'acétonitrile et d'ammoniaque. Les deux dernières fractions sont réunies et concentrées à sec sous pression réduite (20 mm de mercure ; 2,7 kPa) à une température voisine de 50°C. La meringue crème obtenue (4,6 g) est dissoute dans 60 cm3 d'acétate d'éthyle, traitée par 0,5 g de noir décolorant, filtrée et additionnée de 4,8 cm3 d'éther chlorhydrique 3,25N. Après 30 minutes d'agitation à température ambiante, les cristaux apparus sont séparés par filtration, lavés par 100 cm3 au total d'acétate d'éthyle et séchés sous pression réduite (0,1 mm de mercure ; 13,5 Pa) à une température voisine de 50°C. On obtient ainsi 4,8 g de dichlorhydrate de (+)-N-{{α-[(méthyl-4 pipérazinyl-1)-3 propoxyimino] benzyl}-3 phényl} (pyridyl-3)-3 1H,3H-pyrrolo[1,2-c] thiazolecarboxamide-7, forme B, fondant à 168°C.

Le chlorhydrate de chloroformyl-7 (pyridyl-3)-3 1H,3H-pyrrolo[1,2-c] thiazole est préparé selon la méthode décrite dans le brevet européen n° 115 979.

La O-[(méthyl-4 pipérazinyl-1)-3 propyl] amino-3 benzophénone-oxime Z peut être préparée de la façon suivante : une solution de 5 g d'amino-3 benzophénone-oxime Z dans 35 cm3 de N,N-diméthylformamide anhydre est additionnée en 15 minutes environ à une suspension de 1,14 g d'hydrure de sodium (en dispersion à 50 % dans l'huile de vaseline) dans 35 cm3 de N,N-diméthylformamide anhydre. Le milieu est agité pendant 15 minutes à température ambiante et une solution de 4,17 g de (chloro-3 propyl)-1 méthyl-4 pipérazine dans 35 cm3 de N,N-diméthylformamide anhydre est introduite en 5 minutes à une température voisine de 2°C. Après 12 heures d'agitation à température ambiante, le solvant est évaporé à sec sous pression réduite (0,1 mm de mercure ; 13,5 Pa) à une température voisine de 50°C. Le résidu est dilué dans 50 cm3 d'acétate d'éthyle, les sels minéraux sont filtrés, lavés par 50 cm3 au total d'acétate d'éthyle et les filtrats sont concentrés à sec sous pression réduite (20 mm de mercure ; 2,7 kPa) à une température voisine de 45°C. On obtient ainsi 9,2 g de O-[(méthyl-4 pipérazinyl-1)-3 propyl] amino-3 benzophénone-oxime Z sous la forme d'une huile orangée [Rf = 0,23 ; chromatographie sur couche mince de silice éluant : acétate d'éthyle- diéthylamine (95-5 en volumes)].

L'amino-3 benzophénone-oxime Z peut être préparée de la façon suivante : on chauffe pendant 4 heures au reflux 600 cm3 d'une solution pyridinique de 32 g d'amino-3 benzophénone et de 22,6 g de chlorhydrate d'hydroxylamine, puis le solvant est évaporé sous pression réduite (20 mm de mercure ; 2,7 kPa) à une température voisine de 50°C. Le résidu est alors dilué par 400 cm3 d'eau distillée et la cristallisation est amorcée. Après 2 heures d'agitation à température ambiante, les cristaux sont séparés par filtration et lavés par 1 litre au total d'eau distillée. On obtient ainsi 35,4 g d'amino-3 benzophénone-oxime, (mélange des formes E et Z) sous la forme d'une poudre beige. 3 fractions de 11,8 g ont été chromatographiées sur une colonne de 7 cm de diamètre contenant 600 g de silice (0,02-0,045 mm) par un mélange de chlorure de méthylène et d'acétate d'éthyle (80-20 en volumes) sous une pression de 0,5 bar (51 kPa) et en recueillant des fractions de 100 cm3. Les 19 premières fractions sont éliminées. Les 8 fractions suivantes correspondant à la forme Z sont rassemblées, les 13 suivantes, mélange des formes E et Z sont éliminées et les 26 dernières sont réunies pour former un lot enrichi en forme E. Les deux lots sont concentrés à sec sous pression réduite (20 mm de mercure ; 2,7 kPa) à une température voisine de 40°C. On obtient respectivement 11,2 et 9,5 g. Les deux produits sont chacun remis en suspension dans 50 cm3 d'oxyde de diisopropyle, les cristaux sont séparés par filtration, lavés par 20 cm3 au total d'oxyde de diisopropyle et séchés sous pression réduite (20 mm de mercure ; 2,7 kPa) à une température voisine de 20°C. On obtient ainsi 10,43 g d'amino-3 benzophénone-oxime Z, et 5,7 g d'un mélange enrichi à 80 % en amino-3 benzophénone-oxime E qui sera utilisée tel quel.

### EXEMPLE 29

On ajoute successivement, en 5 minutes environ, 3,5 g de chlorhydrate de chloroformyl-7 (pyridyl-3)-3 1H,3H-pyrrolo[1,2-c] thiazole puis 3,0 cm3 de triéthylamine à une solution de 2,9 g de O-[(méthyl-4 pipérazinyl-1)-3 propyl] amino-3 benzophénone-oxime forme E (pure à 80 %) dans 30 cm3 de dichlorométhane et on agite à température ambiante pendant 1 heure et 30 minutes. Après addition de 0,25 g supplémentaires de chlorhydrate de chloroformyl-7 (pyridyl-3)-3 1H,3H-pyrrolo[1,2-c] thiazole, la solution est agitée de nouveau pendant 2 heures et 15 minutes, puis diluée par 30 cm3 d'eau distillée. La phase organique est décantée, lavée par 60 cm3 au total d'eau distillée, séchée sur sulfate de magnésium anhydre et concentrée sous pression réduite (20 mm de mercure ; 2,7 kPa). Les 4,1 g de meringue beige obtenus sont chromatographiés sur une colonne de 6 cm de diamètre contenant environ 400 g de silice (0,02-0,045 mm). On élue par un mélange d'acétate d'éthyle et de diéthylamine (95-5 en volumes) sous une pression de 0,5 bar (51 kPa) en recueillant des fractions de 100 cm3. Les 25 premières fractions sont éliminées. Les 30 fractions suivantes sont réunies et concentrées à sec sous pression réduite (20 mm de mercure ; 2,7 kPa) à une température voisine de 40°C. On obtient 2,9 g de meringue qui sont mis en suspension dans 120 cm3 d'acétate d'éthyle et sont chauffés au reflux jusqu'à dissolution totale, puis refroidis vers 25°C. A cette solution, on ajoute 3,0 cm3 d'éther chlorhydrique 3,25M et on agite à une température voisine de 5°C pendant 30 minutes. Les cristaux obtenus sont séparés par filtration, lavés par 25 cm3 d'acétate d'éthyle et 50 cm3 d'éther diéthylique et séchés sous pression réduite (0,1 mm de mercure ; 13,5 Pa) à une température voisine de 50°C. On obtient ainsi 2,6 g de dichlorhydrate de (+)-N-{{α-[(méthyl-4 pipérazinyl-1)-3 propoxyimino] benzyl}-3 phényl} (pyridyl-3)-3 1H,3H-pyrrolo[1,2-c] thiazolecarboxamide-7, forme A, fondant à 160°C.

Le chlorhydrate de chloroformyl-7 (pyridyl-3)-3 1H,3H-pyrrolo[1,2-c] thiazole est préparé selon la méthode décrite dans le brevet européen n° 0 115 979.

La O-[(méthyl-4 pipérazinyl-1)-3 propyl] amino-3 benzophénone-oxime E peut être préparée de la façon suivante : à une solution de 3,2 g d'amino-3 benzophénone-oxime E dans 20 cm3 de N,N-diméthylformamide anhydre, on ajoute, à température ambiante, sous courant d'azote et sous agitation, 0,7 g d'hydrure de sodium (en dispersion à 60 % dans l'huile de vaseline). On agite pendant 1 heure puis une solution de 2,7 g de (chloro-3 propyl)-1 méthyl-4 pipérazine dans 20 cm3 de N,N-diméthylformamide anhydre est introduite en 15 minutes environ. Après 1 heure et 15 minutes d'agitation à température ambiante, le solvant est évaporé à sec sous pression réduite (0,1 mm de mercure ; 13,5 Pa) à une température voisine de 50°C. Le résidu est repris par 50 cm3 d'acétate d'éthyle ; les sels minéraux sont éliminés par filtration, lavés par 50 cm3 d'acétate d'éthyle et le solvant est évaporé à sec sous pression réduite (20 mm de mercure ; 2,7 kPa) à une température voisine de 35°C. Le résidu est dilué dans 40 cm3 d'acétate d'éthyle et extrait 3 fois par 40 cm3 au total d'acide chlorhydrique 1N puis les phases aqueuses sont réunies, amenées à pH 8 avec de la soude 2N et la solution est extraite 2 fois par 50 cm3 au total d'acétate d'éthyle. Les extraits organiques sont réunis, séchés sur sulfate de magnésium anhydre, filtrés et concentrés sous pression réduite (20 mm de mercure ; 2,7 kPa) à une température voisine de 40°C. Les 4,5 g d'huile orange obtenus sont chromatographiés sur une colonne de 6 cm de diamètre contenant environ 500 g de silice (0,02-0,045 mm). On élue par un mélange d'acétate d'éthyle et de diéthylamine (95-5 en volumes) sous une pression de 0,5 bar (51 kPa) et en recueillant des fractions de 80 cm3. Les 16 premières fractions sont éliminées. Les 16 fractions suivantes sont réunies et concentrées à sec sous pression réduite (20 mm de mercure ; 2,7 kPa) à une température voisine de 40°C. On obtient ainsi 3,8 g de O-[(méthyl-4 pipérazinyl-1)-3 propyl] amino-3 benzophénone-oxime E, sous la forme d'une huile orangée [Rf = 0,32, chromatographie couche mince de silice ; éluant : acétate d'éthyle-diéthylamine (95-5 en volumes)].

L'amino-3 benzophénone-oxime enrichie à 80 % en forme E est obtenue comme décrit ci-dessus.

### EXEMPLE 30

1 g de (+)-N-{{α-[(méthyl-1 pipéridyl-4) oxyimino] benzyl}-3 phényl} (pyridyl-3)-3 1H,3H-pyrrolo[1,2-c] thiazolecarboxamide-7, à l'état de mélange des formes E et Z est chromatographié sur une colonne de 4 cm de diamètre contenant 100 g environ de silice (0,02-0,045 mm). On élue par un mélange d'éther diéthylique et de diéthylamine (85-15 en volumes) sous une pression de 0,2 bar (20 kPa) en recueillant des fractions de 25 cm3. Les 20 premières fractions sont éliminées. Les 11 fractions suivantes correspondant à la première forme d'oxime éluée, appelée forme A sont réunies, ainsi que les 15 fractions suivantes, correspondant à un mélange enrichi à 70 % en la deuxième forme d'oxime appelée forme B. Après évaporation du solvant sous pression réduite (20 mm de mercure ; 2,7 kPa) à une température voisine de 40°C on obtient 0,4 g de forme A et 0,6 g d'un mélange enrichi en forme B.

### EXEMPLE 31

La forme A (0,4 g) est dissoute dans 1 cm3 d'éthanol absolu, filtrée puis additionnée successivement de 0,9 cm3 d'éther chlorhydrique 0,4N, 3 cm3 d'acétate d'éthyle, 0,9 cm3 d'éther chlorhydrique 0,4N et 5 cm3 d'éther diéthylique. Après 3 heures d'agitation à température ambiante, les cristaux apparus sont séparés par filtration et séchés sous pression réduite (0,1 mm de mercure ; 13,5 Pa) à une température voisine de 20°C. On obtient 0,18 g de dichlorhydrate de (+)-N-{{α-[(méthyl-1 pipéridyl-4) oxyimino] benzyl}-3 phényl} (pyridyl-3)-3 1H,3H-pyrrolo[1,2-c] thiazolecarboxamide-7, forme A, fondant à 175-180°C.

### EXEMPLE 32

Le produit enrichi à 70 % en forme B (0,6 g) est chromatographié une deuxième fois sur une colonne de 4 cm de diamètre contenant environ 150 g,de silice (0,02-0,045 mm). On élue par un mélange d'éther diéthylique et de diéthylamine (85-15 en volumes) sous une pression de 0,2 bar (20 kPa) en recueillant des fractions de 25 cm3. Les 20 premières fractions sont éliminées. Les 18 fractions suivantes sont réunies et concentrées à sec sous pression réduite (20 mm de mercure ; 2,7 kPa) à une température voisine de 40°C. On obtient ainsi 0,39 g de forme B pure qui est dissoute dans 2 cm3 d'éthanol absolu, traitée par 0,1 g de noir décolorant, filtrée et additionnée de 2 cm3 d'éthanol absolu et de 10 cm3 d'acétate d'éthyle. On ajoute alors, sous agitation, 0,18 cm3 d'éther chlorhydrique 4N et après 1 heure à température ambiante, les cristaux sont séparés par filtration et séchés sous pression réduite (0,1 mm de mercure ; 13,5 Pa) à une température voisine de 20°C. On obtient 0,31 g de monochlorhydrate de(+)-N-{{α-[(méthyl-1 pipéridyl-4) oxyimino] benzyl}-3 phényl} (pyridyl-3)-3 1H,3H-pyrrolo[1,2-c] thiazolecarboxamide-7, forme B, fondant à 264°C.

### EXAMPLE 33

On dissout 11,8 g de (+)-N-{{α-[(méthyl-1 pipéridyl-4) oxyimino] benzyl}-3 phényl} (pyridyl-3)-3 1H,3H-pyrrolo[1,2-c] thiazolecarboxamide-7 (mélange des formes E et Z) dans 55 cm3 d'éthanol absolu et on additionne 6 cm3 d'éther chlorhydrique 3,65M. Après 2 heures d'agitation à température ambiante, les cristaux sont séparés par filtration et séchés sous pression réduite (0,1 mm de mercure ; 13,5 Pa) à une température voisine de 20°C. On obtient 8,0 g de monochlorhydrate d'un produit enrichi à 85 % en forme B. Le filtrat est concentré à sec sous pression réduite (20 mm de mercure ; 2,7 kPa) à une température voisine de 40°C. On obtient 4,9 g d'une huile marron enrichie à 90% en forme A.

Le solide (forme B majoritaire) est mis en suspension dans 100 cm3 d'éthanol absolu et chauffé au reflux pendant 5 minutes puis la température est amenée à 20°C. Les cristaux sont séparés par filtration et séchés sous pression réduite (0,1 mm de mercure ; 13,5 Pa) à une température voisine de 20°C. On obtient 6,6 g de monochlorhydrate de la forme B pure. Le filtrat, évaporé à sec sous pression réduite (20 mm de mercure ; 2,7 kPa) à une température voisine de 40°C, conduit à 3,7 g d'une meringue jaune qui sera utilisée dans la préparation de l'oxime de forme A. Les 6,6 g de monochlorhydrate de forme B pure sont alors additionnés de 25 cm3 d'acétone puis de 14 cm3 de soude 1N et sont extraits par 50 cm3 au total d'acétate d'éthyle. Les extraits organiques sont lavés à l'eau distillée juqu'à neutralité, séchés sur sulfate de magnésium anhydre et concentrés à sec sous pression réduite (20 mm de mercure ; 2,7 kPa) à une température voisine de 40°C. On obtient 6,2 g d'une meringue blanche. A 4 g du produit dissous dans 140 cm3 d'acétonitrile on ajoute de 7,4 cm3 d'éthanol sulfurique 1M. Après 15 minutes d'agitation à température ambiante, les cristaux apparus sont séparés par filtration, lavés par 20 cm3 d'acétonitrile et 40 cm3 d'éther diéthylique et séchés sous pression réduite (0,1 mm de mercure ; 13,5 Pa) à une température voisine de 20°C. On obtient ainsi 3,5 g de (+)-N-{{α-[(méthyl-1 pipéridyl-4) oxyimino] benzyl}-3 phényl} (pyridyl-3)-3 1H,3H-pyrrolo[1,2-c] thiazolecarboxamide-7, forme B, sous forme de sulfate acide fondant à 145°C.

Par ailleurs, les 4,9 g d'huile obtenus (forme A à 90 %) sont repris par 35 cm3 d'acétone et additionnés de 20 cm3 de soude 1N. La phase aqueuse est extraite 2 fois par 100 cm3 au total d'acétate d'éthyle. Les extraits organiques sont réunis, lavés à l'eau distillée jusqu'à neutralité, séchés sur du sulfate de magnésium anhydre, filtrés et concentrés sous pression réduite (20 mm de mercure ; 2,7 kPa) à une température voisine de 45°C. On obtient ainsi 4,85 g d'un résidu orange pâteux. Ce produit est chromatographié sur une colonne de 6 cm de diamètre contenant environ 500 g de silice (0,02-0,045 mm). On élue par un mélange d'éther diéthylique et de diéthylamine (85-15 en volumes) sous une pression de 0,5 bar (51 kPa) en recueillant des fractions de 100 cm3. Les 33 premières fractions sont éliminées. Les 19 fractions suivantes composées de forme A pure sont réunies en un seul lot ; les 17 dernières enrichies en forme A forment un deuxième lot. Le solvant est évaporé à sec sous pression réduite (20 mm de mercure ; 2,7 kPa) à une temperature voisine de 40°C. On obtient ainsi 2,0 g de forme A pure et 1,5 g où A est majoritaire.

Ces 1,5 g et les 3,7 g de meringue obtenues précédemment lors de la purification de la forme B sont réunis et chromatographiés sur une colonne de 4 cm contenant 150 g de silice (0,043-0,063 mm). On élue par un mélange d'éther diéthylique et de diéthylamine (88-12 en volumes) sous une pression de 0,5 bar (51 kPa) en recueillant des fractions de 100 cm3. Les 46 premières fractions sont éliminées. Les 20 fractions suivantes sont réunies et concentrées à sec sous pression réduite (20 mm de mercure ; 2,7 kPa) à une température voisine de 40°C. On obtient ainsi 2,5 g de forme A pure.

Ce lot et les 2 g déjà obtenus sont dissous dans 40 cm3 d'acétate d'éthyle, traités par 0,2 g de noir décolorant. Après filtration, la solution est additionnée de 4,1 cm3 d'éther chlorhydrique 3,65N et est agitée à une température voisine de 20°C pendant 15 minutes. Les cristaux sont séparés par filtration, lavés successivement par 20 cm3 d'acétate d'éthyle et 20 cm3 au total d'éther diéthylique et séchés sous pression réduite (0,1 mm de mercure ; 13,5 Pa) à une température voisine de 20°C. On obtient ainsi 4,1 g de (+)-N-{{α-[(méthyl-1 pipéridyl-4) oxyimino] benzyl}-3 phényl} (pyridyl-3)-3 1H,3H-pyrrolo[1,2-c] thiazolecarboxamide-7, forme A, sous la forme de dichlorhydrate, fondant à 166°C.

### EXEMPLE 34

A une solution de 4,1 g de O-(méthyl-1 pipéridyl-4) amino-3 benzophénone-oxime Z dans 50 cm3 de dichlorométhane, on ajoute, sous agitation 4,8 g de chlorhydrate de chloroformyl-7 (pyridyl-3)-3 1H,3H-pyrrolo[1,2-c] thiazole et 4,5 cm3 de triéthylamine. La suspension est agitée 15 heures à température ambiante, puis on rajoute 0,48 g de chlorhydrate de chloroformyl-7 (pyridyl-3)-3 1H,3H-pyrrolo[1,2-c] thiazole. Après une heure d'agitation, 50 cm3 d'eau distillée sont ajoutés et la phase organique est décantée et lavée par 100 cm3 au total d'eau distillée. Les extraits organiques sont séchés sur du sulfate de magnésium anhydre, traités en présence de noir décolorant, filtrés et concentrés sous pression réduite (20 mm de mercure ; 2,7 kPa) à une température voisine de 45°C. On obtient ainsi 6,8 g d'un résidu orange pâteux. Ce produit est chromatographié sur une colonne de 6 cm de diamètre contenant 450 g environ de silice (0,02-0,045 mm). On élue par un mélange d'acétate d'éthyle et de diéthylamine (95-5 en volumes) sous une pression de 0,5 bar (51 kPa) en recueillant des fractions de 100 cm3. Les 16 premières fractions sont éliminées. Les 24 fractions suivantes sont réunies et concentrées à sec sous pression réduite (20 mm de mercure ; 2,7 kPa) à une température voisine de 40°C. On obtient ainsi 4,4 g de produit, sous forme d'une meringue beige qui, additionnés de 1,7 g provenant d'une autre opération est chromatographiée une deuxième fois sur une colonne de 2,7 cm de diamètre contenant 80 g de silice (0,02-0,045 mm) environ. On élue par un mélange d'acétate d'éthyle et de diéthylamine (95-5 en volumes) sous une pression de 0,5 bar (51 kPa) en recueillant des fractions de 30 cm3. Les 15 premières fractions sont éliminées. Les 35 fractions suivantes sont réunies et concentrées à sec sous pression réduite (20 mm de mercure ; 2,7 kPa) à une température voisine de 40°C. On obtient ainsi 5,5 g d'une meringue beige qui est dissoute dans 120 cm3 d'acétonitrile et on ajoute 10,2 cm3 d'éthanol sulfurique 1M. Après 1 heure d'agitation à température ambiante, les cristaux apparus sont séparés par filtration, lavés par 50 cm3 d'éther éthylique et séchés sous pression réduite (0,1 mm de mercure ; 13,5 Pa) à une température voisine de 20°C. On obtient 5,9 g de (+)-N-{{α-[méthyl-1 pipéridyl-4) oxyimino] benzyl}-3 phényl} (pyridyl-3)-3 1H,3H-pyrrolo[1,2-c] thiazolecarboxamide-7, forme B, sous forme de sulfate acide fondant à 153°C.

La O-(méthyl-1 pipéridyl-4) amino-3 benzophénone-oxime Z peut être préparée de la manière suivante : à une solution de 3,48 g d'amino-3 benzophénone-oxime Z dans 30 cm3 de N,N-diméthylformamide anhydre, on ajoute, à température ambiante, sous courant d'azote et sous agitation 0,72 g d'hydrure de sodium (en dispersion à 60 % dans l'huile de vaseline). La suspension est agitée à température ambiante pendant 45 minutes et une solution de 5,4 g de paratoluènesulfonate de méthyl-1 pipéridyle-4 dans 50 cm3 de N,N-diméthylformamide anhydre est ensuite introduite en 15 minutes environ. La solution est alors agitée pendant 1 heure et 20 minutes à température ambiante puis 0,2, 0,3 et 0,6 g de paratoluènesulfonate de méthyl-1 pipéridyle-4 sont ajoutés à respectivement 1, 15 et 4 heures d'intervalle. On additionne alors 0,14 g d'hydrure de sodium (en dispersion à 60 % dans l'huile de vaseline) et une solution de 1,8 g de paratoluènesulfonate de méthyl-1 pipéridyle-4 dans cm3 de N,N-diméthylformamide anhydre. La solution est agitée pendant 15 heures à température ambiante puis on ajoute 300 cm3 d'eau distillée et la solution est extraite 4 fois avec 400 cm3 au total d'acétate d'éthyle. Les extraits organiques sont réunis, lavés par 500 cm3 d'eau distillée, séchés sur du sulfate de magnésium anhydre, filtrés et concentrés sous pression réduite (20 mm de mercure ; 2,7 kPa) à une température voisine de 45°C. On obtient ainsi 6,8 g d'un résidu orange pâteux. Ce produit est chromatographié sur une colonne de 6 cm de diamètre contenant 500 g environ de silice (0,02-0,045 mm). On élue par un mélange d'acétate d'éthyle et de diéthylamine (95-5 en volumes) sous une pression de 0,5 bar (51 kPa) en recueillant des fractions de 80 cm3. Les 15 premières fractions sont éliminées. Les 25 fractions suivantes sont réunies et concentrées à sec sous pression réduite (20 mm de mercure ; 2,7 kPa) à une température voisine de 40°C. On obtient ainsi 4,13 g de O-(méthyl-1 pipéridyl-4) amino-3 benzophénone-oxime Z, sous la forme d'une huile jaune.

L'amino-3 benzophénone-oxime Z peut être préparée comme dans l'exemple 28.

Le paratoluènesulfonate de méthyl-1 pipéridyle-4 peut être préparé comme décrit à l'exemple 27.

La présente invention concerne également les médicaments constitués par un produit de formule générale (I), sous forme libre ou sous forme de sel d'addition avec un acide pharmaceutiquement acceptable, à l'état pur ou sous forme d'une composition dans laquelle il est associé à tout autre produit pharmaceutiquement compatible pouvant être inerte ou physiologiquement actif. Les médicaments selon l'invention peuvent être utilisés par voie orale, parentérale, rectale ou topique.

Comme compositions solides pour administration orale peuvent être utilisés des comprimés, pilules, poudres (notamment dans des capsules en gélatine ou des cachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétables ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou tout autre solvant organique convenable.

Ces compositions peuvent également contenir des adjuvants en particulier des agents mouillants, isotonisants, émulsifiants, dispersant et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu injectable stérile.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les produits selon l'invention sont particulièrement utiles dans le traitement de toutes les conditions pathologiques dans lesquelles le PAF-acéther peut être incriminé directement ou indirectement, notamment les affections allergiques, inflammatoires et les affections du système digestif tels que les ulcères, les colites et les lésions intestinales causées par les irradiations ou les chocs endotoxiniques.

Les doses dépendent de l'effet recherché et de la durée du traitement. Elles sont généralement comprises entre 25 et 300 mg par jour par voie orale, intraveineuse ou par inhalation pour un adulte en une ou plusieurs prises. D'une façon générale, le médecin déterminera la posologie qu'il estime être la plus appropriée en fonction de l'age, du poids et de tout autre facteur propre au sujet à traiter.

D'un intérêt tout particulier sont les produits de formule générale (I) dans laquelle R₁ représente un atome d'hydrogène ou d'halogène ou un radical alcoyloxy dont la partie alcoyle contient 1 à 4 atomes de carbone, Ar représente un radical phényle ou pyridyle, p est égal à 0, 1 ou 2 et :
A - soit Z représente une liaison de valence et R₂ représente un atome d'hydrogène
B- soit Z représente une liaison de valence et R₂ représente un radical quinuclidinyle ou pipéridyle-3 ou -4 dont l'atome d'azote est éventuellement substitué par un radical alcoyle contenant 1 à 4 atomes de carbone,
C- soit Z représente un radical alcoylène contenant 1 à 4 atomes de carbone et R₂ représente un radical pipéridyle-2, -3 ou -4 dont l'atome d'azote est éventuellement substitué par un radical alcoyle contenant 1 à 4 atomes de carbone ou bien R₂ représente un radical 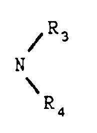 dans lequel R₃ représente un atome d'hydrogène ou un radical alcoyle contenant 1 à 4 atomes de carbone ou phényle et R₄ représente un radical 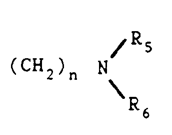 dans lequel n est un nombre entier compris entre 1 et 4 inclusivement et R₅ et R₆, identiques ou différents, représentent un atome d'hydrogène ou un radical alcoyle contenant 1 à 4 atomes de carbone ou bien R₂ représente un radical 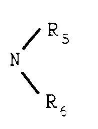 dans lequel R₅ et R₆, identiques ou différents, représentent un radical alcoyle contenant 1 à 4 atomes de carbone ou phénylalcoyle dont la partie alcoyle contient 1 à 4 atomes de carbone ou bien R₅ et R₆ forment ensemble avec l'atome d'azote auquel ils sont liés un cycle morpholine, pipéridine ou pipérazine dont le second atome d'atoe peut être éventuellement substitué par un radical alcoyle contenant 1 à 4 atomes de carbone, hydroxyalcoyle dont la partie alcoyle contient 1 à 4 atomes de carbone ou phénylalcoyle dont la partie alcoyle contient 1 à 4 atomes de carbone, leurs énantiomères, leurs diastéréoisomères et leurs isomères E et Z pris seuls ou en mélanges ainsi que leurs sels pharmaceutiquement acceptables.

Plus particulièrement encore sont intéressants les produits suivants :
- (+)-N-{{α-[(méthyl-4 pipérazinyl-1)-3 propoxyimino] benzyl}-3 phényl} (pyridyl-3)-3 1H,3H-pyrrolo[1,2-c] thiazolecarboxamide-7, mélange des formes E et Z ;
- (+)-N-{[α-(diméthylamino-2 éthoxyimino) benzyl]-3 phényl} (pyridyl-3)-3 1H,3H-pyrrolo[1,2-c] thiazolecarboxamide-7, mélange des formes E et Z ;
- (+)-N-{[α-(morpholino-2 éthoxyimino) benzyl]-3 phényl} (pyridyl-3)-3 1H,3H-pyrrolo[1,2-c] thiazolecarboxamide-7, mélange des formes E et Z ;
- (+)-N-{{α-[(méthyl-4 pipérazinyl-1)-2 éthoxyimino] benzyl}-3 phényl} (pyridyl-3)-3 1H,3H-pyrrolo[1,2-c] thiazolecarboxamide-7, mélange des formes E et Z ;
- (+)-N-{{α-[(méthyl-1 pipéridyl-4) oxyimino] benzyl}-3 phényl} (pyridyl-3)-3 1H,3H-pyrrolo[1,2-c] thiazolecarboxamide-7, mélange des formes E et Z ;
- (+)-N-{{α-[(méthyl-4 pipérazinyl-1)-3 propoxyimino] benzyl}-3 phényl} (pyridyl-3)-3 1H,3H-pyrrolo[1,2-c] thiazolecarboxamide-7, forme A ;
- (+)-N-{{α-[(méthyl-1 pipéridyl-4) oxyimino] benzyl}-3 phényl} (pyridyl-3)-3 1H,3H-pyrrolo[1,2-c] thiazolecarboxamide-7, forme B ;
- (+)-N-{{α-[(méthyl-1 pipéridyl-4) oxyimino] benzyl}-3 phényl} (pyridyl-3)-3 1H,3H-pyrrolo[1,2-c] thiazolecarboxamide-7, mélange des formes E et Z ;
- (+)-N{{α-[(méthyl-1 pipéridyl-4) oxyimino] benzyl}-3 phényl} (pyridyl-3)-3 1H,3H-pyrrolo[1,2-c] thiazolecarboxamide-7 oxyde-2 (2RS,3R), mélange des formes E et Z ;
- (+)-N-{{α-[(pipérazinyl-1)-3 propoxyimino] benzyl}-3 phényl) (pyridyl-3)-3 1H,3H-pyrrolo[1,2-c] thiazolecarboxamide-7, mélange des formes E et Z ;
- (+)-N-{{{α-[(hydroxy-2 éthyl)-4 pipérazinyl-1]-3 propoxyimino} benzyl}-3 phényl} (pyridyl-3)-3 1H,3H-pyrrolo[1,2-c] thiazolecarboxamide-7, mélange des formes E et Z ;
- (+)-N-{{α-[(méthyl-1 pipéridyl-3-(3RS)) oxyimino] benzyl}-3 phényl} (pyridyl-3)-3 1H,3H-pyrrolo[1,2-c] thiazolecarboxamide-7-(3R), mélange des formes E et Z.

L'exemple suivant illustre une composition selon l'invention.

### EXEMPLE A

On prépare selon la technique habituelle des doses unitaires injectables contenant 25 mg de produit actif en dissolvant 366,79 mg de diméthanesulfonate de (+)-N-{[α-(morpholino-2 éthoxyimino) benzyl]-3 phényl} (pyridyl-3)-3 1H,3H-pyrrolo[1,2-c] thiazolecarboxamide-7 (mélange des formes E et Z) dans 25 cm3 d'eau distillée et en répartissant de façon égale la solution obtenue dans 10 ampoules identiques.

## Revendications (Revendications pour l'(les) Etat(s) contractant(s) suivant(s): AT, BE, CH, DE, DK, FR, GB, IT, LI, LU, NL, SE)

1. Nouveaux dérivés du 1H,3H-pyrrolo[1,2-c] thiazolecarboxamide-7, caractérisés en ce qu'ils répondent à la formule générale : 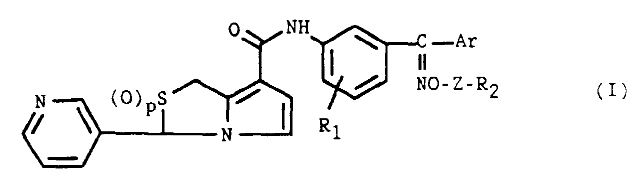 dans laquelle R₁ représente un atome d'hydrogène ou d'halogène ou un radical alcoyle, alcoyloxy, trifluorométhyle, amino, alcoylamino, dialcoylamino, hydroxy, cyano, phényle ou phénoxy, Ar représente un radical phényle, naphtyle, pyridyle, quinolyle, isoquinolyle, thiényle, thiéno[2,3-b] thiényle-2 ou thiéno[3,2-b] thiényle-2, ces radicaux pouvant éventuellement porter un ou plusieurs substituants choisis parmi les atomes d'halogène ou les radicaux alcoyle, alcoyloxy, trifluorométhyle, amino, alcoylamino, dialcoylamino, hydroxy ou cyano, p représente un nombre entier égal à zéro, un ou deux et
A- soit Z représente une liaison de valence et R₂ représente un atome d'hydrogène,
B- soit Z représente une liaison de valence et R₂ représente un radical pyridyle-2 ou -4, quinuclidinyle-3, pyrrolidinyle-3, pipéridyle-3 ou -4, ces deux derniers radicaux pouvant être éventuellement substitués sur l'atome d'azote par un radical alcoyle, hydroxyalcoyle, phényle ou phénylalcoyle
C- soit Z représente un radical alcoylène contenant 1 à 4 atomes de carbone et R₂ représente un radical pyridyle-2, -3 ou -4, quinuclidinyle-3, pyrrolidinyle-2 ou -3, pipéridyle-2, -3 ou -4, ces deux derniers radicaux pouvant être substitués sur l'atome d'azote par un radical alcoyle, hydroxyalcoyle, phényle ou phénylalcoyle ou bien R₂ représente un radical de formule générale : 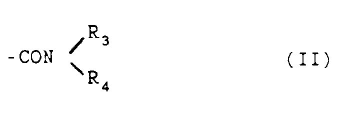 dans laquelle :
a) soit R₃ et R₄ forment ensemble avec l'atome d'azote auquel ils sont liés un cycle pipérazine éventuellement substitué sur le deuxième atome d'azote par un radical alcoyle, hydroxyalcoyle, pyridyle, phényle ou phénylalcoyle,
b) soit R₃ représente un atome d'hydrogène ou un radical alcoyle, phényle ou phénylalcoyle ou un radical de formule générale : 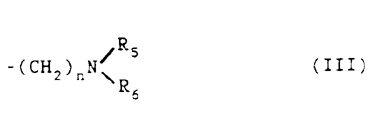 dans laquelle n représente un nombre entier compris entre 1 et 4 et R₅ et R₆, identiques ou différents, représentent un atome d'hydrogène ou un radical alcoyle, phényle, phénylalcoyle, ou bien R₅ et R₆ forment ensemble avec l'atome d'azote auquel ils sont liés un cycle morpholine, thiomorpholine, pyrrolidine, pipéridine ou pipérazine dont le second atome d'azote peut être éventuellement substitué par un radical alcoyle, hydroxyalcoyle, pyridyle, phényle, phénylalcoyle ou phénylcarbonyle, et R₄ représente un radical de formule générale (III) défini comme précédemment, étant entendu que les définitions de n, R₅ et R₆ dans les symboles R₃ et R₄ peuvent être identiques ou différents,
D- soit Z représente un radical alcoylène contenant 1 à 4 atomes de carbone et R₂ représente un radical de formule générale : 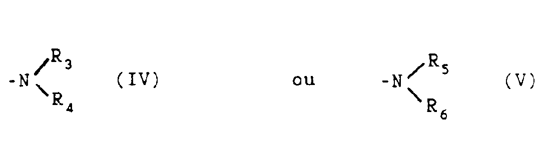 dans lesquelles R₃, R₄, R₅ et R₆ sont définis comme précédemment en C a) et b), étant entendu que les radicaux alcoyle et portions alcoyle contiennent 1 à 4 atomes de carbone en chaîne droite ou ramifiée et que l'invention concerne les produits racémiques, les énantiomères dus à la présence d'un carbone asymétrique en position 3 du cycle pyrrolothiazole, les mélanges de ces énantiomères, les diastéréoisomères purs ou en mélange dus à la présence éventuelle d'un autre centre chiral, les isomères E et Z (encore appelés syn et anti) et leurs mélanges provenant de la présence de la fonction oxime C=NO-Z-R₂, ainsi que les sels pharmaceutiquement acceptables des produits de formule (I) ainsi définie.

2. Procédé de préparation de nouveaux dérivés selon la revendication 1, caractérisé en ce que l'on fait réagir une hydroxylamine de formule générale :
H₂NO-Z-R₂ (VI)
dans laquelle Z et R₂ sont définis comme à la revendication 1 sur une cétone de formule générale : 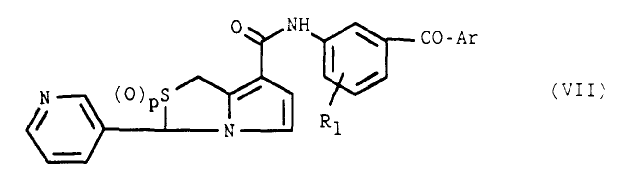 dans laquelle les symboles p, R₁ et Ar sont définis comme à la revendication 1.

3. Procédé de préparation de nouveaux dérivés selon la revendication 1 dans la formule desquels les symboles sont définis comme à la revendication 1 en B ou en D, caractérisé en ce que l'on fait réagir un produit de formule générale :
X-Z-R₂ (VIII)
dans laquelle Z et R₂ sont définis à la revendication 1 et X représente un atome d'halogène sur un produit de formule générale (I) défini comme à la revendication 1 en A, c'est-à-dire un produit de formule générale : 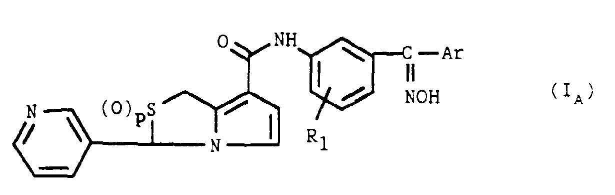 dans laquelle p, R₁ et Ar sont définis comme à la revendication 1.

4. Procédé de préparation de nouveaux dérivés selon la revendication 1 dans la formule desquels p est égal à 0 caractérisé en ce que l'on fait réagir un produit de formule générale : 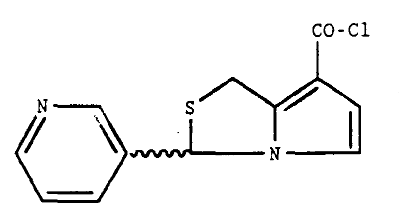 sous forme R ou S ou leurs mélanges, sur une oxime de formule générale : 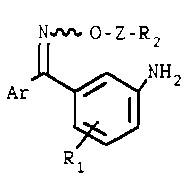 dans laquelle Ar, Z, R₁ et R₂ sont définis comme dans la revendication 1, sous forme E ou Z ou leurs mélanges.

5. Procédé de préparation de nouveaux dérivés selon la revendication 1 dans la formule desquels p est égal à 1 caractérisé en ce que l'on oxyde un nouveau dérivé selon la revendication 1 dans la formule duquel p est égal à 0.

6. Compositions pharmaceutiques, caractérisées en ce qu'elles contiennent au moins un dérivé selon la revendication 1, en association avec un ou plusieurs diluants ou adjuvants compatibles et pharmaceutiquement acceptables.

## Revendications (Revendications pour l'(les) Etat(s) contractant(s) suivant(s): ES, GR)

1. Procédé de préparation de nouveaux dérivés du 1H,3H-pyrrolo[1,2-c] thiazolecarboxamide-7, caractérisés en ce qu'ils répondent à la formule générale : 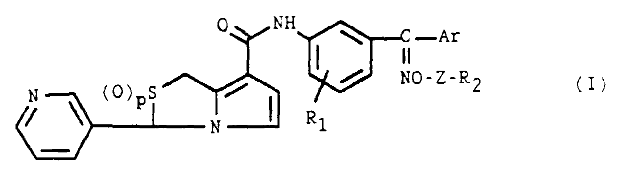 dans laquelle R₁ représente un atome d'hydrogène ou d'halogène ou un radical alcoyle, alcoyloxy, trifluorométhyle, amino, alcoylamino, dialcoylamino, hydroxy, cyano, phényle ou phénoxy, Ar représente un radical phényle, naphtyle, pyridyle, quinolyle, isoquinolyle, thiényle, thiéno[2,3-b] thiényle-2 ou thiéno[3,2-b] thiényle-2, ces radicaux pouvant éventuellement porter un ou plusieurs substituants choisis parmi les atomes d'halogène ou les radicaux alcoyle, alcoyloxy, trifluorométhyle, amino, alcoylamino, dialcoylamino, hydroxy ou cyano, p représente un nombre entier égal à zéro, un ou deux et
A- soit Z représente une liaison de valence et R₂ représente un atome d'hydrogène,
B- soit Z représente une liaison de valence et R₂ représente un radical pyridyle-2 ou -4, quinuclidinyle-3, pyrrolidinyle-3, pipéridyle-3 ou -4, ces deux derniers radicaux pouvant être éventuellement substitués sur l'atome d'azote par un radical alcoyle, hydroxyalcoyle, phényle ou phénylalcoyle
C- soit Z représente un radical alcoylène contenant 1 à 4 atomes de carbone et R₂ représente un radical pyridyle-2, -3 ou -4, quinuclidinyle-3, pyrrolidinyle-2 ou -3, pipéridyle-2, -3 ou -4, ces deux derniers radicaux pouvant être substitués sur l'atome d'azote par un radical alcoyle, hydroxyalcoyle, phényle ou phénylalcoyle ou bien R₂ représente un radical de formule générale : 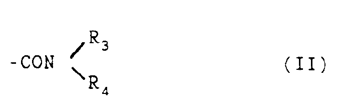 dans laquelle :
a) soit R₃ et R₄ forment ensemble avec l'atome d'azote auquel ils sont liés un cycle pipérazine éventuellement substitué sur le deuxième atome d'azote par un radical alcoyle, hydroxyalcoyle, pyridyle, phényle ou phénylalcoyle,
b) soit R₃ représente un atome d'hydrogène ou un radical alcoyle, phényle ou phénylalcoyle ou un radical de formule générale : 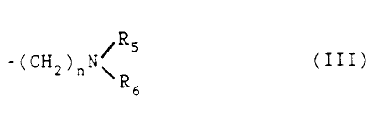 dans laquelle n représente un nombre entier compris entre 1 et 4 et R₅ et R₆, identiques ou différents, représentent un atome d'hydrogène ou un radical alcoyle, phényle, phénylalcoyle, ou bien R₅ et R₆ forment ensemble avec l'atome d'azote auquel ils sont liés un cycle morpholine, thiomorpholine, pyrrolidine, pipéridine ou pipérazine dont le second atome d'azote peut être éventuellement substitué par un radical alcoyle, hydroxyalcoyle, pyridyle, phényle, phénylalcoyle ou phénylcarbonyle, et R₄ représente un radical de formule générale (III) défini comme précédemment, étant entendu que les définitions de n, R₅ et R₆ dans les symboles R₃ et R₄ peuvent être identiques ou différents,
D- soit Z représente un radical alcoylène contenant 1 à 4 atomes de carbone et R₂ représente un radical de formule générale : 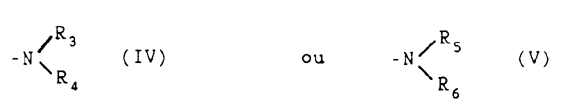 dans lesquelles R₃, R₄, R₅ et R₆ sont définis comme précédemment en C a) et b), étant entendu que les radicaux alcoyle et portions alcoyle contiennent 1 à 4 atomes de carbone en chaîne droite ou ramifiée, ainsi que les produits racémiques, les énantiomères dus à la présence d'un carbone asymétrique en position 3 du cycle pyrrolothiazole, les mélanges de ces énantiomères, les diastéréoisomères purs ou en mélange dus à la présence éventuelle d'un autre centre chiral, les isomères E et Z (encore appelés syn et anti) et leurs mélanges provenant de la présence de la fonction oxime C=NO-Z-R₂, et les sels pharmaceutiquement acceptables, caractérisé en ce que l'on fait réagir une hydroxylamine de formule générale :
H₂NO-Z-R₂ (VI)
dans laquelle Z et R₂ sont définis comme à la revendication 1 sur une cétone de formule générale : 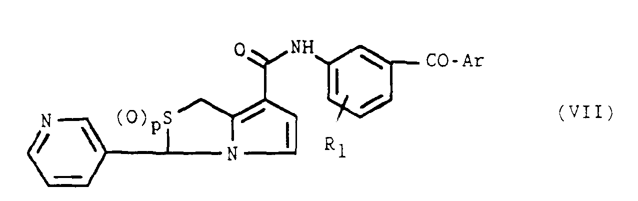 dans laquelle les symboles p, R₁ et Ar sont définis comme à la revendication 1.

2. Procédé de préparation de nouveaux dérivés tels que définis dans la revendication 1 dans la formule desquels les symboles sont définis comme dans la revendication 1 en B ou en D, caractérisé en ce que l'on fait réagir un produit de formule générale :
X-Z-R₂ (VIII)
dans laquelle Z et R₂ sont définis à la revendication 1 et X représente un atome d'halogène sur un produit de formule générale (I) défini comme à la revendication 1 en A, c'est-à-dire un produit de formule générale : 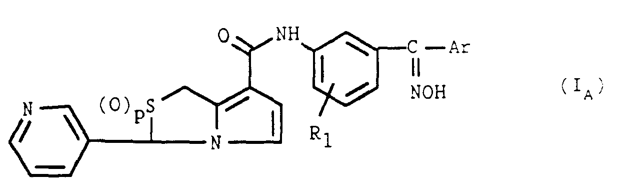 dans laquelle p, R₁ et Ar sont définis comme à la revendication 1.

3. Procédé de préparation de nouveaux dérivés tels que définis dans la revendication 1 dans la formule desquels p est égal à 0 caractérisé en ce que l'on fait réagir un produit de formule générale : 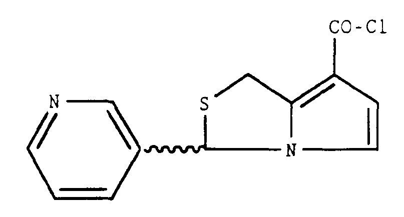 sous forme R ou S ou leurs mélanges, sur une oxime de formule générale : 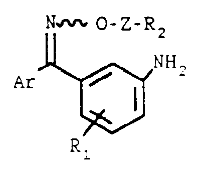 dans laquelle Ar, Z, R₁ et R₂ sont définis comme dans la revendication 1, sous forme E ou Z ou leurs mélanges.

4. Procédé de préparation de nouveaux dérivés tels que définis dans la revendication 1 dans la formule desquels p est égal à 1 caractérisé en ce que l'on oxyde un nouveau dérivé tel que défini dans la revendication 1 dans la formule duquel p est égal à 0.

## Patentansprüche (Patentansprüche für folgende(n) Vertragsstaat(en): AT, BE, CH, DE, DK, FR, GB, IT, LI, LU, NL, SE)

1. Neue Derivate des 1H,3H-Pyrrolo[1,2-c]thiazol-7-carboxamids, dadurch gekennzeichnet, daß sie der allgemeinen Formel 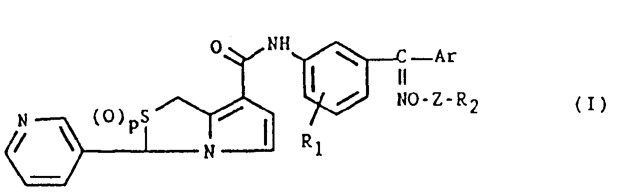 entsprechen, worin
R₁ ein Wasserstoff- oder Halogenatom bedeutet, oder einen Rest Alkyl, Alkyloxy, Trifluormethyl, Amino, Alkylamino, Dialkylamino, Hydroxy, Cyano, Phenyl oder Phenoxy darstellt,
Ar einen Rest Phenyl, Naphthyl, Pyridyl, Chinolyl, Isochinolyl, Thienyl, Thieno[2,3-b]thienyl-2 oder Thieno[3,2-b]thienyl-2 bedeutet, wobei diese Reste gegebenenfalls einen oder mehrere Substituenten tragen können, ausgewählt unter den Halogenatomen oder den Resten Alkyl, Alkyloxy, Trifluormethyl, Amino, Alkylamino, Dialkylamino, Hydroxy oder Cyano,
p eine ganze Zahl gleich 0, 1 oder 2 bedeutet, und
A- entweder bedeutet Z eine Valenzbindung und R₂ bedeutet ein Wasserstoffatom,
B- oder Z bedeutet eine Valenzbindung und R₂ bedeutet einen Rest Pyridyl-2 oder -4, Chinuclidinyl-3, Pyrrolidin-3-yl, Piperidyl-3 oder -4, wobei diese zwei letzteren Reste gegebenenfalls am Stickstoffatom durch einen Alkyl-, Hydroxyalkyl-, Phenyl- oder Phenylalkylrest substituiert sein können,
C- oder Z bedeutet einen Alkylenrest mit 1 bis 4 Kohlenstoffatomen und R₂ bedeutet einen Rest Pyridyl-2, -3 oder -4, Chinuclidin-3-yl, Pyrrolidinyl-2 oder -3, Piperidyl-2, -3 oder -4, wobei diese zwei letzteren Reste am Stickstoffatom substituiert sein können durch einen Alkyl-, Hydroxyalkyl-, Phenyl- oder Phenylalkylrest oder R₂ bedeutet einen Rest der allgemeinen Formel 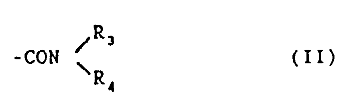 worin:
a) entweder R₃ und R₄ bilden zusammen mit dem Stickstoffatom, an das sie gebunden sind, einen Piperazinring, der gegebenenfalls am zweiten Stickstoffatom durch einen Alkyl-, Hydroxyalkyl-, Pyridyl-, Phenyl- oder Phenylalkylrest substituiert ist,
b) oder R₃ bedeutet ein Wasserstoffatom oder einen Alkyl-, Phenyl- oder Phenylalkylrest oder einen Rest der allgemeinen Formel 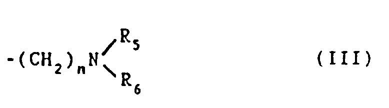 worin n eine ganze Zahl zwischen 1 und 4 bedeutet, und R₅ und R₆, die identisch oder verschieden sind, bedeuten ein Wasserstoffatom oder einen Alkyl-, Phenyl-, Phenylalkylrest, oder R₅ und R₆ bilden zusammen mit dem Stickstoffatom, an das sie gebunden sind, einen Morpholin-, Thiomorpholin-, Pyrrolidin-, Piperidin- oder Piperazinring, von dem das zweite Stickstoffatom gegebenenfalls substituiert sein kann durch einen Alkyl-, Hydroxyalkyl-, Pyridyl-, Phenyl-, Phenylalkyl- oder Phenylcarbonylrest, und R₄ bedeutet einen Rest der allgemeinen Formel (III), wie vorstehend definiert, wobei die Definitionen von n, R₅ und R₆ in den Symbolen R₃ und R₄ identisch oder verschieden sein können,
D- oder Z bedeutet einen Alkylenrest mit 1 bis 4 Kohlenstoffatomen und R₂ bedeutet einen Rest der allgemeinen Formel 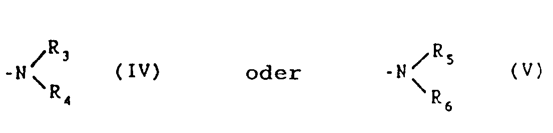 worin R₃, R₄, R₅ und R₆ wie vorstehend in C-a) und b) definiert sind, wobei die Alkylreste und Alkylteile 1 bis 4 Kohlenstoffatome in gerader oder verzweigter Kette enthalten und die Erfindung die racemischen Produkte, die Enantiomeren aufgrund der Anwesenheit eines asymmetrischen Kohlenstoffatoms in 3-Stellung des Pyrrolothiazolrings, die Gemische dieser Enantiomeren, die reinen Diastereoisomeren oder im Gemisch aufgrund der eventuellen Anwesenheit eines anderen chiralen Zentrums, die Isomeren E und Z (auch syn und anti genannt) und deren Gemische, welche von der Anwesenheit der Oximfunktion C=NO-Z-R₂ stammt, sowie die pharmazeutisch annehmbaren Salze der Produkte der so definierten Formel (I) betrifft.

2. Verfahren zur Herstellung der neuen Derivate gemäß Anspruch 1, dadurch gekennzeichnet, daß man ein Hydroxylamin der allgemeinen Formel
H₂NO-Z-R₂ (VI)
worin Z und R₂ wie in Anspruch 1 definiert sind, auf ein Keton der allgemeinen Formel 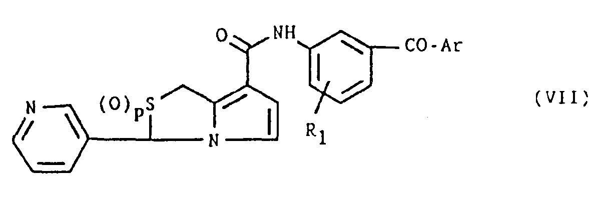 einwirken läßt, worin die Symbole p, R₁ und Ar wie in Anspruch 1 definiert sind.

3. Verfahren zur Herstellung der neuen Derivate gemäß Anspruch 1, in deren Formel die Symbole wie in Anspruch 1 in B oder in D definiert sind, dadurch gekennzeichnet, daß man ein Produkt der allgemeinen Formel
X-Z-R₂ (VIII)
worin Z und R₂ wie in Anspruch 1 definiert sind, und X ein Halogenatom bedeutet, mit einem Produkt der allgemeinen Formel (I) wie in Anspruch 1 in A definiert, d. h. ein Produkt der allgemeinen Formel 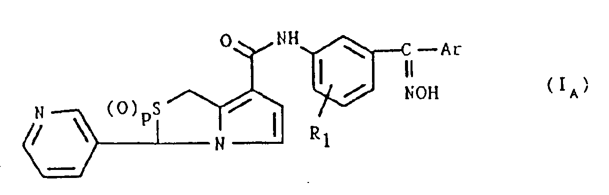 worin p, R₁ und Ar wie in Anspruch 1 definiert sind, zur Reaktion bringt.

4. Verfahren zur Herstellung der neuen Derivate gemäß Anspruch 1, in deren Formel p gleich 0 ist, dadurch gekennzeichnet, daß man ein Produkt der allgemeinen Formel 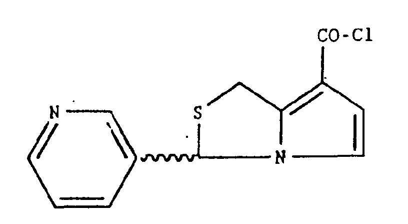 in R- oder S-Form oder deren Gemische mit einem Oxim der allgemeinen Formel 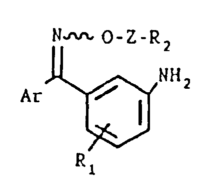 worin Ar, Z, R₁ und R₂ wie in Anspruch 1 definiert sind, in der E- oder Z-Form oder deren Gemischen zur Reaktion bringt.

5. Verfahren zur Herstellung der neuen Derivate gemäß Anspruch 1, in deren Formel p gleich 1 ist, dadurch gekennzeichnet, daß man ein neues Derivat gemäß Anspruch 1,in dessen Formel p gleich 0 ist, oxidiert.

6. Pharmazeutische Zusammensetzungen, dadurch gekennzeichnet, daß sie mindestens ein Derivat gemäß Anspruch 1 in Verbindung mit einem oder mehreren verträglichen und pharmazeutisch annehmbaren Verdünnungs- oder Hilfsmitteln enthalten.

## Patentansprüche (Patentansprüche für folgende(n) Vertragsstaat(en): ES, GR)

1. Verfahren zur Herstellung von neuen Derivaten des 1H,3H-Pyrrolo[1,2-c]thiazol-7-carboxamids, dadurch gekennzeichnet, daß sie der allgemeinen Formel 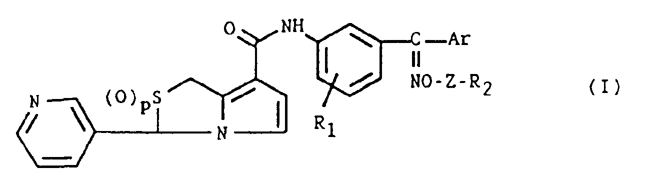 entsprechen, worin
R₁ ein Wasserstoff- oder Halogenatom bedeutet, oder einen Rest Alkyl, Alkyloxy, Trifluormethyl, Amino, Alkylamino, Dialkylamino, Hydroxy, Cyano, Phenyl oder Phenoxy darstellt,
Ar einen Rest Phenyl, Naphthyl, Pyridyl, Chinolyl, Isochinolyl, Thienyl, Thieno[2,3-b]thienyl-2 oder Thieno[3,2-b]thienyl-2 bedeutet, wobei diese Reste gegebenenfalls einen oder mehrere Substituenten tragen können, ausgewählt unter den Halogenatomen oder den Resten Alkyl, Alkyloxy, Trifluormethyl, Amino, Alkylamino, Dialkylamino, Hydroxy oder Cyano,
p eine ganze Zahl gleich 0, 1 oder 2 bedeutet, und
A- entweder bedeutet Z eine Valenzbindung und R₂ bedeutet ein Wasserstoffatom,
B- oder Z bedeutet eine Valenzbindung und R₂ bedeutet einen Rest Pyridyl-2 oder -4, Chinuclidinyl-3, Pyrrolidin-3-yl, Piperidyl-3 oder -4, wobei diese zwei letzteren Reste gegebenenfalls am Stickstoffatom durch einen Alkyl-, Hydroxyalkyl-, Phenyl- oder Phenylalkylrest substituiert sein können,
C- oder Z bedeutet einen Alkylenrest mit 1 bis 4 Kohlenstoffatomen und R₂ bedeutet einen Rest Pyridyl-2, -3 oder -4, Chinuclidin-3-yl, Pyrrolidinyl-2 oder -3, Piperidyl-2, -3 oder -4, wobei diese zwei letzteren Reste am Stickstoffatom substituiert sein können durch einen Alkyl-, Hydroxyalkyl-, Phenyl- oder Phenylalkylrest, oder R₂ bedeutet einen Rest der allgemeinen Formel 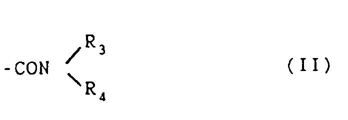 worin:
a) entweder R₃ und R₄ bilden zusammen mit dem Stickstoffatom, an das sie gebunden sind, einen Piperazinring, der gegebenenfalls am zweiten Stickstoffatom durch einen Alkyl-, Hydroxyalkyl-, Pyridyl-, Phenyl- oder Phenylalkylrest substituiert ist,
b) oder R₃ bedeutet ein Wasserstoffatom oder einen Alkyl-, Phenyl- oder Phenylalkylrest oder einen Rest der allgemeinen Formel 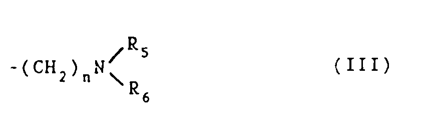 worin n eine ganze Zahl zwischen 1 und 4 bedeutet, und R₅ und R₆, die identisch oder verschieden sind, bedeuten ein Wasserstoffatom oder einen Alkyl-, Phenyl-, Phenylalkylrest, oder R₅ und R₆ bilden zusammen mit dem Stickstoffatom, an das sie gebunden sind, einen Morpholin-, Thiomorpholin-, Pyrrolidin-, Piperidin- oder Piperazinring, von dem das zweite Stickstoffatom gegebenenfalls substituiert sein kann durch einen Alkyl-, Hydroxyalkyl-, Pyridyl-, Phenyl-, Phenylalkyl- oder Phenylcarbonylrest, und R₄ bedeutet einen Rest der allgemeinen Formel (III), wie vorstehend definiert, wobei die Definitionen von n, R₅ und R₆ in den Symbolen R₃ und R₄ identisch oder verschieden sein können,
D- oder Z bedeutet einen Alkylenrest mit 1 bis 4 Kohlenstoffatomen und R₂ bedeutet einen Rest der allgemeinen Formel 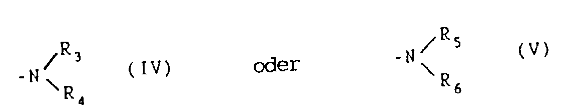 worin R₃, R₄, R₅ und R₆ wie vorstehend in C- a) und b) definiert sind, wobei die Alkylreste und Alkylteile 1 bis 4 Kohlenstoffatome in gerader oder verzweigter Kette enthalten,sowie
die racemischen Produkte, die Enantiomeren aufgrund der Anwesenheit eines asymmetrischen Kohlenstoffatoms in 3-Stellung des Pyrrolothiazolrings, die Gemische dieser Enantiomeren, die reinen Diastereoisomeren oder im Gemisch aufgrund der eventuellen Anwesenheit eines anderen chiralen Zentrums, die Isomeren E und Z (auch syn und anti genannt) und deren Gemische, welche von der Anwesenheit der Oximfunktion C=NO-Z-R₂ stammt, sowie die pharmazeutisch annehmbaren Salze, dadurch gekennzeichnet, daß man ein Hydroxylamin der allgemeinen Formel
H₂NO-Z-R₂ (VI)
worin Z und R₂ wie in Anspruch 1 definiert sind, auf ein Keton der allgemeinen Formel 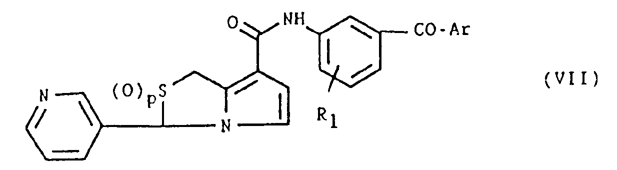 einwirken läßt, worin die Symbole p, R₁ und Ar wie in Anspruch 1 definiert sind.

2. Verfahren zur Herstellung der neuen Derivate gemäß Anspruch 1, in deren Formel die Symbole wie in Anspruch 1 in B oder in D definiert sind, dadurch gekennzeichnet, daß man ein Produkt der allgemeinen Formel
X-Z-R₂ (VIII)
worin Z und R₂ wie in Anspruch 1 definiert sind, und X ein Halogenatom bedeutet, mit einem Produkt der allgemeinen Formel (I) wie in Anspruch 1 in A definiert, d. h. ein Produkt der allgemeinen Formel 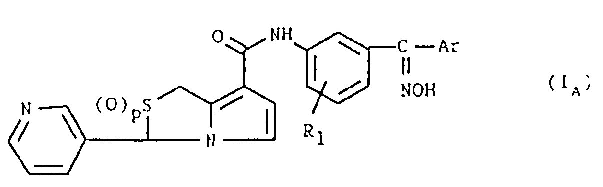 worin p, R₁ und Ar wie in Anspruch 1 definiert sind, zur Reaktion bringt.

3. Verfahren zur Herstellung der neuen Derivate gemäß Anspruch 1, in deren Formel p gleich 0 ist, dadurch gekennzeichnet, daß man ein Produkt der allgemeinen Formel 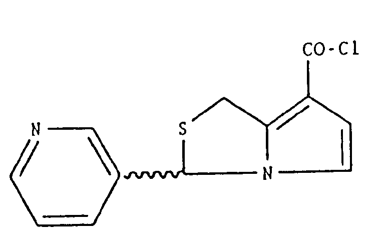 in R- oder S-Form oder deren Gemische mit einem Oxim der allgemeinen Formel 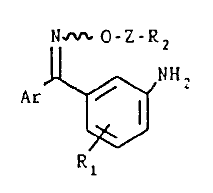 worin Ar, Z, R₁ und R₂ wie in Anspruch 1 definiert sind, in der E- oder Z-Form oder deren Gemischen zur Reaktion bringt.

4. Verfahren zur Herstellung der neuen Derivate gemäß Anspruch 1, in deren Formel p gleich 1 ist, dadurch gekennzeichnet, daß man ein neues Derivat gemäß Anspruch 1, in dessen Formel p gleich 0 ist, oxidiert.

## Claims (Claims for the following Contracting State(s): AT, BE, CH, DE, DK, FR, GB, IT, LI, LU, NL, SE)

1. New 1H,3H-pyrrolo[1,2-c]thiazole-7-carboxamide derivatives, characterized in that they correspond to the general formula: 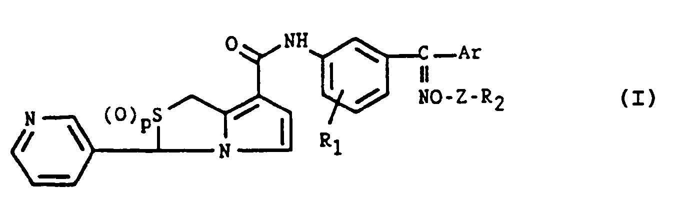 in which R₁ represents a hydrogen or halogen atom or an alkyl, alkyloxy, trifluoromethyl, amino, alkylamino, dialkylamino, hydroxy, cyano, phenyl or phenoxy radical, Ar represents a phenyl, naphthyl, pyridyl, quinolyl, isoquinolyl, thienyl, thieno[2,3-b]thien-2-yl or thieno[3,2-b]thien-2-yl, it being possible for these radicals optionally to bear one or more substituents selected from halogen atoms or alkyl, alkyloxy, trifluoromethyl, amino, alkylamino, dialkylamino, hydroxy or cyano radicals, p represents an integer equal to zero, one or two, and
A- either Z represents a valency bond and R₂ represents a hydrogen atom,
B- or Z represents a valency bond and R₂ represents a 2- or 4-pyridyl, 3-quinuclidinyl, 3-pyrrolidinyl or 3- or 4-piperidyl radical, it being possible for the latter two radicals to be optionally substituted on the nitrogen atom with an alkyl, hydroxyalkyl, phenyl or phenylalkyl radical,
C- or Z represents an alkylene radical containing 1 to 4 carbon atoms and R₂ represents a 2-, 3- or 4-pyridyl, 3-quinuclidinyl, 2- or 3-pyrrolidinyl or 2-, 3- or 4-piperidyl radical, it being possible for the latter two radicals to be substituted on the nitrogen atom with an alkyl, hydroxyalkyl, phenyl or phenylalkyl radical, or alternatively R₂ represents a radical of general formula: 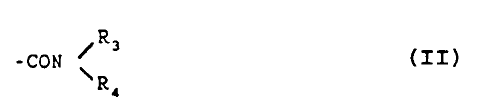 in which:
a) either R₃ and R₄, together with the nitrogen atom to which they are attached, form a piperazine ring optionally substituted on the second nitrogen atom with an alkyl, hydroxyalkyl, pyridyl, phenyl or phenylalkyl radical,
b) or R₃ represents a hydrogen atom or an alkyl, phenyl or phenylalkyl radical or a radical of general formula: 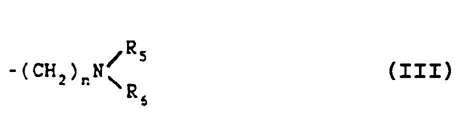 in which n represents an integer between 1 and 4 and R₅ and R₆, which may be identical or different, represent a hydrogen atom or an alkyl, phenyl or phenylalkyl radical, or alternatively R₅ and R₆, together with the nitrogen atom to which they are attached, form a morpholine, thiomorpholine, pyrrolidine, piperidine or piperazine ring in which the second nitrogen atom can be optionally substituted with an alkyl, hydroxyalkyl, pyridyl, phenyl, phenylalkyl or phenylcarbonyl radical, and R₄ represents a radical of general formula (III) defined as above, on the understanding that the definitions of n, R₅ and R₆ in the symbols R₃ and R₄ can be identical or different,
D- or Z represents an alkylene radical containing 1 to 4 carbon atoms and R₂ represents a radical of general formula: 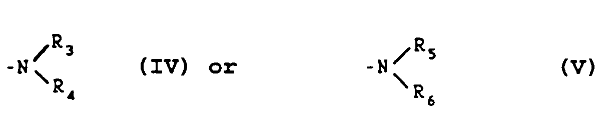 in which R₃, R₄, R₅ and R₆ are defined as above in C a) and b), on the understanding that the alkyl radicals and alkyl portions contain 1 to 4 carbon atoms in a straight or branched chain and that the invention relates to the racemic products, the enantiomers due to the presence of an asymmetric carbon at the 3-position of the pyrrolothiazole ring, the mixtures of these enantiomers, the diastereoisomers, pure or mixed, due to the possible presence of another chiral centre, and the E and Z (also known as syn and anti) isomers and mixtures thereof originating from the presence of the oxime group C=NO-Z- R₂, as well as the pharmaceutically acceptable salts of the products of formula (I) thus defined.

2. Process for preparing new derivatives according to claim 1, characterized in that a hydroxylamine of general formula:
H₂NO-Z-R₂ (VI)
in which Z and R₂ are defined as in claim 1, is reacted with a ketone of general formula: 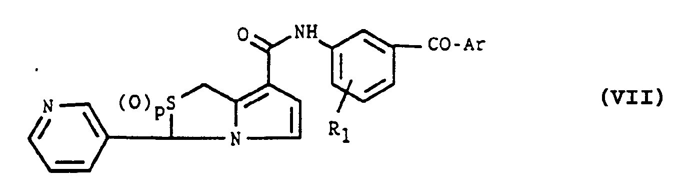 in which the symbols p, R₁ and Ar are defined as in claim 1.

3. Process for preparing new derivatives according to claim 1 in the formula of which the symbols are defined as in claim 1 in B or in D, characterized in that a product of general formula:
X-Z-R₂ (VIII)
in which Z and R₂ are defined as in claim 1 and X represents a halogen atom, is reacted with a product of general formula (I), defined as in claim 1 in A, i.e. a product of general formula: 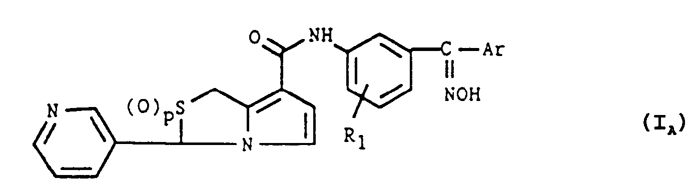 in which p, R₁ and Ar are defined as in claim 1.

4. Process for preparing new derivatives according to claim 1 in the formula of which p is equal to 0, characterized in that a product of general formula: 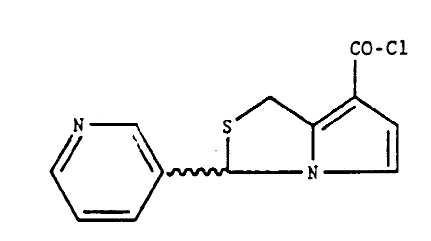 in R or S form or mixtures thereof, is reacted with an oxime of general formula: 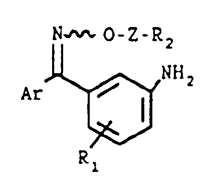 in which Ar, Z, R₁ and R₂ are defined as in claim 1, in E or Z form or mixtures thereof.

5. Process for preparing new derivatives according to claim 1 in the formula of which p is equal to 1, characterized in that a new derivative according to claim 1 in the formula of which p is equal to 0 is oxidized.

6. Pharmaceutical compositions, characterized in that they contain at least one derivative according to claim 1, in combination with one or more compatible and pharmaceutically acceptable adjuvants or diluents.

## Claims (Claims for the following Contracting State(s): ES, GR)

1. Process for preparing new 1H,3H-pyrrolo[1,2-c]thia- zole-7-carboxamide derivatives, characterized in that they correspond to the general formula: 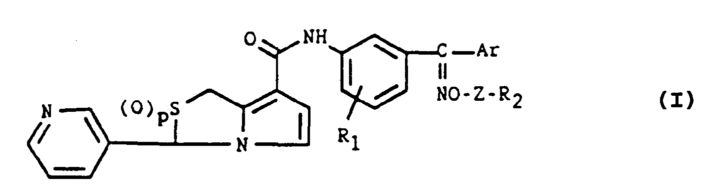 in which R₁ represents a hydrogen or halogen atom or an alkyl, alkyloxy, trifluoromethyl, amino, alkylamino, dialkylamino, hydroxy, cyano, phenyl or phenoxy radical, Ar represents a phenyl, naphthyl, pyridyl, quinolyl, isoquinolyl, thienyl, thieno[2,3-b]thien-2-yl or thieno[3,2-b]thien-2-yl, it being possible for these radicals optionally to bear one or more substituents selected from halogen atoms or alkyl, alkyloxy, trifluoromethyl, amino, alkylamino, dialkylamino, hydroxy or cyano radicals, p represents an integer equal to zero, one or two, and
A- either Z represents a valency bond and R₂ represents a hydrogen atom,
B- or Z represents a valency bond and R₂ represents a 2- or 4-pyridyl, 3-quinuclidinyl, 3-pyrrolidinyl or 3- or 4-piperidyl radical, it being possible for the latter two radicals to be optionally substituted on the nitrogen atom with an alkyl, hydroxyalkyl, phenyl or phenylalkyl radical,
C- or Z represents an alkylene radical containing 1 to 4 carbon atoms and R₂ represents a 2-, 3- or 4-pyridyl, 3-quinuclidinyl, 2- or 3-pyrrolidinyl or 2-, 3- or 4-piperidyl radical, it being possible for the latter two radicals to be substituted on the nitrogen atom with an alkyl, hydroxyalkyl, phenyl or phenylalkyl radical, or alternatively R₂ represents a radical of general formula: 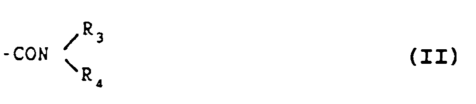 in which:
a) either R₃ and R₄, together with the nitrogen atom to which they are attached, form a piperazine ring optionally substituted on the second nitrogen atom with an alkyl, hydroxyalkyl, pyridyl, phenyl or phenylalkyl radical,
b) or R₃ represents a hydrogen atom or an alkyl, phenyl or phenylalkyl radical or a radical of general formula: 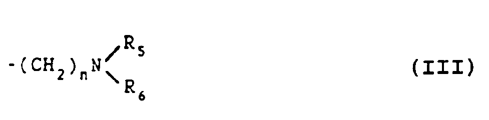 in which n represents an integer between 1 and 4 and R₅ and R₆, which may be identical or different, represent a hydrogen atom or an alkyl, phenyl or phenylalkyl radical, or alternatively R₅ and R₆, together with the nitrogen atom to which they are attached, form a morpholine, thiomorpholine, pyrrolidine, piperidine or piperazine ring in which the second nitrogen atom can be optionally substituted with an alkyl, hydroxyalkyl, pyridyl, phenyl, phenylalkyl or phenylcarbonyl radical, and R₄ represents a radical of general formula (III) defined as above, on the understanding that the definitions of n, R₅ and R₆ in the symbols R₃ and R₄ can be identical or different,
D- or Z represents an alkylene radical containing 1 to 4 carbon atoms and R₂ represents a radical of general formula: 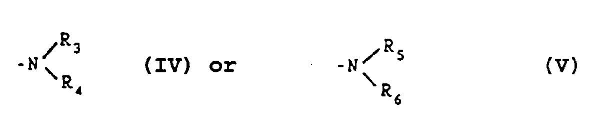 in which R₃, R₄, R₅ and R₆ are defined as above in C a) and b), on the understanding that the alkyl radicals and alkyl portions contain 1 to 4 carbon atoms in a straight or branched chain, as well as the racemic products, the enantiomers due to the presence of an asymmetric carbon at the 3-position of the pyrrolothiazole ring, the mixtures of these enantiomers, the diastereoisomers, pure or mixed, due to the possible presence of another chiral centre, and the E and Z (also known as syn and anti) isomers and mixtures thereof originating from the presence of the oxime group C=NO-Z-R₂, and the pharma- ceutically acceptable salts, characterized in that a hydroxylamine of general formula:
H₂NO-Z-R₂ (VI)
in which Z and R₂ are defined as above, is reacted with a ketone of general formula: 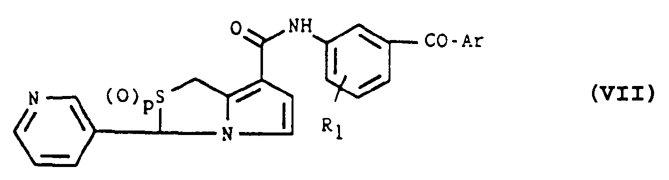 in which the symbols p, R₁ and Ar are defined as in above.

2. Process for preparing new derivatives as defined in claim 1 in the formula of which the symbols are defined as in claim 1 in B or in D, characterized in that a product of general formula:
X-Z-R₂ (VIII)
in which Z and R₂ are defined in claim 1 and X represents a halogen atom, is reacted with a product of general formula (I), defined as in claim 1 in A, i.e. a product of general formula: 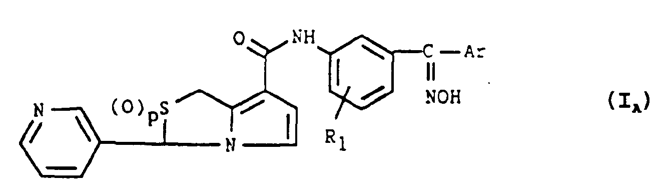 in which p, R₁ and Ar are defined as in claim 1.

3. Process for preparing new derivatives as defined in claim 1 in the formula of which p is equal to 0, characterized in that a product of general formula: 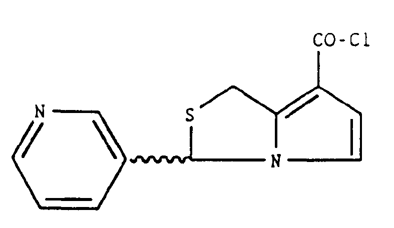 in R or S form or mixtures thereof, is reacted with an oxime of general formula: 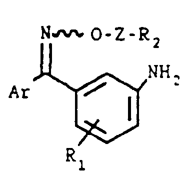 in which Ar, Z, R₁ and R₂ are defined as in claim 1, in E or Z form or mixtures thereof.

4. Process for preparing new derivatives as defined in claim 1 in the formula of which p is equal to 1, characterized in that a new derivative as defined in claim 1 in the formula of which p is equal to 0 is oxidized.
